# EUROPEAN PATENT APPLICATION

(11) **EP 3 639 855 A1**
(43) Date of publication of application: **22.04.2020**
(21) Application number: 18817847.9
(22) Date of filing: 15.06.2018
(51) Int. Cl.: A61K 45/00, A61K 31/135, A61K 31/138, A61K 31/192, A61K 31/42, A61K 31/436, A61K 31/573, A61K 31/58, A61K 31/665, A61K 31/714, A61P 3/02, A61P 27/02, A61P 29/00, A61P 43/00

(54) **COMPOUNDS HAVING CASPASE INHIBITORY ACTIVITY, PHARMACEUTICAL AGENT CONTAINING SAID COMPOUNDS AND FOR TREATING OR PREVENTING CORNEAL ENDOTHELIAL SYMPTOMS, DISORDERS, OR DISEASES, AND APPLICATION OF SAID PHARMACEUTICAL AGENT**

(30) Priority: 16.06.2017 JP 2017118617; 25.01.2018 JP 2018010825; 20.03.2018 JP 2018053235
(71) Applicant: The Doshisha, Kyoto-shi, Kyoto 602-8580 (JP)
(72) Inventor: KOIZUMI Noriko, Kyotanabe-shi Kyoto 610-0394 (JP); OKUMURA Naoki, Kyotanabe-shi Kyoto 610-0394 (JP)
(74) Representative: J A Kemp LLP
(86) International application number: PCT/JP2018/022945
(87) International publication number: WO 2018/230713

(57) **Abstract**

The present invention provides a composition for treating or preventing corneal endothelial symptoms, disorders, or diseases that are attributed to TGF-β signaling in corneal endothelial cells. Provided by the present invention is a composition that includes a compound and that is for treating or preventing endothelial symptoms, disorders, or diseases, wherein, when the compound comes into contact with immortalized cells of Fuchs' corneal endothelial dystrophy, (i) said immortalized cells exhibit a cell survival rate (%) of approximately 90% or more after being cultured for 24-28 hours in Dulbecco's modified Eagle medium (DMEM) + 2% fetal bovine serum (FBS) + 1% penicillin/streptomycin (P/S), and (ii) the ratio of caspase 3/7 activity (%) in the presence of TGF-β with respect to said cellular survival rate (%) is at most 0.8 after being cultured for 24-28 hours in Dulbecco's modified Eagle medium (DMEM) + 2% fetal bovine serum (FBS) + 1% penicillin/streptomycin (P/S).

## Description

### [Technical Field]

The present invention relates to a technique or method for treating or preventing a corneal endothelial condition, disorder, or disease due to a transforming growth factor-β (TGF-β) signal in corneal endothelial cells, and an agent therefor.

### [Background Art]

Visual information is recognized when light transmitted into the cornea, which is a transparent tissue at the front-most part of an eye ball, reaches the retina and excites nerve cells of the retina, and a generated electrical signal is transmitted through the optic nerve to the visual cortex of the cerebrum. To attain good vision, it is necessary that the cornea is transparent. The transparency of the cornea is retained by maintaining constant water content with pumping and barrier functions of corneal endothelial cells.

Human corneal endothelial cells are present at a density of about 3000 cells per 1 mm² at birth. Once damaged, human corneal endothelial cells have a very limited ability to regenerate. For example, Fuchs' endothelial corneal dystrophy is a disease causing abnormality in endothelial cells inside the cornea, resulting in edema of the cornea. The cause thereof is unknown. In Fuchs' endothelial corneal dystrophy, extracellular matrix such as collagen is deposited on a part of the back surface of a Descemet's membrane at the back of the cornea, resulting in guttae (Corneal guttae) and hypertrophy of the Descemet's membrane. Guttae (Corneal guttae) and hypertrophy of the Descemet's membrane are the cause of photophobia or blurred vision in Fuchs' endothelial corneal dystrophy patients, which significantly compromises the QOL of the patients. It is understood that there is no effective therapeutic method other than corneal transplant for Fuchs' endothelial corneal dystrophy. However, there is a shortage in cornea donation in Japan, where the number of patients waiting for corneal transplant is about 2600, whereas the number of corneal transplants performed in Japan is approximately 1700 annually.

For Fuchs' endothelial corneal dystrophy, culture (Non Patent Literatures 1 and 3) and immortalization (Non Patent Literature 2) of corneal endothelial cells from Fuchs' corneal dystrophy patients have been reported, but cells suitable for screening of a therapeutic drug or progression preventing drug which maintain the features of the disease, such as overproduction of extracellular matrices, have not been reported. Therefore, there is a limit to the development of a therapeutic drug thereof. Currently, there is no therapeutic drug that is used in clinical practice, so that therapy is reliant on corneal transplant.

### [Citation List]

### [Non Patent Literature]

[NPL 1] Zaniolo K, et al. Exp Eye Res.;94(1) : 22-31. 2012
[NPL 2] Azizi B, et al. Invest Ophthalmol Vis Sci. 2;52 (13):9291-9297. 2011
[NPL 3] Kelliher C. et al. Exp Eye Res Vol.93(6), 880-888, 2011

### [Summary of Invention]

### [Solution to Problem]

The inventors have discovered that a specific compound which was not previously known to suppress disorders or suppress caspase activity in a corneal endothelium has activity to suppress or inhibit caspase activity surprisingly, and that caspase 3/7 activity can be suppressed in cells of a corneal endothelial disorder model of Fuchs' endothelial corneal dystrophy in the presence of TGF-β.

In addition, the inventors have elucidated that a part of compounds which were able to suppress caspase 3/7 activity in the presence of TGF-β also has inhibiting activity against MG-132, and the inventors have also discovered that such compounds suppress endoplasmic reticulum (ER) associated stress induced by unfolded proteins.

A compound used in the present invention has been confirmed to be less toxic and highly safe to cells (iFCED or iHCEC). Thus, it is considered that a compound used in the present invention is very useful as a medicament preventing a corneal endothelial condition, disorder, or disease with respect to this point as well.

The present invention therefore provides, for example, the following items.
(Item 1) A composition for treating or preventing a corneal endothelial condition, disorder, or disease, the composition comprising a compound which, when contacted with immortalized Fuchs' endothelial corneal dystrophy cells, exhibits: (i) cell viability (%) of the cells of about 90% or more after being cultured in Dulbecco's Modified Eagle Medium (DMEM) + 2% fetal bovine serum (FBS) + 1% penicillin/streptomycin (P/S) for 24 to 28 hours; and (ii) ratio of caspase 3/7 activity (%) in the presence of TGF-β with respect to the cell viability (%) of 0.8 or less after being cultured in Dulbecco's Modified Eagle Medium (DMEM) + 2% fetal bovine serum (FBS) + 1% penicillin/streptomycin (P/S) for 24 to 28 hours.
(Item 2) The composition of the preceding item, wherein the culturing time is 28 hours.
(Item 3) The composition of any one of the preceding items, wherein the culturing time is 24 hours.
(Item 4) The composition of any one of the preceding items, wherein the compound is selected from the group consisting of an anti-inflammatory drug, vitamin B12 group, vitamin D group, selective glucocorticoid receptor agonist (SEGRA), selective glucocorticoid receptor modulator (SEGRM) and a combination thereof.
(Item 5) The composition of any one of the preceding items, comprising an anti-inflammatory drug.
(Item 6) The composition of any one of the preceding items, wherein the anti-inflammatory drug is a steroidal anti-inflammatory drug or a nonsteroidal anti-inflammatory drug (NSAID), or a combination thereof.
(Item 7) The composition of any one of the preceding items, wherein the anti-inflammatory drug is a steroidal anti-inflammatory drug, the steroidal anti-inflammatory drug being a compound selected from the group consisting of Mometasone Furoate, Clobetasol Propionate, Loteprednol Etabonate, Difluprednate, Dexamethasone, Amcinonide, Flurandrenolide, Prednisolone, Fluocinolone Acetonide, Desonide, Triamcinolone Acetonide, Budesonide, Fludrocortisone Acetate, Fluocinonide, Methylprednisolone, Betamethasone, Desoximetasone, Halcinonide, Fluorometholone, Beclomethasone Dipropionate, and Dutasteride, a derivative or an analogue thereof, or a pharmaceutically acceptable salt thereof, or a solvate thereof.
(Item 8) The composition of any one of the preceding items, wherein the anti-inflammatory drug is a nonsteroidal anti-inflammatory drug (NSAID), the nonsteroidal anti-inflammatory drug (NSAID) being a compound selected from the group consisting of Amlexanox, Leflunomide, Olsalazine•Na, Orphenadrine Citrate, Flurbiprofen, and Phenoxybenzamine•HCl, a derivative or an analogue thereof, or a pharmaceutically acceptable salt thereof, or a solvate thereof.
(Item 9) The composition of any one of the preceding items, wherein the compound is a vitamin B12 group or includes a vitamin B12 group, the vitamin B12 group being Hydroxocobalamin, a derivative or an analogue thereof, or a pharmaceutically acceptable salt thereof (e.g., Hydroxocobalamin•HCl or the like), or a solvate thereof.
(Item 10) The composition of any one of the preceding items, wherein the compound is a vitamin D group or includes a vitamin D group, the vitamin D group being Calcipotriene, a derivative or an analogue thereof, or a pharmaceutically acceptable salt thereof, or a solvate thereof.
(Item 11) The composition of any one of the preceding items, wherein the compound is selected from at least one compound shown in Table A:

**[Table A-1]**

| |
|---|
| Amlexanox |
| Leflunomide |
| Olsalazine•Na |
| Hydroxocobalamin•HCl |
| Mometasone Furoate |
| Febuxostat |
| Orphenadrine Citrate |
| Clobetasol Propionate |
| Loteprednol Etabonate |
| Difluprednate |
| Dexamethasone |
| Dobutamine•HCl |
| Amcinonide |
| Flurandrenolide |
| Fluorouracil (5-Fluorouracil) |
| Prednisolone |
| Fluocinolone Acetonide |
| Desonide |
| Triamcinolone Acetonide |
| Ketoconazole |
| Flurbiprofen |
| Budesonide |

**[Table A-2]**

| |
|---|
| Fludrocortisone Acetate |
| Fluocinonide |
| Methylprednisolone |
| Betamethasone |
| Desoximetasone |
| Toremifene Base |
| Terazosin•HCl |
| Halcinonide |
| Bromocriptine Mesylate |
| Fluorometholone |
| Oxaliplatin |
| Calcipotriene |
| Beclomethasone Dipropionate |
| Reserpine |
| Phenoxybenzamine•HCl |
| Droperidol |
| Promethazine•HCl |
| Esmolol |
| Dutasteride |
| Dihydroergotamine Mesylate |
| Imipramine•HCl |
| Betaxolol•HCl |
| Trimethobenzamide•HCl |

, a derivative or an analogue thereof, or a pharmaceutically acceptable salt thereof, or a solvate thereof.
(Item 12) The composition of any one of the preceding items, wherein the condition, disorder, or disease is a corneal endothelial condition, disorder, or disease due to transforming growth factor-β (TGF-β).
(Item 13) The composition of any one of the preceding items, wherein the condition, disorder, or disease is selected from the group consisting of Fuchs' endothelial corneal dystrophy, post-corneal transplant disorder, corneal endotheliitis, trauma, post-ophthalmic surgery disorder, post-ophthalmic laser surgery disorder, aging, posterior polymorphous dystrophy (PPD), congenital hereditary endothelial dystrophy (CHED), idiopathic corneal endothelial disorder, and cytomegalovirus corneal endotheliitis.
(Item 14) The composition of any one of the preceding items, wherein the condition, disorder, or disease includes Fuchs' endothelial corneal dystrophy.
(Item 15) The composition of any one of the preceding items, wherein the compound, when further contacted with immortalized human corneal endothelial cells, exhibits:
(i) cell viability (%) of the cells of about 90% or more after being cultured in Dulbecco's Modified Eagle Medium (DMEM) + 2% fetal bovine serum (FBS) + 1% penicillin/streptomycin (P/S) for 18 hours, and
(ii) ratio of caspase 3/7 activity (%) in the presence of MG-132 with respect to cell viability (%) of 0.8 or less after being cultured in Dulbecco's Modified Eagle Medium (DMEM) + 2% fetal bovine serum (FBS) + 1% penicillin/streptomycin (P/S) for 18 hours.

(Item 16) The composition of any one of the preceding items, wherein the compound is a compound selected from the group consisting of Amlexanox, Olsalazine•Na, Hydroxocobalamin•HCl, Leflunomide, Febuxostat, Flurbiprofen, Terazosin•HCl, and Fluorouracil (5-Fluorouracil), a derivative or an analogue thereof, or a pharmaceutically acceptable salt thereof, or a solvate thereof.
(Item 17) The composition of any one of the preceding items, wherein the condition, disorder, or disease is a corneal endothelial condition, disorder, or disease due to transforming growth factor-β (TGF-β) and endoplasmic reticulum (ER) associated stress.
(Item 18) The composition of any one of the preceding items, wherein the endoplasmic reticulum (ER) associated stress is due to abnormal folding of proteins and/or accumulation of abnormal proteins.
(Item 19) The composition of any one of the preceding items, wherein the condition, disorder, or disease is selected from conditions, disorders, or diseases associated with endoplasmic reticulum (ER) stress among damage to corneal endothelial cells in Fuchs' endothelial corneal dystrophy, corneal endothelial disorder, decreased corneal endothelial density, guttae formation, hypertrophy of the Descemet's membrane, hypertrophy of a cornea, turbidity, corneal epithelial disorder, turbidity in corneal stroma, photophobia, blurred vision, visual impairment, ophthalmalgia, epiphora, hyperemia, pain, bullous keratopathy, eye discomfort, diminished contrast, glare, edema of the corneal stroma, corneal epithelial erosion, and angiogenesis.
(Item 20) The composition of any one of the preceding items, wherein the condition, disorder, or disease includes Fuchs' endothelial corneal dystrophy.
(Item 21) A caspase inhibitor comprising a compound selected from at least one compound shown in Table B:

**[Table B-1]**

| |
|---|
| Amlexanox |
| Leflunomide |
| Olsalazine•Na |
| Hydroxocobalamin•HCl |
| Mometasone Furoate |
| Febuxostat |
| Orphenadrine Citrate |
| Clobetasol Propionate |
| Loteprednol Etabonate |
| Difluprednate |
| Dexamethasone |
| Dobutamine•HCl |
| Amcinonide |
| Flurandrenolide |
| Fluorouracil (5-Fluorouracil) |
| Prednisolone |
| Fluocinolone Acetonide |
| Desonide |
| Triamcinolone Acetonide |
| Ketoconazole |
| Flurbiprofen |
| Budesonide |
| Fludrocortisone Acetate |
| Fluocinonide |
| Methylprednisolone |
| Betamethasone |
| Desoximetasone |
| Toremifene Base |
| Terazosin•HCl |
| Halcinonide |
| Bromocriptine Mesylate |
| Fluorometholone |
| Oxaliplatin |
| Calcipotriene |
| Beclomethasone Dipropionate |

**[Table B-2]**

| |
|---|
| Reserpine |
| Phenoxybenzamine•HCl |
| Droperidol |
| Promethazine•HCl |
| Esmolol |
| Dutasteride |
| Dihydroergotamine Mesylate |
| Imipramine•HCl |
| Betaxolol•HCl |
| Trimethobenzamide•HCl |

, a derivative or an analogue thereof, or a pharmaceutically acceptable salt thereof, or a solvate thereof.
(Item 22) The caspase inhibitor of any one of the preceding items, inhibiting caspase 3/7 activity.
(Item 23) A composition for treating or preventing a corneal endothelial condition, disorder, or disease, the composition comprising a compound selected from at least one compound shown in Table C:

**[Table C]**

| |
|---|
| Nitazoxanide |
| Mycophenolate Mofetil |
| Mycophenolic Acid |
| Deferasirox |
| Carvedilol |
| Fluoxetine•HCl |
| Fulvestrant |
| Tolterodine Tartrate |
| Pyrimethamine |
| Rifapentine |
| Nisoldipine |
| Oxiconazole Nitrate |
| Pimecrolimus |
| Calcitriol |
| Aripiprazole |
| Asenapine Maleate |
| Terbinafine•HCl |

, a derivative or an analogue thereof, or a pharmaceutically acceptable salt thereof, or a solvate thereof.
(Item 24) A caspase inhibitor comprising a compound selected from at least one compound shown in Table D:

**[Table D]**

| |
|---|
| Nitazoxanide |
| Mycophenolate Mofetil |
| Mycophenolic Acid |
| Deferasirox |
| Carvedilol |
| Fluoxetine•HCl |
| Fulvestrant |
| Tolterodine Tartrate |
| Pyrimethamine |
| Rifapentine |
| Nisoldipine |
| Oxiconazole Nitrate |
| Pimecrolimus |
| Calcitriol |
| Aripiprazole |
| Asenapine Maleate |
| Terbinafine•HCl |

, a derivative or an analogue thereof, or a pharmaceutically acceptable salt thereof, or a solvate thereof.
(Item 25) A composition for treating or preventing a corneal endothelial condition, disorder, or disease, or a caspase inhibitor, the composition or the caspase inhibitor comprising a compound selected from at least one compound shown in Table E:

**[Table E-1]**

| |
|---|
| Terazosin•HCl |
| Ketoconazole |
| Toremifene Base |
| Nitazoxanide |
| Mometasone Furoate |
| Mycophenolate Mofetil |
| Mycophenolic Acid |
| Deferasirox |
| Carvedilol |
| Fluoxetine•HCl |
| Fulvestrant |
| Methylprednisolone |
| Dexamethasone |
| Betamethasone |
| Difluprednate |
| Prednisolone |
| Fludrocortisone Acetate |
| Flurandrenolide |
| Amcinonide |
| Budesonide |
| Clobetasol Propionate |
| Fluocinolone Acetonide |
| Tolterodine Tartrate |
| Imipramine•HCl |
| Beclomethasone Dipropionate |
| Pyrimethamine |
| Desonide |
| Oxaliplatin |
| Fluocinonide |
| Everolimus |
| Rifapentine |
| Bromocriptine Mesylate |
| Nisoldipine |
| Triamcinolone Acetonide |
| Fluorometholone |
| Desoximetasone |

**[Table E-2]**

| |
|---|
| Oxiconazole Nitrate |
| Halcinonide |
| Pimecrolimus |
| Calcitriol |
| Dihydroergotamine Mesylate |
| Aripiprazole |
| Asenapine Maleate |
| Terbinafine•HCl |

, a derivative or an analogue thereof, or a pharmaceutically acceptable salt thereof, or a solvate thereof.
(Item 26) The composition or the caspase inhibitor of any one of the preceding items, wherein the compound includes at least one selected from the group consisting of a selective glucocorticoid receptor agonist (SEGRA) and a selective glucocorticoid receptor modulator (SEGRM).
(Item 27) A composition for treating or preventing a corneal endothelial condition, disorder, or disease, the composition comprising at least one compound selected from the group consisting of a selective glucocorticoid receptor agonist (SEGRA) and a selective glucocorticoid receptor modulator (SEGRM).
(Item 28) The composition or the caspase inhibitor of any one of the preceding items, wherein the compound includes a selective glucocorticoid receptor agonist (SEGRA).
(Item 29) The composition or the caspase inhibitor of any one of the preceding items, wherein the compound includes a selective glucocorticoid receptor modulator (SEGRM).
(Item 30) The composition or the caspase inhibitor of any one of the preceding items, wherein the compound or the selective glucocorticoid receptor modulator (SEGRM) is a compound represented by the following general formula (1) or general formula (2) 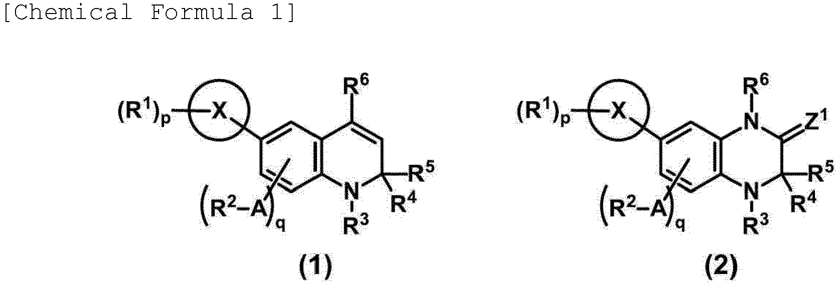 or a salt thereof, wherein:
ring X represents a benzene ring or a pyridine ring;
R¹ represents a halogen atom, a lower alkyl group which may have a substituent, a lower cycloalkyl group which may have a substituent, an aryl group which may have a substituent, an arylalkyloxy group which may have a substituent, an arylalkyloxyalkyloxy group, a heterocyclic group which may have a substituent, a hydroxy group, an ester of a hydroxy group, a lower alkoxy group which may have a substituent, a lower cycloalkyloxy group which may have a substituent, an aryloxy group which may have a substituent, a heterocyclic oxy group which may have a substituent, a mercapto group, an ester of a mercapto group, a lower alkylthio group which may have a substituent, a lower cycloalkylthio group which may have a substituent, an arylthio group which may have a substituent, a heterocyclic thio group which may have a substituent, a lower alkylamino group which may have a substituent, a lower cycloalkylamino group which may have a substituent, an arylamino group which may have a substituent, a heterocyclic amino group which may have a substituent, an amide of an amino group, an amide of a lower alkylamino group which may have a substituent, an amide of a lower cycloalkylamino group which may have a substituent, an amide of an arylamino group which may have a substituent, an amide of a heterocyclic amino group which may have a substituent, a formyl group, a lower alkylcarbonyl group which may have a substituent, a lower cycloalkylcarbonyl group which may have a substituent, an arylcarbonyl group which may have a substituent, a heterocyclic carbonyl group which may have a substituent, a carboxy group, an ester of a carboxy group, an amide of a carboxy group, a lower alkylsulfonyl group which may have a substituent, a lower cycloalkylsulfonyl group which may have a substituent, an arylsulfonyl group which may have a substituent, a heterocyclic sulfonyl group which may have a substituent, a sulfonic acid group, an ester of a sulfonic acid group, an amide of a sulfonic acid group, a lower alkenyloxy group which may have a substituent, a silyl group which may have a substituent,-W¹-C(=O)-R¹⁸, -W¹-C(=O)-O-R¹⁸, -W¹-S(=O)-R¹9, -W¹-S(=O)₂-R²⁰,-W¹-C(=O)NR²¹R²², -OCH₂CH(R²³)-OR²⁴, an amino group which may have a substituent, a nitro group, or a cyano group;
R^{18,} R¹⁹, R²⁰, R²¹ and R²² each independently represent a hydrogen atom, a lower alkyl group which may have a substituent, a lower alkenyl group which may have a substituent, a lower alkynyl group which may have a substituent, a lower cycloalkyl group which may have a substituent, an aryl group which may have a substituent, a heterocyclic group which may have a substituent, an aralkyl group which may have a substituent, a heterocyclic alkyl group which may have a substituent, an amino lower alkyl group which may have a substituent, a lower alkoxy group which may have a substituent, a lower alkenyloxy group which may have a substituent, a lower alkynyloxy group which may have a substituent, a lower cycloalkyloxy group which may have a substituent, an aryloxy group which may have a substituent, or a heterocyclic oxy group which may have a substituent;
p represents an integer of 0 to 5;
when p is 2 to 5, each R¹ may be the same or different;
W¹ represents an oxygen atom, a sulfur atom, or -N(R²⁵)-;
Z¹ represents an oxygen atom, a sulfur atom, or =N-R²⁵;
R²³ represents a hydrogen atom, a lower alkyl group which may have a substituent, a carboxy group, an ester of a carboxy group, an amide of a carboxy group, or a cyano group;
R²⁴ represents a hydrogen atom, a lower alkylcarbonyl group which may have a substituent, a lower cycloalkylcarbonyl group which may have a substituent, an arylcarbonyl group which may have a substituent, a heterocyclic carbonyl group which may have a substituent, an ester of a carboxy group, an amide of a carboxy group, a phosphoric acid group, or an ester of a phosphoric acid group;
R²⁵ represents a hydrogen atom, a lower alkyl group which may have a substituent, a lower alkenyl group which may have a substituent, a lower alkynyl group which may have a substituent, an aryl group which may have a substituent, a lower alkylcarbonyl group which may have a substituent, a lower alkenylcarbonyl group which may have a substituent, an arylalkyl group which may have a substituent, an arylalkyloxyalkyl group which may have a substituent, a heterocyclic alkyl group which may have a substituent, a silyl group which may have a substituent, a lower alkynylcarbonyl group which may have a substituent, or an arylcarbonyl group which may have a substituent;
R² represents a halogen atom, a lower alkyl group which may have a substituent, -OR⁸, -NR⁸R⁹, -SR⁸, -S(=O)-R⁸ or-S(=O)₂-R⁸;
q represents an integer of 0 to 3;
when q is 2 or 3, each R² may be the same or different;
R³ represents a hydrogen atom, a lower alkyl group which may have a substituent, a lower alkenyl group which may have a substituent, a lower alkynyl group which may have a substituent, an aryl group which may have a substituent, a lower alkylcarbonyl group which may have a substituent, a lower alkenylcarbonyl group which may have a substituent, an arylalkyl group which may have a substituent, an arylalkyloxyalkyl group which may have a substituent, a silyl group which may have a substituent, a lower alkynylcarbonyl group which may have a substituent, or an arylcarbonyl group which may have a substituent;
R⁴ and R⁵ each independently represent a hydrogen atom, a halogen atom, a lower alkyl group which may have a substituent, a lower alkenyl group which may have a substituent, a lower alkynyl group which may have a substituent, a lower cycloalkyl group which may have a substituent, an aryl group which may have a substituent, or a heterocyclic group which may have a substituent, or R⁴ and R⁵ may together form a 3- to 8-membered lower cycloalkane ring which may have a substituent;
R⁶ represents a hydrogen atom, a lower alkyl group which may have a substituent, a lower alkenyl group which may have a substituent, a lower alkynyl group which may have a substituent, a lower cycloalkyl group which may have a substituent, an aryl group which may have a substituent, or a heterocyclic group which may have a substituent;
A represents a lower alkylene group which may have a substituent or a single bond;
R⁸ and R⁹ each independently represent a hydrogen atom, a lower alkyl group which may have a substituent, a lower alkenyl group which may have a substituent, a lower alkynyl group which may have a substituent, a lower cycloalkyl group which may have a substituent, an aryl group which may have a substituent, a heterocyclic group which may have a substituent, a formyl group, a lower alkylcarbonyl group which may have a substituent, a lower alkenylcarbonyl group which may have a substituent, a lower alkynylcarbonyl group which may have a substituent, a lower cycloalkylcarbonyl group which may have a substituent, an arylcarbonyl group which may have a substituent, a heterocyclic carbonyl group which may have a substituent, a carboxy group, a lower alkoxycarbonyl group which may have a substituent, a lower alkenyloxycarbonyl group which may have a substituent, a lower alkynyloxycarbonyl group which may have a substituent, a lower cycloalkyloxycarbonyl group which may have a substituent, an aryloxycarbonyl group which may have a substituent, a heterocyclic oxycarbonyl group which may have a substituent, an arylcarbonyloxy group which may have a substituent, a heterocyclic carbonyloxy group which may have a substituent, an aryloxy group which may have a substituent, a heterocyclic oxy group which may have a substituent, an arylthio group which may have a substituent, a lower alkylsulfonyl group which may have a substituent, a lower alkenylsulfonyl group which may have a substituent, a lower alkynylsulfonyl group which may have a substituent, a lower cycloalkylsulfonyl group which may have a substituent, an arylsulfonyl group which may have a substituent, a heterocyclic sulfonyl group which may have a substituent, an arylamino group which may have a substituent, an aminocarbonyl group, a lower alkylaminocarbonyl group which may have a substituent, a lower alkenylaminocarbonyl group which may have a substituent, a lower alkynylaminocarbonyl group which may have a substituent, a lower cycloalkylaminocarbonyl group which may have a substituent, an arylaminocarbonyl group which may have a substituent, or a heterocyclic aminocarbonyl group which may have a substituent; and
when R² is NR⁸R⁹, R⁸ and R⁹ may together form a 3- to 8-membered nitrogen-containing heterocycle which may have a substituent.

(Item 31) The composition or the caspase inhibitor of any one of the preceding items, wherein the compound or the selective glucocorticoid receptor modulator (SEGRM) is a compound represented by the following general formulas (3), (4), (5), (6), (7), (8), (9), (10-a), (10-b), (12), (13-a), (13-b), (14), (15), (16), (17), (18), (19) or (20) 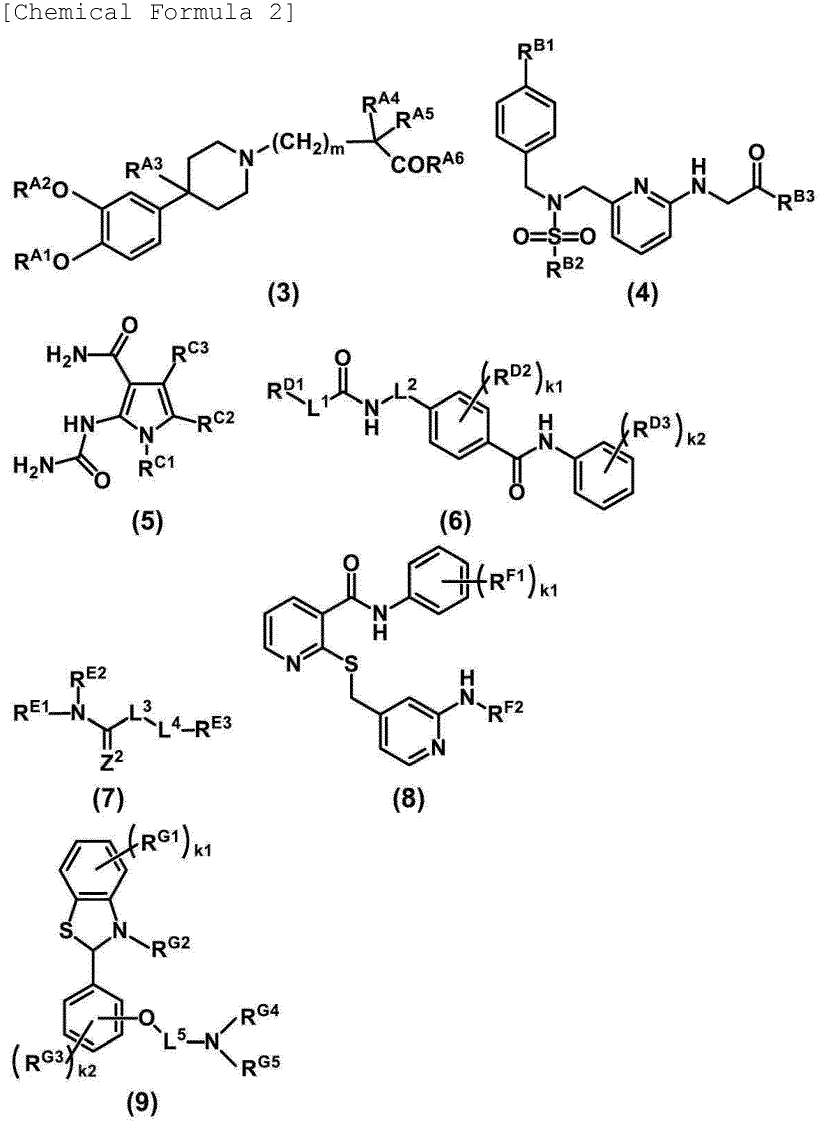 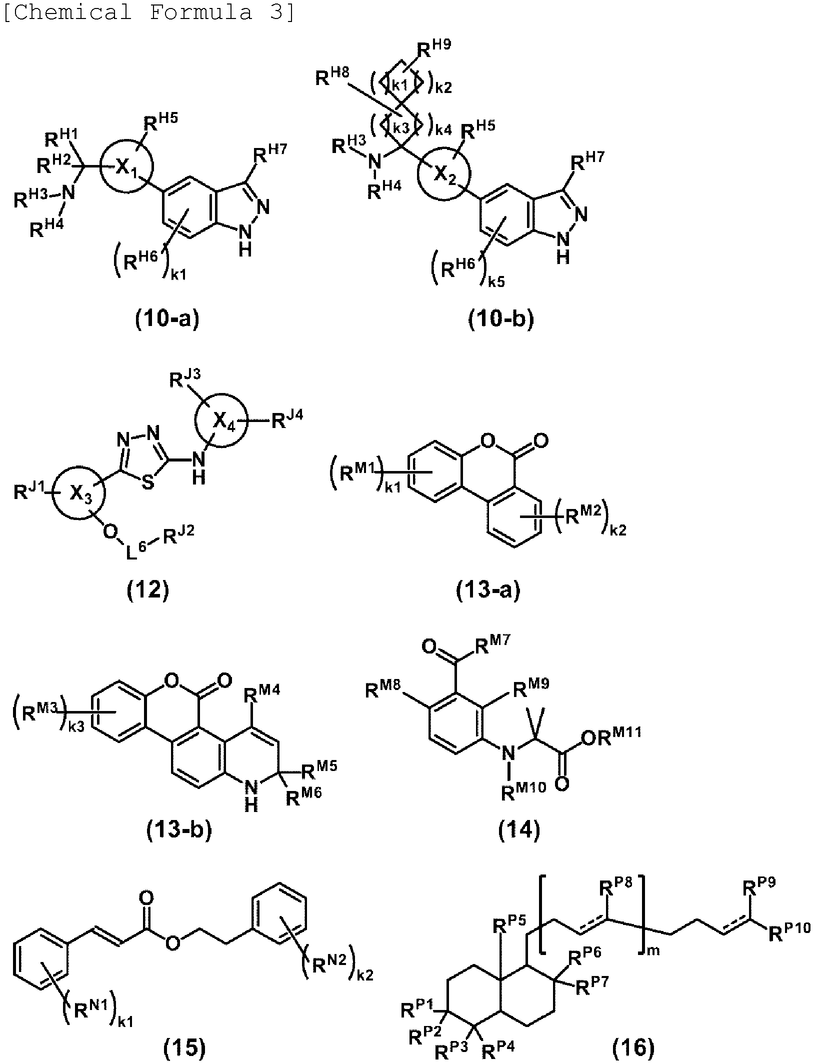 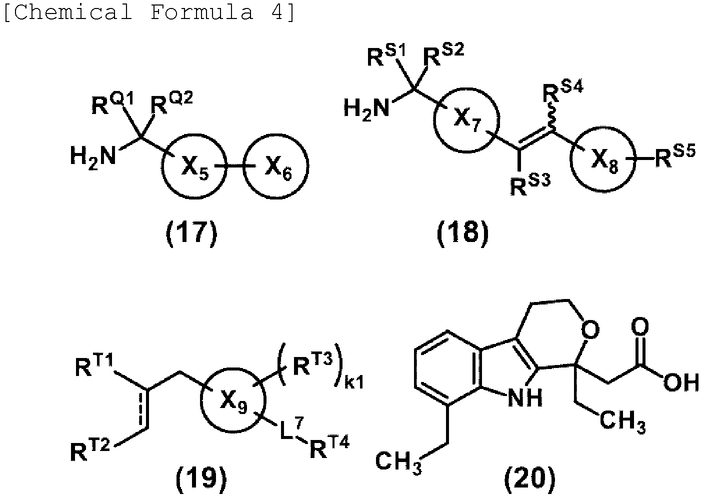 or a salt thereof, wherein

[Chemical Formula 5] **---**

represents a single bond or a double bond,
R^{A1}, R^{A2}, R^{A3}, R^{A4}, R^{A5}, R^{A6}, R^{B1}, R^{B2}, R^{B3}, R^{C1}, R^{C2}, R^{C3}, R^{D1}, R^{D2}, R^{D3}, R^{E1}, R^{E2}, R^{E3}, R^{F1}, R^{F2}, R^{G1}, R^{G2}, R^{G3}, R^{G4}, R^{G5}, R^{H1}, R^{H2}, R^{H3}, R^{H4}, R^{H5}, R^{H6}, R^{H7}, R^{H8}, R^{H9}, R^{J1}, R^{J2}, R^{J3}, R^{J4}, R^{M1}, R^{M2}, R^{M3}, R^{M4}, R^{M5}, R^{M6}, R^{M7}, R^{M8}, R^{M9}, R^{M10}, R^{M11}, R^{N1}, R^{N2}, R^{P1}, R^{P2}, R^{P3}, R^{P4}, R^{P5}, R^{P6}, R^{P7}, R^{P8}, R^{P9}, R^{P10}, R^{Q1}, R^{Q2}, R^{S1}, R^{S2}, R^{S3}, R^{S4}, R^{S5}, R^{T1}, R^{T2}, R^{T3}, and R^{T4} are each independently selected from the substituent group A,
ring X₁, X₂, X₃, X₄, X₅, X₆, X₇, X₈, and X₉ are each independently a lower cycloalkyl group which may have a substituent, a lower cycloalkenyl group which may have a substituent, an aryl group which may have a substituent, or a heterocyclic group which may have a substituent,
   L¹, L², L³, L⁴, L⁵, L⁶ and L⁷ are each independently a single bond, a lower alkylene group which may have a substituent, an arylene group which may have a substituent, a heteroarylene group which may have a substituent, a heterocyclylene group which may have a substituent, -C(=O)-, -C(=O)-O-, -S(=O)-, -S(=O)₂- or -C(=O)-NH-,
each m, each k1, each k2, each k3, each k4, and each k5 are independently 0, 1, 2, 3, 4, or 5, and
a wavy line bond is a single bond representing a stereoisomerism of (E) or (Z).

(Item 32) The composition or the caspase inhibitor of any one of the preceding items, wherein the selective glucocorticoid receptor modulator (SEGRM) is a compound selected from the group consisting of (R)-trans-N-(pyridine-4-yl)-4-(1-aminoethyl)cyclohexane carboxamide: 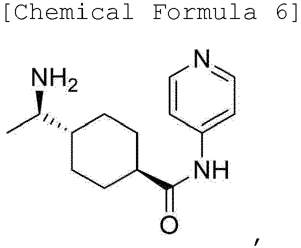 (R)-(+)-N-(1H-pyrrolo[2,3-b]pyridine-4-yl)-4-(1-aminoethyl)benzamide: 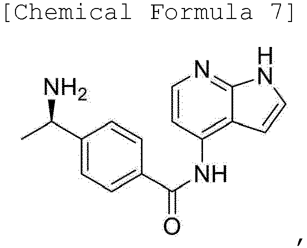 1-(5-isoquinolinesulfonyl)homopiperazine: 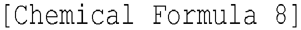 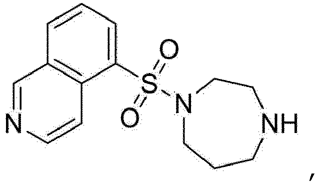 and
1-(5-isoquinolinesulfonyl)-2-methylpiperazine: 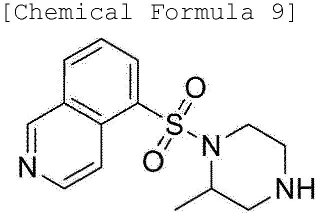 or a salt thereof.
(Item 33) The composition or the caspase inhibitor of any one of the preceding items, wherein the compound or the selective glucocorticoid receptor agonist (SEGRA) is selected from the group consisting of fluticasone, flumethasone, and RU-24858.
(Item 34) The composition or the caspase inhibitor of any one of the preceding items, wherein the compound or the selective glucocorticoid receptor agonist (SEGRA) is fluticasone propionate or fluticasone furoate.
(Item 35) The composition or the caspase inhibitor of any one of the preceding items, wherein the compound or the selective glucocorticoid receptor modulator (SEGRM) is selected from the group consisting of Mapracorat, ZK216348, ZK209614, Dagrocorat, Fosdagrocorat, Compound A, AL-438, LGD-5552, C108297, MK-5932, Org 214007-0, PF-802, DE-110, and Compound 10.
(Item 36) The composition or the caspase inhibitor of any one of the preceding items, wherein the condition, disorder, or disease is a corneal endothelial condition, disorder, or disease due to transforming growth factor-β (TGF-β) and endoplasmic reticulum (ER) associated stress.
(Item 37) The composition or the caspase inhibitor of any one of the preceding items, wherein the endoplasmic reticulum (ER) associated stress is due to abnormal folding of proteins and/or accumulation of abnormal proteins.
(Item 38) The composition or the caspase inhibitor of any one of the preceding items, wherein the condition, disorder, or disease is selected from conditions, disorders, or diseases associated with endoplasmic reticulum (ER) stress among damage to corneal endothelial cells in Fuchs' endothelial corneal dystrophy, corneal endothelial disorder, decreased corneal endothelial density, guttae formation, hypertrophy of the Descemet's membrane, hypertrophy of a cornea, turbidity, corneal epithelial disorder, turbidity in corneal stroma, photophobia, blurred vision, visual impairment, ophthalmalgia, epiphora, hyperemia, pain, bullous keratopathy, eye discomfort, diminished contrast, glare, edema of the corneal stroma, corneal epithelial erosion, and angiogenesis.
(Item 39) The composition or the caspase inhibitor of any one of the preceding items, wherein the condition, disorder, or disease includes Fuchs' endothelial corneal dystrophy.
(Item 40) A method for treating or preventing a corneal endothelial condition, disorder, or disease, the method comprising administering an effective amount of a compound to a subject in need thereof, wherein the compound, when contacted with immortalized Fuchs' endothelial corneal dystrophy cells, exhibits: (i) cell viability (%) of the cells of about 90% or more after being cultured in Dulbecco's Modified Eagle Medium (DMEM) + 2% fetal bovine serum (FBS) + 1% penicillin/streptomycin (P/S) for 24 to 28 hours; and (ii) ratio of caspase 3/7 activity (%) in the presence of TGF-β with respect to the cell viability (%) of 0.8 or less after being cultured in Dulbecco's Modified Eagle Medium (DMEM) + 2% fetal bovine serum (FBS) + 1% penicillin/streptomycin (P/S) for 24 to 28 hours.
(Item 41) A method for treating or preventing a corneal endothelial condition, disorder, or disease, the method comprising administering an effective amount of a compound to a subject in need thereof, wherein the compound includes at least one selected from the group consisting of a selective glucocorticoid receptor agonist (SEGRA) and a selective glucocorticoid receptor modulator (SEGRM).
(Item 42) A method for treating or preventing a corneal endothelial condition, disorder, or disease, the method comprising administering an effective amount of a compound to a subject in need thereof, wherein the compound is a compound selected from at least one compound shown in Table C:

**[Table C]**

| |
|---|
| Nitazoxanide |
| Mycophenolate Mofetil |
| Mycophenolic Acid |
| Deferasirox |
| Carvedilol |
| Fluoxetine•HCl |
| Fulvestrant |
| Tolterodine Tartrate |
| Pyrimethamine |
| Rifapentine |
| Nisoldipine |
| Oxiconazole Nitrate |
| Pimecrolimus |
| Calcitriol |
| Aripiprazole |
| Asenapine Maleate |
| Terbinafine•HCl |

, a derivative or an analogue thereof, or a pharmaceutically acceptable salt thereof, or a solvate thereof.
(Item 43) The method of any one of items 40 to 42, comprising any one or more features of the preceding items.
(Item 44) Use of a compound for manufacturing a medicament for treating or preventing a corneal endothelial condition, disorder, or disease, wherein the compound, when contacted with immortalized Fuchs' endothelial corneal dystrophy cells, exhibits: (i) cell viability (%) of the cells of about 90% or more after being cultured in Dulbecco's Modified Eagle Medium (DMEM) + 2% fetal bovine serum (FBS) + 1% penicillin/streptomycin (P/S) for 24 to 28 hours; and (ii) ratio of caspase 3/7 activity (%) in the presence of TGF-β with respect to the cell viability (%) of 0.8 or less after being cultured in Dulbecco's Modified Eagle Medium (DMEM) + 2% fetal bovine serum (FBS) + 1% penicillin/streptomycin (P/S) for 24 to 28 hours.
(Item 45) Use of a compound for manufacturing a medicament for treating or preventing a corneal endothelial condition, disorder, or disease, wherein the compound includes at least one selected from the group consisting of a selective glucocorticoid receptor agonist (SEGRA) and a selective glucocorticoid receptor modulator (SEGRM).
(Item 46) Use of a compound for manufacturing a medicament for treating or preventing a corneal endothelial condition, disorder, or disease, wherein the compound is a compound selected from at least one compound shown in Table C:

**[Table C]**

| |
|---|
| Nitazoxanide |
| Mycophenolate Mofetil |
| Mycophenolic Acid |
| Deferasirox |
| Carvedilol |
| Fluoxetine•HCl |
| Fulvestrant |
| Tolterodine Tartrate |
| Pyrimethamine |
| Rifapentine |
| Nisoldipine |
| Oxiconazole Nitrate |
| Pimecrolimus |
| Calcitriol |
| Aripiprazole |
| Asenapine Maleate |
| Terbinafine•HCl |

, a derivative or an analogue thereof, or a pharmaceutically acceptable salt thereof, or a solvate thereof.
(Item 47) The use of any one of items 44 to 46, comprising any one or more features of the preceding items.
(Item 48) A compound for treating or preventing a corneal endothelial condition, disorder, or disease, wherein the compound, when contacted with immortalized Fuchs' endothelial corneal dystrophy cells, exhibits: (i) cell viability (%) of the cells of about 90% or more after being cultured in Dulbecco's Modified Eagle Medium (DMEM) + 2% fetal bovine serum (FBS) + 1% penicillin/streptomycin (P/S) for 24 to 28 hours; and (ii) ratio of caspase 3/7 activity (%) in the presence of TGF-β with respect to the cell viability (%) of 0.8 or less after being cultured in Dulbecco's Modified Eagle Medium (DMEM) + 2% fetal bovine serum (FBS) + 1% penicillin/streptomycin (P/S) for 24 to 28 hours.
(Item 49) At least one compound for treating or preventing a corneal endothelial condition, disorder, or disease, wherein the at least one compound is selected from the group consisting of a selective glucocorticoid receptor agonist (SEGRA) and a selective glucocorticoid receptor modulator (SEGRM).
(Item 50) A compound which is a compound selected from at least one compound shown in Table C:

**[Table C]**

| |
|---|
| Nitazoxanide |
| Mycophenolate Mofetil |
| Mycophenolic Acid |
| Deferasirox |
| Carvedilol |
| Fluoxetine•HCl |
| Fulvestrant |
| Tolterodine Tartrate |
| Pyrimethamine |
| Rifapentine |
| Nisoldipine |
| Oxiconazole Nitrate |
| Pimecrolimus |
| Calcitriol |
| Aripiprazole |
| Asenapine Maleate |
| Terbinafine•HCl |

, a derivative or an analogue thereof, or a pharmaceutically acceptable salt thereof, or a solvate thereof.
(Item 51) The use of a compound of any one of items 48 to 50, comprising any one or more of features of the preceding items.
(Item 52) A method for inhibiting caspase activity, the method comprising contacting an effective amount of a compound with a subject in need thereof, wherein the compound is a compound selected from at least one compound shown in Table B:

**[Table B-1]**

| |
|---|
| Amlexanox |
| Leflunomide |
| Olsalazine•Na |
| Hydroxocobalamin•HCl |
| Mometasone Furoate |
| Febuxostat |
| Orphenadrine Citrate |
| Clobetasol Propionate |
| Loteprednol Etabonate |
| Difluprednate |
| Dexamethasone |
| Dobutamine•HCl |
| Amcinonide |
| Flurandrenolide |
| Fluorouracil (5-Fluorouracil) |
| Prednisolone |
| Fluocinolone Acetonide |
| Desonide |
| Triamcinolone Acetonide |
| Ketoconazole |
| Flurbiprofen |
| Budesonide |
| Fludrocortisone Acetate |
| Fluocinonide |
| Methylprednisolone |
| Betamethasone |
| Desoximetasone |
| Toremifene Base |
| Terazosin•HCl |
| Halcinonide |
| Bromocriptine Mesylate |
| Fluorometholone |
| Oxaliplatin |
| Calcipotriene |
| Beclomethasone Dipropionate |

**[Table B-2]**

| |
|---|
| Reserpine |
| Phenoxybenzamine•HCl |
| Droperidol |
| Promethazine•HCl |
| Esmolol |
| Dutasteride |
| Dihydroergotamine Mesylate |
| Imipramine•HCl |
| Betaxolol•HCl |
| Trimethobenzamide•HCl |

, a derivative or an analogue thereof, or a pharmaceutically acceptable salt thereof, or a solvate thereof.
(Item 53) A method for inhibiting caspase activity, the method comprising contacting an effective amount of a compound with a subject in need thereof, wherein the compound is a compound selected from at least one compound shown in Table D:

**[Table D]**

| |
|---|
| Nitazoxanide |
| Mycophenolate Mofetil |
| Mycophenolic Acid |
| Deferasirox |
| Carvedilol |
| Fluoxetine•HCl |
| Fulvestrant |
| Tolterodine Tartrate |
| Pyrimethamine |
| Rifapentine |
| Nisoldipine |
| Oxiconazole Nitrate |
| Pimecrolimus |
| Calcitriol |
| Aripiprazole |
| Asenapine Maleate |
| Terbinafine•HCl |

, a derivative or an analogue thereof, or a pharmaceutically acceptable salt thereof, or a solvate thereof.
(Item 54) The method of item 52 or 53, comprising any one or more features of the preceding items.
(Item 55) Use of a compound for manufacturing a medicament for inhibiting caspase activity, wherein the compound is a compound selected from at least one compound shown in
Table B described in the present specification,
a derivative or an analogue thereof, or a pharmaceutically acceptable salt thereof, or a solvate thereof.
(Item 56) Use of a compound for manufacturing a medicament for inhibiting caspase activity, wherein the compound is a compound selected from at least one compound shown in
Table D described in the present specification,
a derivative or an analogue thereof, or a pharmaceutically acceptable salt thereof, or a solvate thereof.
(Item 57) Ths use of item 55 or 56, comprising any one or more features of the preceding items.
(Item 58) A compound for inhibiting caspase activity, wherein the compound is a compound selected from at least one compound shown in
Table B described in the present specification,
a derivative or an analogue thereof, or a pharmaceutically acceptable salt thereof, or a solvate thereof.
(Item 59) A compound for inhibiting caspase activity, wherein the compound is a compound selected from at least one compound shown in
Table D described in the present specification,
a derivative or an analogue thereof, or a pharmaceutically acceptable salt thereof, or a solvate thereof.
(Item 60) The compound of item 58 or 59, comprising any one or more features of the preceding items.

The present invention is intended so that one or more of the aforementioned features can be provided not only as the explicitly disclosed combinations, but also as other combinations thereof. Additional embodiments and advantages of the present invention are recognized by those skilled in the art by reading and understanding the following detailed description, as needed.

### [Advantageous Effects of Invention]

The present invention provides a new medicament that can treat or prevent an endothelial condition, disorder, or disease due to a transforming growth factor-β (TGF-β) signal. The present invention also provides a new medicament that can treat or prevent an endothelial condition, disorder, or disease due to a TGF-β signal and endoplasmic reticulum (ER) associated stress.

### [Brief Description of Drawings]

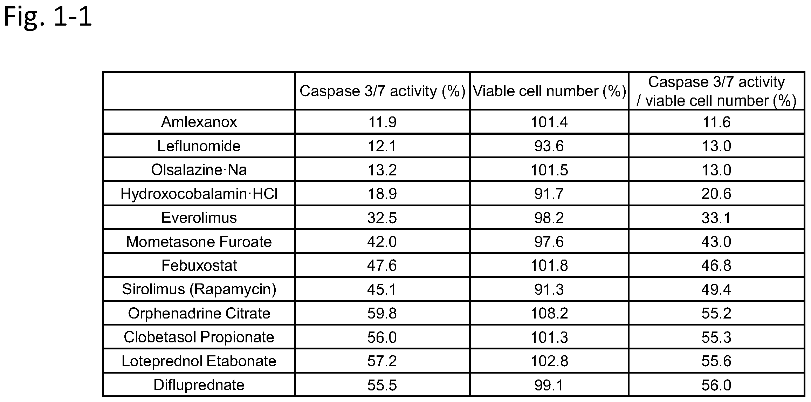

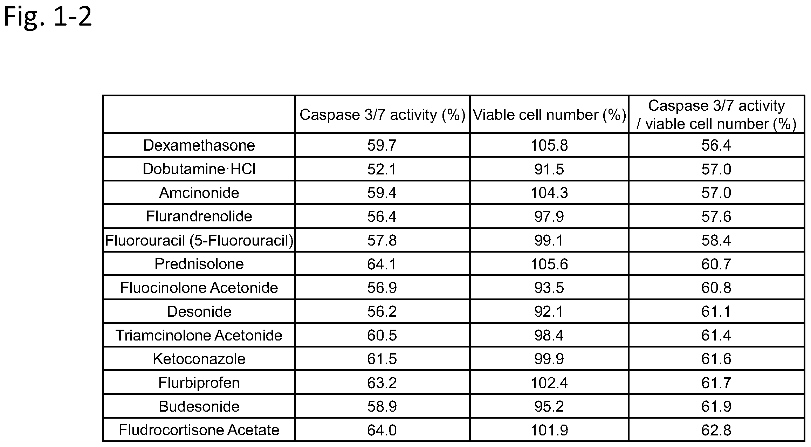

[Figure 1-1] Figure **1-1** and Figure **1-2** show the agent name of each agent of SCREEN-WELL® FDA Approved Drug Library V2, Japan version, results of caspase 3/7 activity (n = 5) under TGF-β2 stimulation, results of cell viability, and values obtained by dividing a value of caspase 3/7 activity by a value of the cell viability ((caspase 3/7 activity)/(cell viability) (%)). Figure **1-1** and Figure **1-2** show the agents ranked from the 1st to the 25th according to the value of (caspase 3/7 activity)/(cell viability) (%).
[Figure 1-2] Figure **1-1** and Figure **1-2** show the agent name of each agent of SCREEN-WELL® FDA Approved Drug Library V2, Japan version, results of caspase 3/7 activity (n = 5) under TGF-β2 stimulation, results of cell viability, and values obtained by dividing a value of caspase 3/7 activity by a value of the cell viability ((caspase 3/7 activity)/(cell viability) (%)). Figure **1-1** and Figure **1-2** show the agents ranked from the 1st to the 25th according to the value of (caspase 3/7 activity)/(cell viability) (%).

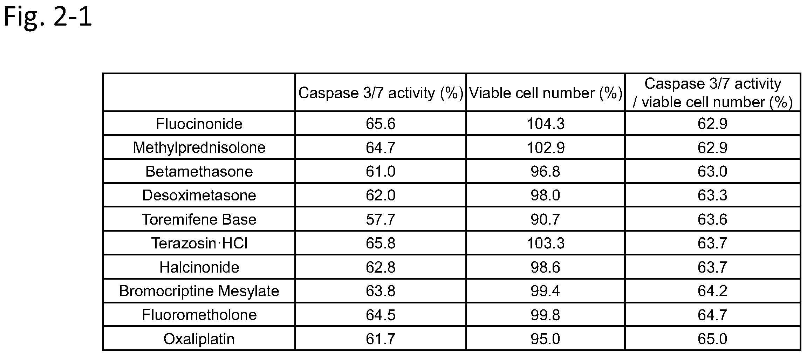

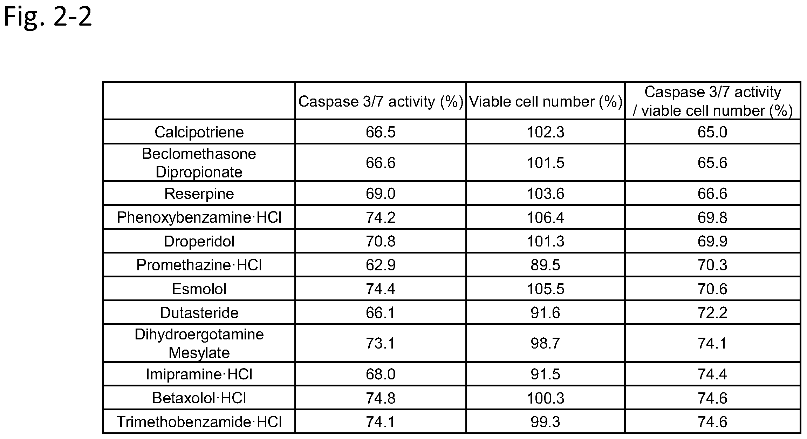

[Figure 2-1] Figure **2-1** and Figure **2-2** show the agent name of each agent of SCREEN-WELL® FDA Approved Drug Library V2, Japan version, results of caspase 3/7 activity (n = 5) under TGF-β2 stimulation, results of cell viability, and values obtained by dividing a value of caspase 3/7 activity by a value of the cell viability ((caspase 3/7 activity)/(cell viability) (%)). Figure **2-1** and **2-2** show the agents ranked from the 26th to the 47th according to the value of (caspase 3/7 activity)/(cell viability) (%).
[Figure 2-2] Figure **2-1** and Figure **2-2** show the agent name of each agent of SCREEN-WELL® FDA Approved Drug Library V2, Japan version, results of caspase 3/7 activity (n = 5) under TGF-β2 stimulation, results of cell viability, and values obtained by dividing a value of caspase 3/7 activity by a value of the cell viability ((caspase 3/7 activity)/(cell viability) (%)). Figure **2-1** and **2-2** show the agents ranked from the 26th to the 47th according to the value of (caspase 3/7 activity)/(cell viability) (%).

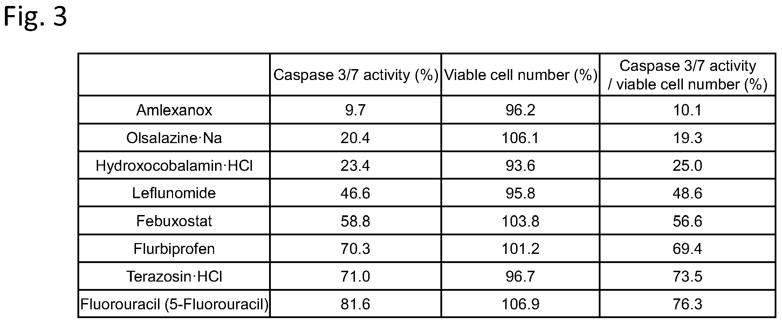

[Figure 3] Figure **3** shows the name of each agent, results of caspase 3/7 activity under MG-132 (0.1 µM) stimulation, results of cell viability, and values obtained by dividing a value of caspase 3/7 activity by a value of the cell viability ((caspase 3/7 activity)/(cell viability) (%)).

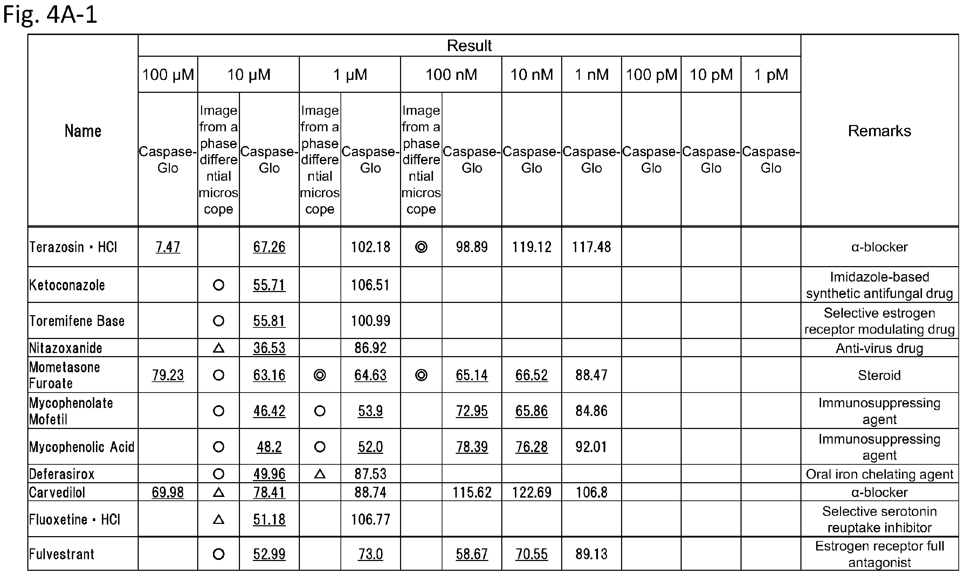

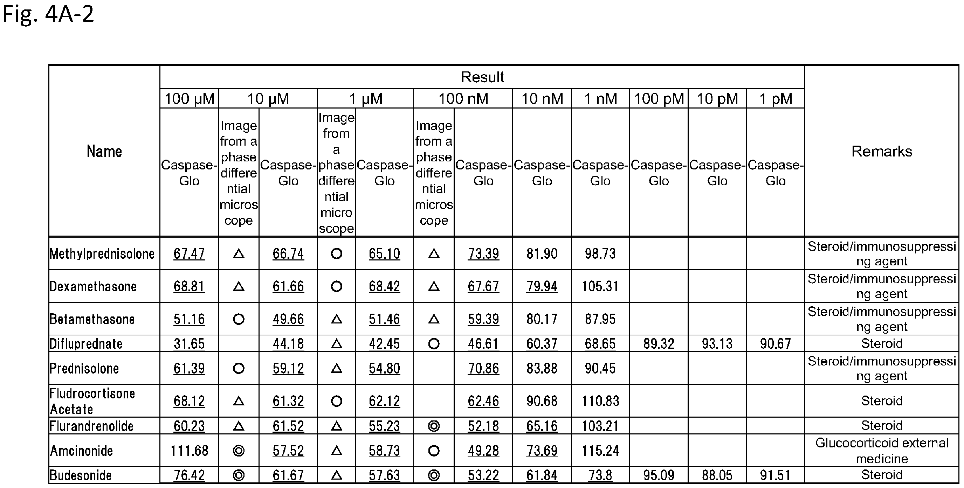

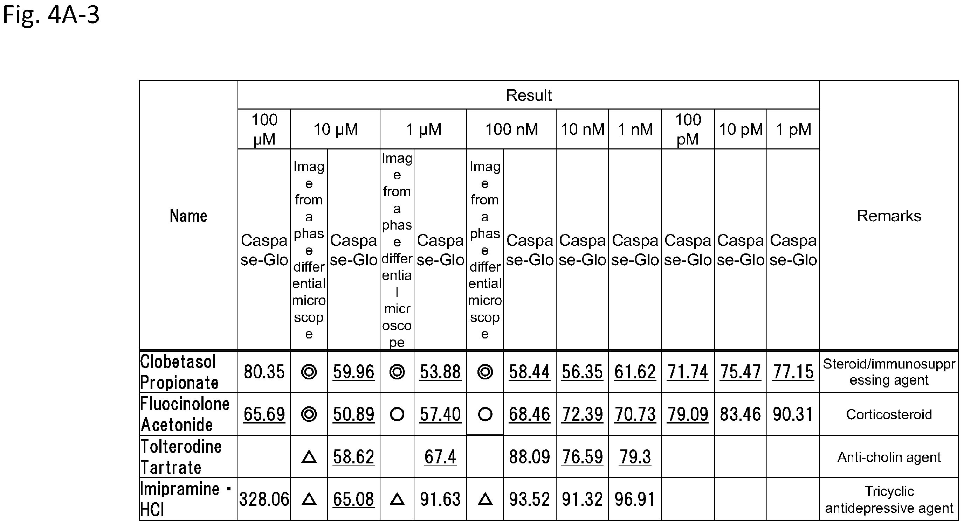

[Figure 4A-1] Figure **4A-1****,** Figure **4A-2****,** and Figure **4A-3** show caspase 3/7 activity of each agent at various concentrations under TGF-β2 stimulation. "Caspase-Glo" shows caspase 3/7 activity measured by Caspase-Glo® 3/7 Assay. In "phase contrast microscope", ⊚ indicates free cell ratio of 1 or more and less than 1.2, ○ indicates free cell ratio of 1.2 or more and less than 1.4, and Δ indicates free cell ratio of 1.4 or more and less than 1.5 when free cells of a control are assumed to be "1" in observation using a phase contrast microscope.
[Figure 4A-2] Figure **4A-1****,** Figure **4A-2****,** and Figure **4A-3** show caspase 3/7 activity of each agent at various concentrations under TGF-β2 stimulation. "Caspase-Glo" shows caspase 3/7 activity measured by Caspase-Glo® 3/7 Assay. In "phase contrast microscope", ⊚ indicates free cell ratio of 1 or more and less than 1.2, ○ indicates free cell ratio of 1.2 or more and less than 1.4, and Δ indicates free cell ratio of 1.4 or more and less than 1.5 when free cells of a control are assumed to be "1" in observation using a phase contrast microscope.
[Figure 4A-3] Figure **4A-1****,** Figure **4A-2****,** and Figure **4A-3** show caspase 3/7 activity of each agent at various concentrations under TGF-β2 stimulation. "Caspase-Glo" shows caspase 3/7 activity measured by Caspase-Glo® 3/7 Assay. In "phase contrast microscope", ⊚ indicates free cell ratio of 1 or more and less than 1.2, ○ indicates free cell ratio of 1.2 or more and less than 1.4, and Δ indicates free cell ratio of 1.4 or more and less than 1.5 when free cells of a control are assumed to be "1" in observation using a phase contrast microscope.

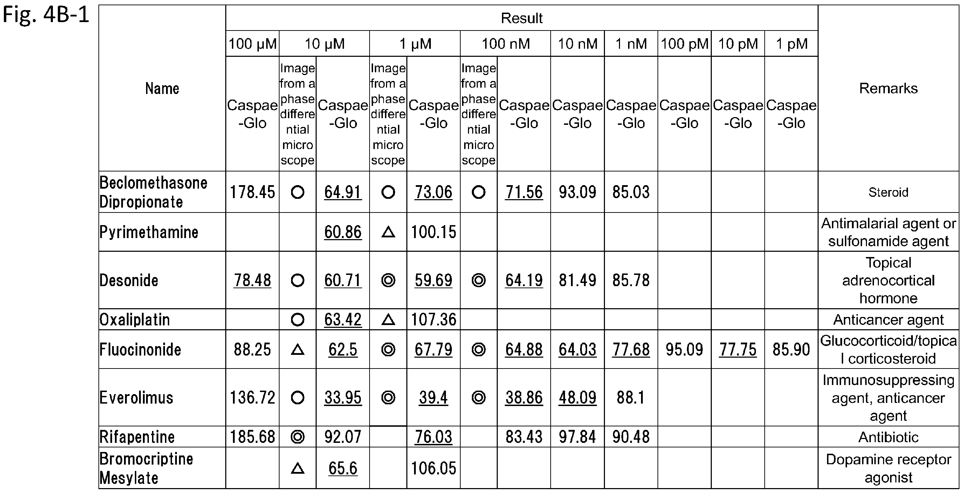

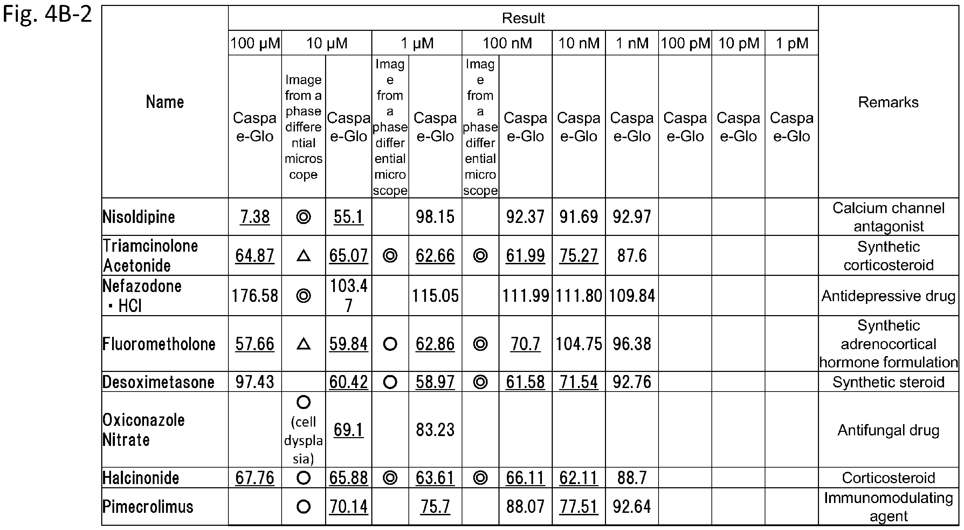

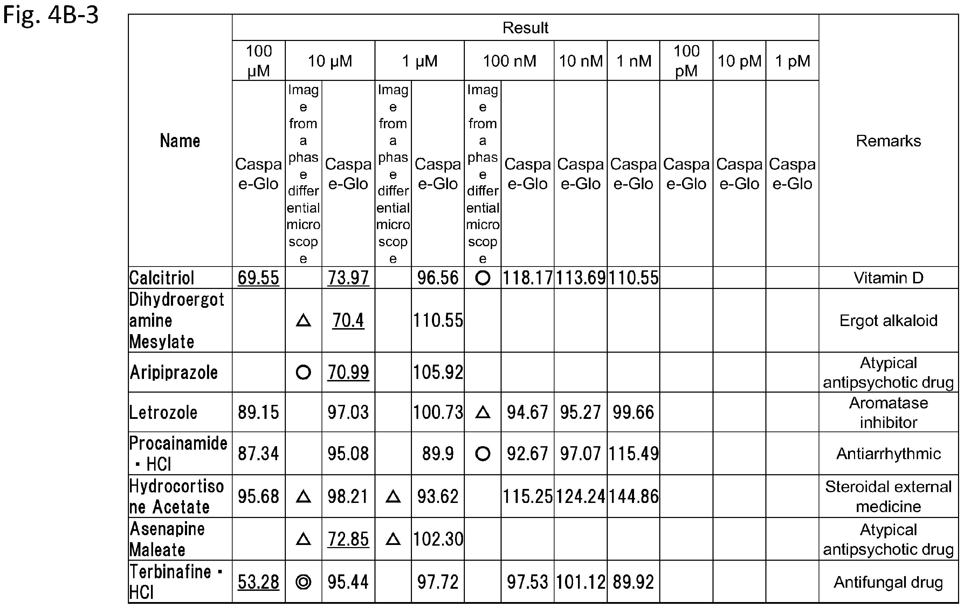

[Figure 4B-1] Figure **4B-1****,** Figure **4B-2****,** and Figure **4B-3** show caspase 3/7 activity of each agent at various concentrations under TGF-β2 stimulation. "Caspase-Glo" shows caspase 3/7 activity measured by Caspase-Glo® 3/7 Assay. In "phase contrast microscope", ⊚ indicates free cell ratio of 1 or more and less than 1.2, ○ indicates free cell ratio of 1.2 or more and less than 1.4, and Δ indicates free cell ratio of 1.4 or more and less than 1.5 when free cells of a control are assumed to be "1" in observation using a phase contrast microscope.
[Figure 4B-2] Figure **4B-1****,** Figure **4B-2****,** and Figure **4B-3** show caspase 3/7 activity of each agent at various concentrations under TGF-β2 stimulation. "Caspase-Glo" shows caspase 3/7 activity measured by Caspase-Glo® 3/7 Assay. In "phase contrast microscope", ⊚ indicates free cell ratio of 1 or more and less than 1.2, ○ indicates free cell ratio of 1.2 or more and less than 1.4, and Δ indicates free cell ratio of 1.4 or more and less than 1.5 when free cells of a control are assumed to be "1" in observation using a phase contrast microscope.
[Figure 4B-3] Figure **4B-1****,** Figure **4B-2****,** and Figure **4B-3** show caspase 3/7 activity of each agent at various concentrations under TGF-β2 stimulation. "Caspase-Glo" shows caspase 3/7 activity measured by Caspase-Glo® 3/7 Assay. In "phase contrast microscope", ⊚ indicates free cell ratio of 1 or more and less than 1.2, ○ indicates free cell ratio of 1.2 or more and less than 1.4, and Δ indicates free cell ratio of 1.4 or more and less than 1.5 when free cells of a control are assumed to be "1" in observation using a phase contrast microscope.

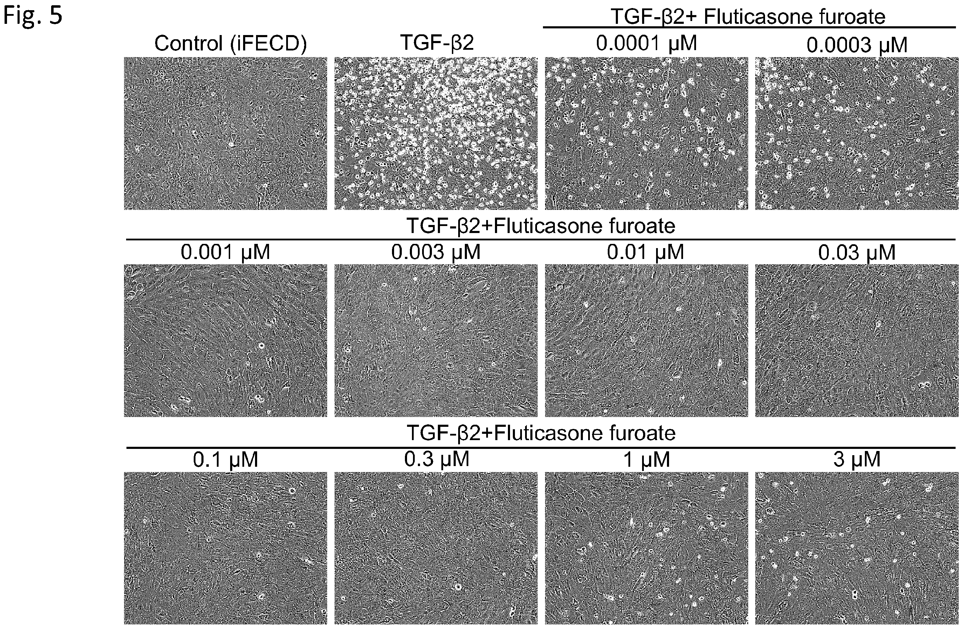

[Figure 5] Figure **5** shows pictures from a phase contrast microscope of immortalized human corneal endothelial cells after stimulating Fuchs' endothelial corneal dystrophy patient derived immortalized corneal endothelial cells, which were pretreated with fluticasone furoate, with TGF-β2.

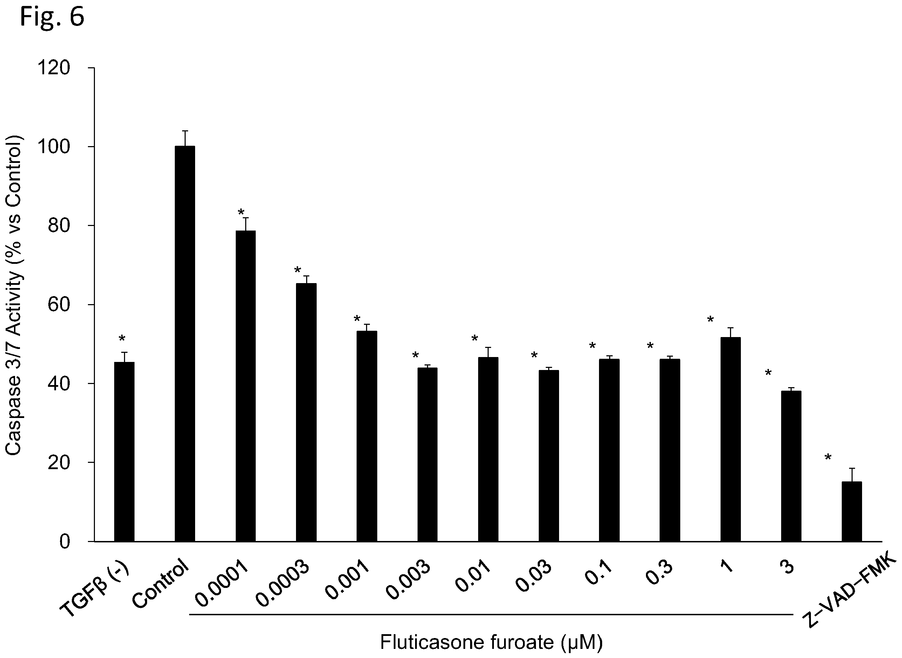

[Figure 6] Figure **6** shows a graph of caspase 3/7 activity in Fuchs' endothelial corneal dystrophy patient derived immortalized corneal endothelial cells in the presence of fluticasone furoate.

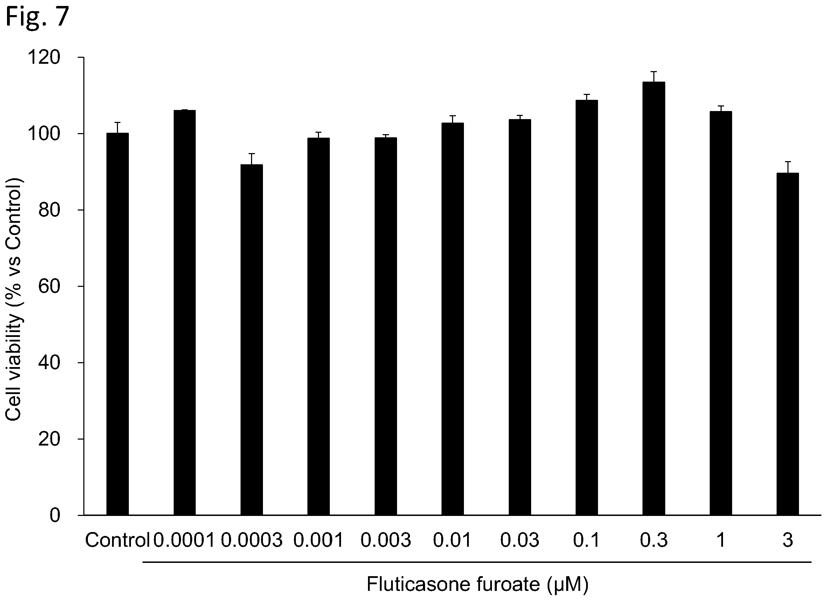

[Figure 7] Figure **7** shows a graph of the cell viability in Fuchs' endothelial corneal dystrophy patient derived immortalized corneal endothelial cells in the presence of fluticasone furoate.

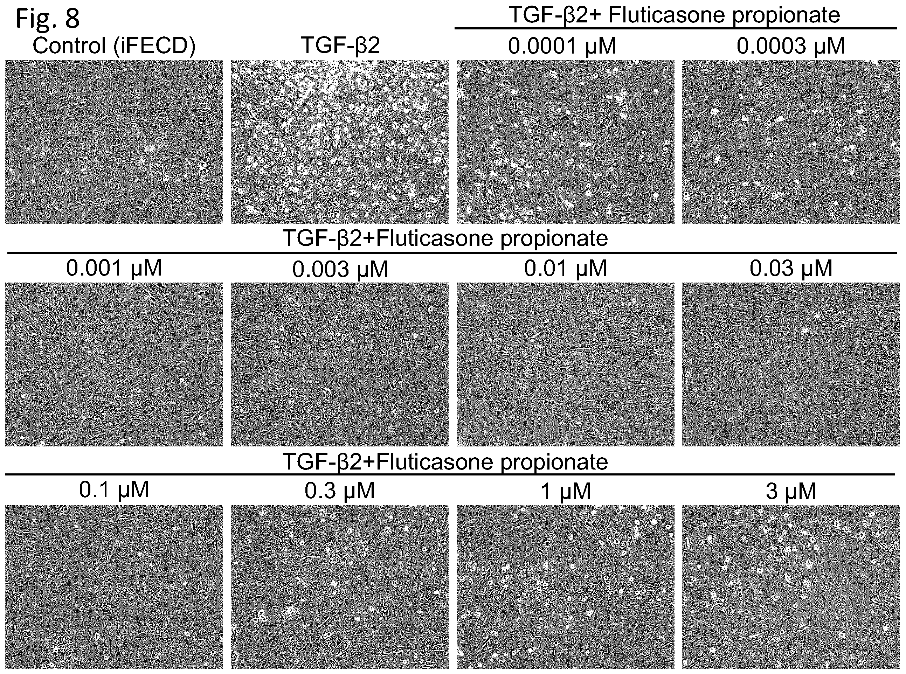

[Figure 8] Figure **8** shows pictures from a phase contrast microscope of immortalized human corneal endothelial cells after stimulating Fuchs' endothelial corneal dystrophy patient derived immortalized corneal endothelial cells, which were pretreated with fluticasone propionate, with TGF-β2.

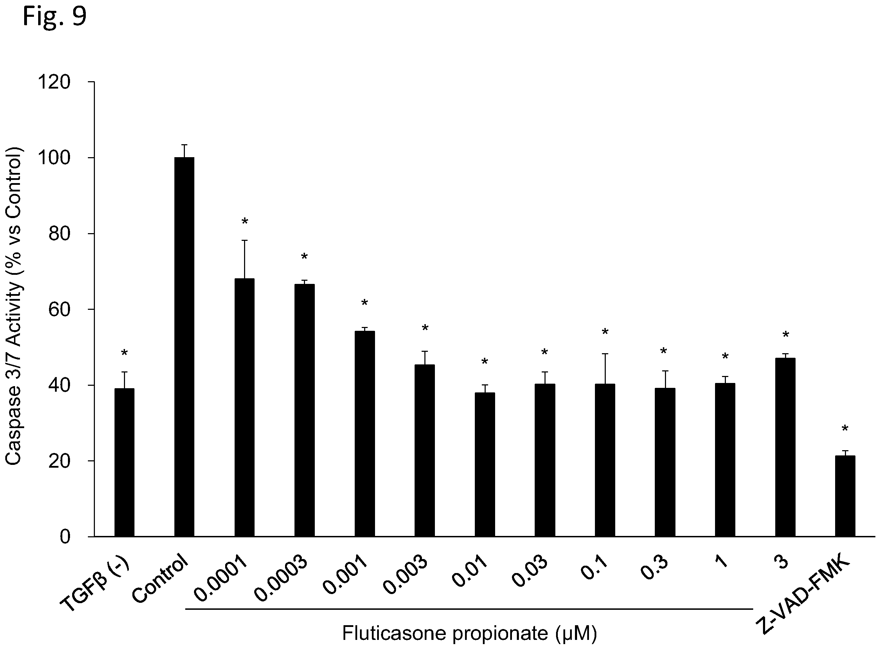

[Figure 9] Figure **9** shows a graph of caspase 3/7 activity in Fuchs' endothelial corneal dystrophy patient derived immortalized corneal endothelial cells in the presence of fluticasone propionate.

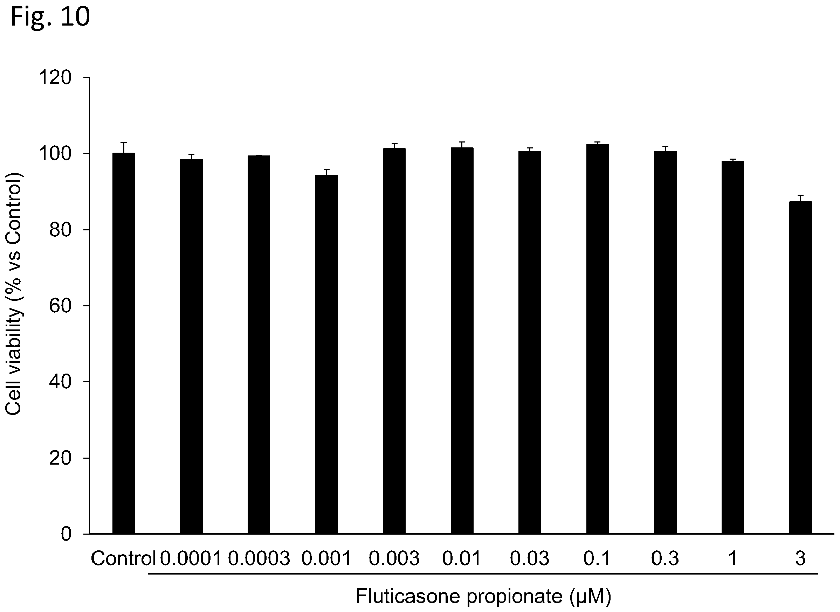

[Figure 10] Figure **10** shows a graph of the cell viability in Fuchs' endothelial corneal dystrophy patient derived immortalized corneal endothelial cells in the presence of fluticasone propionate.

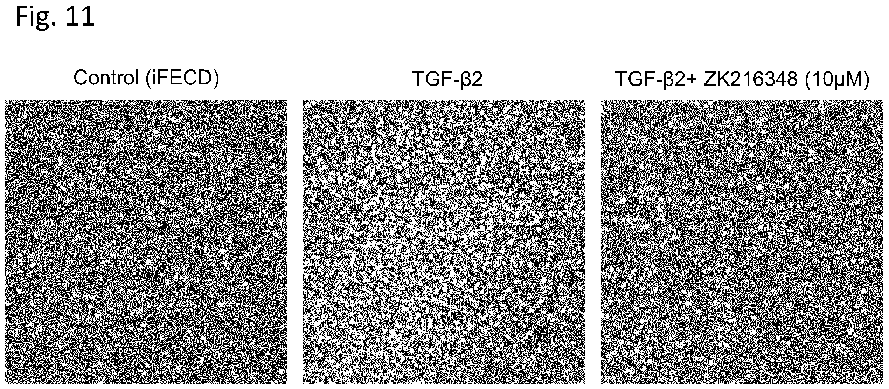

[Figure 11] Figure **11** shows pictures from a phase contrast microscope of immortalized human corneal endothelial cells after stimulating Fuchs' endothelial corneal dystrophy patient derived immortalized corneal endothelial cells, which were pretreated with ZK216348, with TGF-β2.
[Figure 12] Figure **12** shows the ratio of (caspase 3/7 activity)/(cell viability) when fluticasone furoate and fluticasone propionate are used.

### [Description of Embodiments]

The present invention is explained hereinafter. Throughout the entire specification, a singular expression should be understood as encompassing the concept thereof in the plural form, unless specifically noted otherwise. Thus, singular articles (e.g., "a", "an", "the", and the like in the case of English) should also be understood as encompassing the concept thereof in the plural form, unless specifically noted otherwise. Further, the terms used herein should be understood as being used in the meaning that is commonly used in the art, unless specifically noted otherwise. Thus, unless defined otherwise, all terminologies and scientific technical terms that are used herein have the same meaning as the general understanding of those skilled in the art to which the present invention pertains. In case of a contradiction, the present specification (including the definitions) takes precedence.

### (Definition)

As used herein, "about" before a numerical value means ±10% of a numerical value that follows.

As used herein, "cell viability (%)" refers to the ratio of the cell count when iFCED or iHCEC are cultured in the presence of a compound used in the present invention to the cell count when iFCED or iHCEC are cultured in the absence of said compound. The cell viability as used herein was measured by CellTiter-Glo® Luminescent Cell Viability Assay unless specifically instructed otherwise.

As used herein, "caspase 3/7 activity (%)" refers to the ratio of caspase 3/7 activity when iFCED or iHCEC are contacted with a compound used in the present invention in the presence of TGF-β (e.g., TGF-β2) or MG-132 to caspase 3/7 activity in a TGF-β (e.g., TGF-β2) supplemented group (no compound) or MG-132 supplemented group (no compound). Caspase 3/7 activity as used herein was measured by Caspase-Glo® 3/7 Assay unless specifically instructed otherwise.

As used herein, a "compound" encompasses any substance that can achieve prevention or treatment of a condition, disorder, or disease in a corneal endothelium, which includes, for example, a small molecule compound, peptide, protein, nucleic acid, cell, and the like.

As used herein, a "derivative" or an "analogue" refers to a compound which has a core structure that is the same as or very similar to that of a parent compound but has a chemical or physical modification such as a different functional group or an additional functional group. A derivative or an analogue has biological activity that is the same as or similar to that of a parent compound.

As used herein, a "pharmaceutically acceptable salt" refers to a salt of the compound of the present invention (e.g., acidic salt, basic salt, and the like) which is relatively non-toxic. These salts can be temporarily prepared during the final isolation and purification of a compound, or can be prepared by causing a compound purified by the free acid or free base form to individually react with a suitable organic or inorganic base or acid, and isolating a salt formed in such a manner.

A pharmaceutically acceptable basic salt of the compound of the present invention includes, for example: alkali metal salt such as sodium salt or potassium salt; alkaline earth metal salt such as calcium salt or magnesium salt; ammonium salt; aliphatic amine salt such as trimethylamine salt, triethylamine salt, dicyclohexylamine salt, ethanolamine salt, diethanolamine salt, triethanolamine salt, procaine salt, meglumine salt, diethanolamine salt or ethylenediamine salt; aralkylamine salt such as N,N-dibenzylethylenediamine and benethamine salt; heterocyclic aromatic amine salt such as pyridine salt, picoline salt, quinoline salt, or isoquinoline salt; quaternary ammonium salt such as tetramethylammonium salt, tetraethylammonium salt, benzyltrimethylammonium salt, benzyltriethylammonium salt, benzyltributylammonium salt, methyltrioctylammonium salt, or tetrabutylammonium salt; basic amino acid salt such as arginine salt or lysine salt, and the like.

A pharmaceutically acceptable acidic salt of the compound of the present invention includes, for example: inorganic acid salt such as hydrochloride salt, sulfuric acid salt, nitric acid salt, phosphoric acid salt, carbonic acid salt, hydrogencarbonate salt, or perchloric acid salt; organic acid salt such as acetic acid salt, propionic acid salt, lactic acid salt, maleic acid salt, fumaric acid salt, tartaric acid salt, malic acid salt, citric acid salt, or ascorbic acid salt; sulfonic acid salt such as methanesulfonic acid salt, isethionic acid salt, benzenesulfonic acid salt, or p-Toluenesulfonic acid salt; acidic amino acid such as aspartic acid salt or glutamic acid salt, and the like.

As used herein, a "solvate" means a solvate of the compound of the present invention or a pharmaceutically acceptable salt thereof, and encompasses, for example, a solvate with an organic solvent (e.g., solvate with alcohol (such as ethanol)), hydrate and the like. When a hydrate is formed, the hydrate may be coordinated with any number of water molecules. A hydrate can include monohydrate, dihydrate and the like.

As used herein, "inhibiting activity against TGFβ" or "inhibiting activity against endoplasmic reticulum associated stress inducing substance" refers to activity of inhibiting apoptosis of cells due to TGFβ/endoplasmic reticulum associated stress inducing substance. "Having inhibiting activity against TGFβ" or "having inhibiting activity against endoplasmic reticulum associated stress inducing substance" refers to having caspase activity which was substantially decreased compared to a group in which cells (iFCED or iHCEC or the like) have not been contacted with a candidate compound (candidate compound non-contacted group) .

As used herein, "less toxic" refers to that the cell viability is not substantially decreased in the absence of TGF-β and endoplasmic reticulum associated stress inducing substance. "Cell viability that is not substantially decreased" refers to that the cell viability has not significantly decreased compared to a group in which cells (iFCED or iHCEC or the like) have not been contacted with a candidate compound (candidate compound non-contacted group). For example, a numerical value used as a criterion can include decrease by 10% or less, decrease by 5% or less, and the like.

As used herein, an "anti-inflammatory drug" refers to active substance having a property of suppressing inflammation. A steroidal anti-inflammatory drug is known as an anti-inflammatory drug. Steroid anti-inflammatory drugs are generally called "steroid" as well. As used herein, a "steroid" and "steroidal anti-inflammatory drug" are interchangeably used. A steroidal anti-inflammatory drug has a steroid nucleus called cyclopentahydrophenanthrene as a basic skeleton, and is a compound having an anti-inflammatory action. An anti-inflammatory drug includes a nonsteroidal anti-inflammatory drug (NSAID) in addition to a steroidal anti-inflammatory drug. A nonsteroidal anti-inflammatory drug (NSAID) is a compound with an anti-inflammatory action having various structures that do not have a skeleton as in a steroid.

As used herein, "Selective Glucocorticoid Receptor Agonist (SEGRA)" refers to a compound that selectively binds to a glucocorticoid receptor and exhibits the same action as the physiological action of the ligand of the receptor. As used herein, SEGRA only refers to a selective glucocorticoid receptor agonist of which basic skeleton is a steroid. Thus, "SEGRA" as used herein is also called "steroidal SEGRA", in which the former is synonymous with the latter. SEGRA that can be used herein can include, for example, fluticasone (e.g., fluticasone propionate or fluticasone furoate), flumethasone, RU-24858 and the like.

As used herein, "Selective Glucocorticoid Receptor Modulator (SEGRM)" refers to a nonsteroidal compound among the compounds that selectively bind to a glucocorticoid receptor and exhibit the same action as the physiological action of the ligand of the receptor. As used herein, SEGRM only refers to a selective glucocorticoid receptor modulator of which basic skeleton is nonsteroidal. Thus, "SEGRM" as used herein is also called "nonsteroidal SEGRM", in which the former is synonymous with the latter. SEGRM that can be used herein can include, for example, Mapracorat (ZK245186), ZK216348, ZK209614, Dagrocorat, Fosdagrocorat, Compound A, AL-438, LGD-5552, C108297, MK-5932, Org 214007-0, PF-802, DE-110, Compound 10 and the like.

SEGRA was historically named as a compound induced from a steroid skeleton. After that, a compound having the same activity as that of SEGRA and having no steroid skeleton was discovered, and such a nonsteroidal compound was named "Selective Glucocorticoid Receptor Modulator (SEGRM)" in order to distinguish it from SEGRA, which was originally named for a selective glucocorticoid receptor agonist having a steroid skeleton (Sundahl et al., Pharmacol Ther. 2015 Aug;152:28-41). As used herein, the name "nonsteroidal SEGRA" is not used, and all nonsteroidal compounds as described above are called "SEGRM" according to the above description. On the contrary, the name "steroidal SEGRM" is also not used, and all steroidal compounds as described above are called "SEGRA".

As used herein, a "steroidal" compound refers to a compound having a steroid nucleus called cyclopentahydrophenanthrene as a basic skeleton. In such a compound, any group such as functional group may be bound to the steroid nucleus. Those skilled in the art can readily judge whether a compound is a steroidal compound or a nonsteroidal compound based on the structure of the compound.

As used herein, a "vitamin B12 group" encompasses hydroxocobalamin, cyanocobalamin, derivatives thereof (such as mecobalamin or deoxyadenosylcobalamin), and pharmaceutical salts thereof.

As used herein, a "vitamin D group" encompasses vitamin D and a derivative or an analogue having similar activity to that of vitamin D. A derivative or an analogue of vitamin D is described in detail in Japanese National Phase PCT Laid-Open Publication No. 2013-518812, Japanese National Phase PCT Laid-Open Publication No. 2013-515018 and the like that are incorporated by reference herein.

It is preferable that a compound used in the present invention is water-soluble. This is because, if a compound used in the present invention is not water-soluble, it may be necessary to use a solvent that is less likely to be compatible with the body. Water-solubility can be classified based on the definition of solubility in the pharmacopoeia. In other words, the amount of solvent required to dissolve 1 g or 1 mL of solute is defined as extremely readily dissolvable: less than 1 mL; readily dissolvable: 1 mL or greater and less than 10 mL; somewhat readily dissolvable: 10 mL or greater and less than 30 mL; somewhat difficult to dissolve: 30 mL or greater and less than 100 mL; difficult to dissolve: 100 mL or greater and less than 1000 mL; very difficult to dissolve: 1000 mL or greater and less than 10000 mL; and hardly dissolvable: 10000 mL or greater. Solubility is similarly assessed herein. Water solubility is understood to mean that a substance with any solubility can be used, as long as an effective amount thereof can be dissolved when water is used as a solvent.

As used herein, "iFECD" (immortalized Fuchs' endothelial corneal dystrophy) is an abbreviation for immortalized Fuchs' endothelial corneal dystrophy cells. The manufacturing method of iFECD is described for example in WO 2015/015655.

As used herein, "HCEC" (human cornel endothelial cells) is an abbreviation for human corneal endothelial cells. In addition, "iHCEC" is an abbreviation for immortalized human corneal endothelial cells.

As used herein, "programmed cell death" refers to a phenomenon of cells spontaneously dying at a determined time or environment as if the death is pre-programmed. Programmed cell death is used in the meaning that includes, for example, "apoptosis".

As used herein, "transforming growth factor-β (also denoted with the abbreviation TGF-β)" is used in the same meaning as those used in the art. It is a homodimer multifunctional cytokine with a molecular weight of 25 kD exhibiting a variety of biological activity, such as being responsible for pathogenesis of various sclerotic diseases, rheumatoid arthritis, and proliferative vitreoretinopathy, being deeply involved in hair loss, suppressing the functioning of immunocompetent cells while suppressing overproduction of protease to prevent degradation of pulmonary tissue resulting in pulmonary emphysema, and suppressing cancer cell growth. "TGF-β signal" refers to a signal mediated by TGF-β, which is elicited by TGF-β. Examples of TGF-β signals include signals mediated by TGF-β2 in addition to signals mediated by TGF-β1, TGF-β3 or the like. In humans, TGF-β has three isoforms, TGF-β1 to β3, which have homology of about 70% and similar action. TGF-β is produced as an inactive latent form with a molecular weight of about 300 kD which is unable to bind to a receptor. The action thereof is exerted by being activated on a target cell surface or in the surroundings thereof to become an active form that can bind to a receptor. Although not wishing to be bound by any theory, the action of TGF-β in a target cell is understood to be transmitted by a phosphorylation channel of a series of proteins responsible for transmitting information called Smad. First, when activated TGF-β binds to a TGF-β type II receptor on a target cell surface, a receptor complex consisting of two molecules of type II receptors and two molecules of TGF-β type I receptors is formed, and the type II receptors phosphorylate the type I receptors. It is understood that when the phosphorylated type I receptors phosphorylate Smad2 or Smad3, the phosphorylated Smad2 or Smad3 forms a complex with Smad4, which migrates to a nucleus and binds to a target sequence called CAGA box that is present in a target gene promotor region to induce transcription and expression of a target gene with a coactivator.

A transforming growth factor-β (TGF-β) signaling pathway can modulate many cellular activities, such as cell growth and differentiation, growth arrest, programmed cell death (apoptosis), and epithelial mesenchymal transition (EMT), by modulating the target gene. Members of the TGF-β family including TGF-β itself (e.g., TGF-β1, TGF-β2, and TGF-β3), activin, and bone morphogenetic proteins (BMP) are potent modulators of cell growth, differentiation, migration, programmed cell death (apoptosis), and the like.

TGF-β is a protein of about 24 Kd produced by many cells including B lymphocytes, T lymphocytes, and activated macrophages and by many other cell types. Effects of TGF-β on the immune system include IL-2 receptor induction, inhibition of IL-1 induced thymocyte growth, and blocking of IFN-γ induced macrophage activation. TGF-β is considered to be involved in various pathological conditions (Border et al. (1992) J. Clin. Invest. 90:1) and is thoroughly proven to function as either a tumor suppressing substance or a tumor promotor.

Signaling of TGF-β is mediated by two serine/threonine kinase cell surface receptors TGF-βRII and ALK5. TGF-β signaling is initiated by ligand induced receptor dimerization enabling TGF-βRII to phosphorylate an ALK5 receptor. The phosphorylation activates ALK5 kinase activity, and the activated ALK5 then phosphorylates a downstream effector Smad protein (vertebrate homologue of MAD or "Mothers against DPP (decapentaplegic)" protein), Smad2 or Smad3. A p-Smad2/3 complex with Smad4 enters a nucleus and activates transcription of a target gene.

Smad3 is a member of the R-Smad (receptor-activated Smad) subgroup of Smad and a direct mediator of transcription activation by a TGF-β receptor. A TGF-β stimulation results in phosphorylation and activation of Smad2 and Smad3, which form a complex with Smad4 ("common Smad" or "co-Smad" in vertebrates). This accumulates with the nucleus and modulates transcription of a target gene. R-Smad is localized in a cytoplasm and forms a complex with co-Smad through ligand induced phosphorylation by a TGF-β receptor, migrates to the nucleus, where it modulates gene expression associated with a cooperative transcription factor and chromatin. Smad6 and Smad7 are inhibitory Smad ("I-Smad"), i.e., they are transcriptionally induced by TGF-β and function as a TGF-β signaling inhibitor (Feng et al. (2005) Annu. Rev. Cell. Dev. Biol. 21: 659). Smad6/7 obstruct receptor-mediated activation of R-Smad to exert their inhibitory effect; and they are associated with a type I receptor, which competitively obstructs mobilization and phosphorylation of R-Smad. Smad6 and Smad7 are known to replenish E3 ubiquitin ligase, which induces ubiquitination and degradation of Smad6/7 interacting proteins.

TGF-β signaling pathways further have other pathways using BMP-7 transmission or the like, which go through ALK-1/2/3/6 via Smad1/5/8 to express a function. For TGF-β signaling pathways, see J. Massagu'e, Annu. Rev. Biochem. 1998. 67: 753-91; Vilar JMG, Jansen R, Sander C (2006) PLoS Comput Biol 2 (1):e3; Leask, A., Abraham, D. J. FASEB J. 18, 816-827 (2004); Coert Margadant & Arnoud Sonnenberg EMBO reports (2010) 11, 97-105; Joel Rosenbloom et al., Ann Intern Med. 2010; 152: 159-166 and the like.

As used herein, a "corneal endothelial condition, disorder, or disease due to transforming growth factor-β (TGF-β)" refers to any corneal endothelial condition, disorder, or disease induced by TGF-β in corneal endothelial cells. In the present invention, exposure of corneal endothelial cells such as model cells of Fuchs' endothelial corneal dystrophy (e.g., iFECD) to TGF-β2 surprisingly resulted in various disorders (e.g., programmed cell death) . This is a phenomenon that had not been well understood conventionally. The inventors discovered a compound that can suppress caspase 3/7 activity in cells of a corneal endothelial disorder model of Fuchs' endothelial corneal dystrophy in the presence of TGF-β, using caspase 3/7 activity as an indicator of a disorder in a corneal endothelium. Surprisingly, it had not been known that the discovered compound suppresses a disorder in a corneal endothelium or suppresses caspase activity.

As used herein, a "corneal endothelial condition, disorder, or disease due to endoplasmic reticulum (ER) associated stress" refers to any condition, disorder, or disease associated with endoplasmic reticulum (ER) stress. Examples thereof can include, but are not limited to, conditions, disorders, or diseases associated with endoplasmic reticulum (ER) stress among damage to corneal endothelial cells in Fuchs' endothelial corneal dystrophy, corneal endothelial disorder, decreased corneal endothelial density, guttae formation, hypertrophy of the Descemet's membrane, hypertrophy of a cornea, turbidity, corneal epithelial disorder, turbidity in corneal stroma, photophobia, blurred vision, visual impairment, ophthalmalgia, epiphora, hyperemia, pain, bullous keratopathy, eye discomfort, diminished contrast, glare, edema of the corneal stroma, corneal epithelial erosion, angiogenesis and the like.

The definition of the terms (atom, group and the like) used herein is explained in detail below.

As used herein, a "halogen atom" refers to a fluorine atom, a chlorine atom, a bromine atom, or an iodine atom.

As used herein, a "lower alkyl group" refers to a straight chain or a branched alkyl group having 1 to 8 carbon atoms. Specific examples include a methyl group, an ethyl group, an n-propyl group, an n-butyl group, an n-pentyl group, an n-hexyl group, an n-heptyl group, an n-octyl group, an isopropyl group, an isobutyl group, a sec-butyl group, a tert-butyl group, an isopentyl group and the like.

As used herein, a "lower alkenyl group" refers to a straight chain or a branched alkenyl group having 2 to 8 carbon atoms. Specific examples include a vinyl group, a propenyl group, a butenyl group, a pentenyl group, a hexenyl group, a heptenyl group, an octenyl group, an isopropenyl group, a 2-methyl-1-propenyl group, a 2-methyl-2-butenyl group and the like.

As used herein, a "lower alkynyl group" refers to a straight chain or a branched alkynyl group having 2 to 8 carbon atoms. Specific examples include an ethynyl group, a propynyl group, a butynyl group, a pentynyl group, a hexynyl group, a heptynyl group, an octynyl group, an isobutynyl group, an isopentynyl group and the like.

A "lower cycloalkyl group" refers to a cycloalkyl group having 3 to 8 carbon atoms. Specific examples include a cyclopropyl group, a cyclobutyl group, a cyclopentyl group, a cyclohexyl group, a cycloheptyl group, or a cyclooctyl group.

As used herein, a "lower cycloalkane ring" refers to a cycloalkane ring having 3 to 8 carbon atoms. Specific examples include a cyclopropane ring, a cyclobutane ring, a cyclopentane ring, a cyclohexane ring, a cycloheptane ring, or a cyclooctane ring.

As used herein, an "aryl group" refers to a residue in which one hydrogen atom has been removed from a condensed polycyclic aromatic hydrocarbon which is bicyclic or tricyclic or a monocyclic aromatic hydrocarbon having 6 to 14 carbon atoms. Specific examples include a phenyl group, a naphtyl group, an anthryl group, a phenanthryl group and the like.

As used herein, a "heterocyclic group" refers to a residue in which one hydrogen atom has been removed from a saturated or unsaturated monocyclic heterocycle or a condensed polycyclic aromatic hydrocarbon which is bicyclic or tricyclic having one or more hetero atoms selected from a nitrogen atom, an oxygen atom, and a sulfur atom in the ring.

Specific examples of a saturated monocyclic heterocycle include a pyrrolidine ring, a pyrazolidine ring, an imidazolidine ring, a triazolidine ring, a piperidine ring, a hexahydropyridazine ring, a hexahydropyrimidine ring, a piperazine ring, a homopiperidine ring, a homopiperazine ring and the like having a nitrogen atom in the ring, tetrahydrofuran ring, a tetrahydropyran ring and the like having an oxygen atom in the ring, a tetrahydrothiophene ring, a tetrahydrothiopyran ring and the like having a sulfur atom in the ring, an oxazolidine ring, an isoxazoline ring, a morpholine ring and the like having a nitrogen atom and an oxygen atom in the ring, a thiazolidine ring, an isothiazolidine ring, a thiomorpholine ring and the like having a nitrogen atom and a sulfur atom in the ring.

Further, a saturated monocyclic heterocycle may be condensed with a benzene ring or the like to form a condensed polycyclic aromatic hydrocarbon which is bicyclic or tricyclic such as a dihydroindole ring, a dihydroindazole ring, a dihydrobenzimidazole ring, a tetrahydroquinoline ring, a tetrahydroisoquinoline ring, a tetrahydrocinnoline ring, a tetrahydrophthalazine ring, a tetrahydroquinazoline ring, a tetrahydroquinoxaline ring, a dihydrobenzofuran ring, a dihydroisobenzofuran ring, a chromane ring, an isochromane ring, a dihydrobenzothiophene ring, a dihydroisobenzothiophene ring, a thiochromane ring, an isothiochromane ring, an dihydrobenzoxazole ring, a dihydrobenzisoxazole ring, a dihydrobenzoxazine ring, a dihydrobenzothiazole ring, a dihydrobenzisothiazole ring, a dihydrobenzothiazine ring, a xanthene ring, a 4a-carbazole ring, or a perimidine ring.

Specific examples of an unsaturated monocyclic heterocycle include a dihydropyrrole ring, a pyrrole ring, a dihydropyrazole ring, a pyrazole ring, a dihydroimidazole ring, an imidazole ring, a dihydrotriazole ring, a triazole ring, a tetrahydropyridine ring, a dihydropyridine ring, a pyridine ring, a tetrahydropyridazine ring, a dihydropyridazine ring, a pyridazine ring, a tetrahydropyrimidine ring, a dihydropyrimidine ring, a pyrimidine ring, a tetrahydropyrazine ring, a dihydropyrazine ring, a pyrazine ring and the like having a nitrogen atom in the ring, a dihydrofuran ring, a furan ring, a dihydropyran ring, a pyran ring and the like having an oxygen atom in the ring, a dihydrothiophen ring, a thiophen ring, a dihydrothiopyran ring, a thiopyran ring and the like having a sulfur atom in the ring, a dihydrooxazole ring, an oxazole ring, a dihydroisoxazole ring, an isoxazole ring, a dihydrooxazine ring, an oxazine ring and the like having a nitrogen atom and an oxygen atom in the ring, a dihydrothiazole ring, a thiazole ring, a dihydroisothiazole ring, an isothiazole ring, a dihydrothiazine ring, a thiazine ring and the like having a nitrogen atom and a sulfur atom in the ring.

Further, an unsaturated monocyclic heterocycle may be condensed with a benzene ring or the like to form a condensed polycyclic aromatic hydrocarbon which is bicyclic or tricyclic such as an indole ring, an indazole ring, a benzoimidazole ring, a benzotriazole ring, a dihydroquinoline ring, a quinoline ring, a dihydroisoquinoline ring, an isoquinoline ring, a phenanthridine ring, a dihydrocinnoline ring, a cinnoline ring, a dihydrophthalazine ring, a phthalazine ring, a dihydroquinazoline ring, a quinazoline ring, a dihydroquinoxaline ring, a quinoxaline ring, a benzofuran ring, an isobenzofuran ring, a chromene ring, an isochromene ring, a benzothiophen ring, an isobenzothiophen ring, a thiochromene ring, an isothiochromene ring, a benzoxazole ring, a benzisoxazole ring, a benzoxazine ring, a benzothiazole ring, a benzisothiazole ring, a benzothiazine ring, a phenoxanthine ring, a carbazole ring, a β-carboline ring, a phenanthridine ring, an acridine ring, a phenanthroline ring, a phenazine ring, a phenothiazine ring, or a phenoxazine ring.

As used herein, a "lower alkoxy group" refers to a group in which a hydrogen atom of a hydroxy group has been substituted with a lower alkyl group. Specific examples include a methoxy group, an ethoxy group, an n-propoxy group, an n-butoxy group, an n-pentoxy group, an n-hexyloxy group, an n-heptyloxy group, an n-octyloxy group, an isopropoxy group, an isobutoxy group, a sec-butoxy group, a tert-butoxy group, an isopentoxy group and the like.

As used herein, a "lower alkenyloxy group" refers to a group in which a hydrogen atom of a hydroxy group has been substituted with a lower alkenyl group. Specific examples include a vinyloxy group, a propenyloxy group, a butenyloxy group, a pentenyloxy group, a hexenyloxy group, heptenyloxy group, an octenyloxy group, an isopropenyloxy group, a 2-methyl-1-propenyloxy group, a 2-methyl-2-butenyloxy group and the like.

As used herein, a "lower alkynyloxy group" refers to a group in which a hydrogen atom of a hydroxy group has been substituted with a lower alkynyl group. Specific examples include an ethynyloxy group, a propynyloxy group, a butynyloxy group, a pentynyloxy group, a hexynyloxy group, a heptynyloxy group, an octynyloxy group, an isobutynyloxy group, an isopentynyloxy group and the like.

As used herein, a "lower cycloalkyloxy group" refers to a group in which a hydrogen atom of a hydroxy group has been substituted with a lower cycloalkyl group. Specific examples include a cyclopropyloxy group, a cyclobutyloxy group, a cyclopentyloxy group, a cyclohexyloxy group, cycloheptyloxy group, a cyclooctyloxy group and the like.

As used herein, an "aryloxy group" refers to a group in which a hydrogen atom of a hydroxy group has been substituted with an aryl group. Specific examples include a phenoxy group, a naphthoxy group, an anthryloxy group, a phenanthryloxy group and the like.

A "heterocyclic oxy group" refers to a group in which a hydrogen atom of a hydroxy group has been substituted with a heterocyclic group.

A "lower alkylthio group" refers to a group in which a hydrogen atom of a mercapto group has been substituted with a lower alkyl group. Specific examples include a methylthio group, an ethylthio group, an n-propylthio group, an n-butylthio group, an n-pentylthio group, an n-hexylthio group, an n-heptylthio group, an n-octylthio group, an isopropylthio group, an isobutylthio group, a sec-butylthio group, a tert-butylthio group, an isopentylthio group and the like.

As used herein, a "lower cycloalkylthio group" refers to a group in which a hydrogen atom of a mercapto group has been substituted with a lower cycloalkyl group. Specific examples include a cyclopropylthio group, a cyclobutylthio group, a cyclopentylthio group, a cyclohexylthio group, a cycloheptylthio group, or a cyclooctylthio group.

As used herein, an "arylthio group" refers to a group in which a hydrogen atom of a mercapto group has been substituted with an aryl group. Specific examples include a phenylthio group, a naphtylthio group, an anthrylthio group, a phenanthrylthio group and the like.

As used herein, a "heterocyclic thio group" refers to a group in which a hydrogen atom of a mercapto group has been substituted with a heterocyclic group.

As used herein, a "lower alkylamino group" refers to a group in which one of the hydrogen atoms of an amino group has been substituted with a lower alkyl group or both of the hydrogen atoms of an amino group have been substituted with a lower alkyl group. Specific examples include a methylamino group, an ethylamino group, a propylamino group, a dimetylamino group, a diethylamino group, an ethyl(methyl)amino group and the like.

As used herein, a "lower cycloalkylamino group" refers to a group in which one of the hydrogen atoms of an amino group has been substituted with a lower cycloalkyl group or both of the hydrogen atoms of an amino group have been substituted with a lower cycloalkyl group, or a group in which one of the hydrogen atoms of an amino group has been substituted with a lower cycloalkyl group and the other hydrogen atom has been substituted with a lower alkyl group, a lower alkenyl group, or a lower alkynyl group. Specific examples include a cyclopropylamino group, a cyclobutylamino group, a cyclopentylamino group, a cyclohexylamino group, a cycloheptylamino group, a cyclooctylamino group, a dicyclohexyl group, a cyclohexyl(methyl)amino group, a cyclohexyl(vinyl)amino group, a cyclohexyl(ethynyl)amino group and the like.

An "arylamino group" refers to a group in which one of the hydrogen atoms of an amino group has been substituted with an aryl group or both of the hydrogen atoms of an amino group have been substituted with an aryl group, or a group in which one of the hydrogen atoms of an amino group has been substituted with an aryl group and the other hydrogen atom has been substituted with a lower alkyl group, a lower alkenyl group, a lower alkynyl group, or a lower cycloalkyl group. Specific examples include a phenylamino group, a naphtylamino group, an anthrylamino group, a phenanthrylamino group, a diphenylamino group, a methyl(phenyl)amino group, an ethyl(phenyl)amino group, a phenyl(vinyl)amino group, an ethynyl(phenyl)amino group, a cyclohexyl(phenyl)amino group and the like.

As used herein, a "heterocyclic amino group" refers to a group in which one of the hydrogen atoms of an amino group has been substituted with a heterocyclic group or both of the hydrogen atoms of an amino group have been substituted with a heterocyclic group, or a group in which one of the hydrogen atoms of an amino group has been substituted with a heterocyclic group and the other hydrogen atom has been substituted with a lower alkyl group or a lower alkenyl group, a lower alkynyl group, a lower cycloalkyl group, or an aryl group.

As used herein, a "lower alkylcarbonyl group" refers to a group in which a hydrogen atom of a formyl group has been substituted with a lower alkyl group. Specific examples include a methylcarbonyl group, an ethylcarbonyl group, an n-propylcarbonyl group, an n-butylcarbonyl group, an n-pentylcarbonyl group, an n-hexylcarbonyl group, an n-heptylcarbonyl group, an n-octylcarbonyl group, an isopropylcarbonyl group, an isobutylcarbonyl group, a sec-butylcarbonyl group, a tert-butylcarbonyl group, an isopentylcarbonyl group and the like.

As used herein, a "lower alkenylcarbonyl group" refers to a group in which a hydrogen atom of a formyl group has been substituted with a lower alkenyl group. Specific examples include a vinylcarbonyl group, a propenylcarbonyl group, a butenylcarbonyl group, a pentenylcarbonyl group, a hexenylcarbonyl group, a heptenylcarbonyl group, an octenylcarbonyl group, an isopropenylcarbonyl group, a 2-methyl-1-propenylcarbonyl group, a 2-methyl-2-butenylcarbonyl group and the like.

As used herein, a "lower alkynylcarbonyl group" refers to a group in which a hydrogen atom of a formyl group has been substituted with a lower alkynyl group. Specific examples include an ethynylcarbonyl group, a propynylcarbonyl group, a butynylcarbonyl group, a pentynylcarbonyl group, a hexynylcarbonyl group, a heptynylcarbonyl group, an octynylcarbonyl group, an isobutynylcarbonyl group, an isopentynylcarbonyl group and the like.

A "lower cycloalkylcarbonyl group" refers to a group in which a hydrogen atom of a formyl group has been substituted with a lower cycloalkyl group. Specific examples include a cyclopropylcarbonyl group, a cyclobutylcarbonyl group, a cyclopentylcarbonyl group, a cyclohexylcarbonyl group, a cycloheptylcarbonyl group, or a cyclooctylcarbonyl group.

As used herein, an "arylcarbonyl group" refers to a group in which a hydrogen atom of a formyl group has been substituted with an aryl group. Specific examples include a phenylcarbony group, a naphtylcarbony group, an anthrylcarbony group, a phenanthrylcarbony group and the like.

As used herein, a "heterocyclic carbonyl group" refers to a group in which a hydrogen atom of a formyl group has been substituted with a heterocyclic group.

As used herein, a "lower alkoxycarbonyl group" refers to a group in which a hydrogen atom of a formyl group has been substituted with a lower alkoxy group. Specific examples include a methoxycarbonyl group, an ethoxycarbonyl group, an n-propoxycarbonyl group, an n-butoxycarbonyl group, an n-pentoxycarbonyl group, an n-hexyloxycarbonyl group, an n-heptyloxycarbonyl group, an n-octyloxycarbonyl group, an isopropoxycarbonyl group, an isobutoxycarbonyl group, a sec-butoxycarbonyl group, a tert-butoxycarbonyl group, an isopentoxycarbonyl group and the like.

As used herein, a "lower alkenyloxycarbonyl group" refers to a group in which a hydrogen atom of a formyl group has been substituted with a lower alkenyloxy group. Specific examples include a vinyloxycarbonyl group, a propenyloxycarbonyl group, a butenyloxycarbonyl group, a pentenyloxycarbonyl group, a hexenyloxycarbonyl group, heptenyloxycarbonyl group, an octenyloxycarbonyl group, an isopropenyloxycarbonyl group, a 2-methyl-1-propenyloxycarbonyl group, a 2-methyl-2-butenyloxycarbonyl group and the like.

As used herein, a "lower alkynyloxycarbonyl group" refers to a group in which a hydrogen atom of a formyl group has been substituted with a lower alkynyloxy group. Specific examples include an ethynyloxycarbonyl group, a propynyloxycarbonyl group, a butynyloxycarbonyl group, a pentynyloxycarbonyl group, a hexynyloxycarbonyl group, a heptynyloxycarbonyl group, an octynyloxycarbonyl group, an isobutynyloxycarbonyl group, an isopentynyloxycarbonyl group and the like.

As used herein, a "lower cycloalkyloxycarbonyl group" refers to a group in which a hydrogen atom of a formyl group has been substituted with a lower cycloalkyloxy group. Specific examples include a cyclopropyloxycarbonyl group, a cyclobutyloxycarbonyl group, a cyclopentyloxycarbonyl group, a cyclohexyloxycarbonyl group, a cycloheptyloxycarbonyl group, a cyclooctyloxycarbonyl group and the like.

As used herein, an "aryloxycarbonyl group" refers to a group in which a hydrogen atom of a formyl group has been substituted with an aryloxy group. Specific examples include a phenoxycarbonyl group, a naphthoxycarbonyl group, an anthryloxycarbonyl group, a phenanthryloxycarbonyl group and the like.

A "heterocyclic oxycarbonyl group" refers to a group in which a hydrogen atom of a formyl group has been substituted with a heterocyclic oxy group.

As used herein, a "lower alkylaminocarbonyl group" refers to a group in which a hydrogen atom of a formyl group has been substituted with a lower alkylamino group. Specific examples include a methylaminocarbonyl group, an ethylaminocarbonyl group, a propylaminocarbonyl group, a dimetylaminocarbonyl group, a diethylaminocarbonyl group, an ethylmethylaminocarbonyl group and the like.

As used herein, a "lower alkenylaminocarbonyl group" refers to a group in which a hydrogen atom of a formyl group has been substituted with a lower alkenylamino group. Specific examples include a vinylaminocarbonyl group, a propenylaminocarbonyl group, a butenylaminocarbonyl group, a pentenylaminocarbonyl group, a hexenylaminocarbonyl group, heptenylaminocarbonyl group, an octenylaminocarbonyl group, an isopropenylaminocarbonyl group, a 2-methyl-1-propenylaminocarbonyl group, a 2-methyl-2-butenylaminocarbonyl group, a divinylaminocarbonyl group, a methyl(vinyl)aminocarbonyl group and the like.

As used herein, a "lower alkynylaminocarbonyl group" refers to a group in which a hydrogen atom of a formyl group has been substituted with a lower alkynylamino group. Specific examples include an ethynylaminocarbonyl group, a propynylaminocarbonyl group, a butynylaminocarbonyl group, a pentynylaminocarbonyl group, a hexynylaminocarbonyl group, a heptynylaminocarbonyl group, an octynylaminocarbonyl group, an isobutynylaminocarbonyl group, an isopentynylaminocarbonyl group, a diethylaminocarbonyl group, an ethynyl(methyl)aminocarbonyl group, an ethynyl(vinyl)aminocarbonyl group and the like.

As used herein, a "lower cycloalkylaminocarbonyl group" refers to a group in which a hydrogen atom of a formyl group has been substituted with a lower cycloalkylamino group. Specific examples include a cyclopropylaminocarbonyl group, a cyclobutylaminocarbonyl group, a cyclopentylaminocarbonyl group, a cyclohexylaminocarbonyl group, a cycloheptylaminocarbonyl group, a cyclooctylaminocarbonyl group, a dicyclohexylaminocarbonyl group, a cyclohexyl(methyl)aminocarbonyl group, a cyclohexyl(vinyl)aminocarbonyl group, a cyclohexyl(ethynyl)aminocarbonyl group and the like.

As used herein, an "arylaminocarbonyl group" refers to a group in which a hydrogen atom of a formyl group has been substituted with an arylamino group. Specific examples include a phenylaminocarbonyl group, a naphtylaminocarbonyl group, an anthrylaminocarbonyl group, a phenanthrylaminocarbonyl group, a diphenylaminocarbonyl group, a methylphenylaminocarbonyl group, an ethylphenylaminocarbonyl group, a phenyl(vinyl)aminocarbonyl group, an ethynyl(phenyl)aminocarbonyl group, a cyclohexyl(phenyl)aminocarbonyl group and the like.

As used herein, a "heterocyclic aminocarbonyl group" refers to a group in which a hydrogen atom of a formyl group has been substituted with a heterocyclic amino group.

As used herein, a "lower alkylsulfinyl group" refers to a group in which a hydroxy of a sulfinic acid group has been substituted with a lower alkyl group. Specific examples include a methylsulfinyl group, an ethylsulfinyl group, an n-propylsulfinyl group, an n-butylsulfinyl group, an n-pentylsulfinyl group, an n-hexylsulfinyl group, an n-heptylsulfinyl group, an n-octylsulfinyl group, an isopropylsulfinyl group, an isobutylsulfinyl group, a sec-butylsulfinyl group, a tert-butylsulfinyl group, an isopentylsulfinyl group and the like.

As used herein, an "arylsulfinyl group" refers to a group in which a hydroxy of a sulfinic acid group has been substituted with an aryl group. Specific examples include a phenylsulfinyl group, a naphtylsulfinyl group, an anthrylsulfinyl group, a phenanthrylsulfinyl group and the like.

A "lower alkylsulfonyl group" refers to a group in which a hydroxy of a sulfonic acid group has been substituted with a lower alkyl group. Specific examples include a methylsulfonyl group, an ethylsulfonyl group, an n-propylsulfonyl group, an n-butylsulfonyl group, an n-pentylsulfonyl group, an n-hexylsulfonyl group, an n-heptylsulfonyl group, an n-octylsulfonyl group, an isopropylsulfonyl group, an isobutylsulfonyl group, a sec-butylsulfonyl group, a tert-butylsulfonyl group, an isopentylsulfonyl group and the like.

As used herein, a "lower alkenylsulfonyl group" refers to a group in which a hydroxy of a sulfonic acid group has been substituted with a lower alkenyl group. Specific examples include a vinylsulfonyl group, a propenylsulfonyl group, a butenylsulfonyl group, a pentenylsulfonyl group, a hexenylsulfonyl group, a heptenylsulfonyl group, an octenylsulfonyl group, an isopropenylsulfonyl group, a 2-methyl-1-propenylsulfonyl group, a 2-methyl-2-butenylsulfonyl group and the like.

As used herein, a "lower alkynylsulfonyl group" refers to a group in which a hydroxy of a sulfonic acid group has been substituted with a lower alkynyl group. Specific examples include an ethynylsulfonyl group, a propynylsulfonyl group, a butynylsulfonyl group, a pentynylsulfonyl group, a hexynylsulfonyl group, a heptynylsulfonyl group, an octynylsulfonyl group, an isobutynylsulfonyl group, an isopentynylsulfonyl group and the like.

A "lower cycloalkylsulfonyl group" refers to a group in which a hydroxy of a sulfonic acid group has been substituted with a lower cycloalkyl group. Specific examples include a cyclopropylsulfonyl group, a cyclobutylsulfonyl group, a cyclopentylsulfonyl group, a cyclohexylsulfonyl group, a cycloheptylsulfonyl group, a cyclooctylsulfonyl group and the like.

As used herein, a "heterocyclic sulfonyl group" refers to a group in which a hydroxy of a sulfonic acid group has been substituted with a heterocyclic group.

As used herein, an "arylsulfonyl group" refers to a group in which a hydroxy of a sulfonic acid group has been substituted with an aryl group. Specific examples include a phenylsulfonyl group, a naphtylsulfonyl group, an anthrylsulfonyl group, a phenanthrylsulfonyl group and the like.

As used herein, a "lower alkylaminocarbonyloxy group" refers to a group in which a hydrogen atom of a formyloxy group has been substituted with a lower alkylamino group. Specific examples include a methylaminocarbonyloxy group, an ethylaminocarbonyloxy group, a propylaminocarbonyloxy group, a dimetylaminocarbonyloxy group, a diethylaminocarbonyloxy group, an ethyl(methyl)aminocarbonyloxy group and the like.

As used herein, an "arylaminocarbonyloxy group" refers to a group in which a hydrogen atom of a formyloxy group has been substituted with an arylamino group. Specific examples include a phenylaminocarbonyloxy group, a naphtylaminocarbonyloxy group, an anthrylaminocarbonyloxy group, a phenanthrylaminocarbonyloxy group, a diphenylaminocarbonyloxy group, a methyl(phenyl)aminocarbonyloxy group, an ethyl(phenyl)aminocarbonyloxy group, a phenyl(vinyl)aminocarbonyloxy group, an ethynyl(phenyl)aminocarbonyloxy group, a cyclohexyl(phenyl)aminocarbonyloxy group and the like.

As used herein, a "3- to 8-membered nitrogen-containing heterocycle" refers to a saturated monocyclic heterocycle comprising 1 or 2 nitrogen atoms in the ring. Specific examples include an aziridine ring, an azetidine ring, a pyrrolidine ring, a piperidine ring, an imidazolidine ring, a pyrazolidine ring, a piperazine ring, a morpholine ring and the like.

As used herein, an "alkylene group" refers to a straight chain or a branched alkylene group having 1 to 8 carbon atoms. Specific examples include a methylene group, an ethylene group, a trimethylene group, a tetramethylene group, a pentamethylene group, a hexamethylene group, a heptamethylene group, an octamethylene group, a methylmethylene group, an ethylmethylene group and the like.

As used herein, an "ester of a hydroxy group" refers to an ester formed from a hydroxy group and carboxylic acids.

As used herein, an "ester of a mercapto group" refers to a thioester formed from a mercapto group and carboxylic acids.

As used herein, an "amide of an amino group" refers to an amide formed from an amino group and carboxylic acids.

As used herein, an "amide of a lower alkylamino group" refers to an amide formed from a lower alkylamino group and carboxylic acids.

An "amide of an arylamino group" refers to an amide formed from an arylamino group and carboxylic acids.

As used herein, an "amide of a heterocyclic amino group" refers to an amide formed from a heterocyclic amino group and carboxylic acids.

As used herein, "carboxylic acids" refer to a saturated aliphatic monocarboxylic acid, a saturated aliphatic dicarboxylic acid, an unsaturated aliphatic carboxylic acid, a carbocyclic ring system carboxylic acid, a heterocyclic ring system carboxylic acid or the like represented by R^{a}COOH (R^{a} refers to a hydrogen atom, a lower alkyl group which may have a substituent, a lower alkenyl group which may have a substituent, an aryl group which may have a substituent, a heterocyclic group which may have a substituent, a lower alkoxy group which may have a substituent or the like). Specific examples include a saturated aliphatic monocarboxylic acid such as a formic acid, an acetic acid, a propionic acid, a butyric acid, an isobutyric acid, a valeric acid, an isovaleric acid, or a pivalic acid; a saturated aliphatic dicarboxylic acid such as an oxalic acid, a malonic acid, a succinic acid, a glutaric acid, or an adipic acid; an unsaturated aliphatic carboxylic acid such as an acrylic acid, a propiolic acid, a crotonic acid, or a cinnamic acid; a carbocyclic ring system carboxylic acid such as a benzoic acid, a phthalic acid, an isophtalic acid, a terephtalic acid, a naphthoic acid, a toluic acid, a cyclohexanecarboxylic acid, or a cyclohexanedicarboxylic acid; a heterocyclic ring system carboxylic acid such as a furancarboxylic acid, a thiophencarboxylic acid, a nicotinic acid, or an isonicotinic acid, and the like. Further, acid anhydride [(R^{a}CO)₂O] and acid halide (R^{a}COX, X refers to a halogen atom) of these carboxylic acids are also included in "carboxylic acids".

As used herein, an "ester of a carboxy group" refers to an ester formed from a carboxy group and alcohols or phenols.

As used herein, an "ester of a sulfinic acid group" refers to an ester formed from a sulfinic acid group and alcohols or phenols.

As used herein, an "ester of a sulfonic acid group" refers to an ester formed from a sulfonic acid group and alcohols or phenols.

As used herein, "alcohols" refer to a saturated aliphatic system hydroxy compound, an unsaturated aliphatic system hydroxy compound or the like represented by R^{b}OH (R^{b} refers to a lower alkyl group which may have a substituent, an alkenyl group which may have a substituent or the like). As specific examples, alcohols refer to a saturated aliphatic system hydroxy compound such as methanol, ethanol, propanol, butanol, or isopropanol; an unsaturated aliphatic system hydroxy compound such as vinyl alcohol; a saturated aliphatic system hydroxy compound substitued with an aryl group such as benzyl alcohol or phenethyl alcohol, or the like.

As used herein, "phenols" refer to a carbocyclic ring system hydroxy compound or the like represented by R^{c}OH (R^{c} refers to an aryl group which may have a substituent or the like). Specific examples include phenol, naphthol, anthrol, phenanthrol and the like.

As used herein, an "amide of a carboxy group" refers to an acid amide formed from a carboxy group and amines.

As used herein, an "amide of a sulfinic acid group" refers to an acid amide formed from a sulfinic acid group and amines.

As used herein, an "amide of a sulfonic acid group" refers to an acid amide formed from a sulfonic acid group and amines.

As used herein, "amines" refer to ammonium, a saturated aliphatic system amine compound, a carbocyclic ring system amine compound, a heterocyclic ring system amine compound, a saturated cyclic amine compound or the like represented by HNR^{d}R^{e} [R^{d} and R^{e} are the same or different and refer to a hydrogen atom, a lower alkyl group which may have a substituent, an aryl group which may have a substituent, a heterocyclic group or the like. Further, R^{c} and R^{d} may together form a saturated cyclic amine]. As specific examples, amines refer to ammonium; a saturated aliphatic system amine compound such as methylamine, ethylamine, propylamine, pentylamine, dimethylamine, diethylamine, or ethylamine; a saturated aliphatic system amine compound having a substituent such as benzylamine; a carbocyclic ring system amine compound such as phenylamine, naphthylamine, anthrylamine, phenanthrylamine, diphenylamine, methylphenylamine, ethylphenylamine, or cyclohexylamine; a heterocyclic ring system amine compound such as furylamine, thienylamine, pyrrolidylamine, pyridylamine, quinolylamine, or methylpyridylamine; a saturated cyclic amine compound such as aziridine, azetidine, pyrrolidine, piperidine, or 4-methylpiperidine, or the like.

As used herein, a "lower alkyl group which may have a substituent", a "lower alkenyl group which may have a substituent group", a "lower alkynyl group which may have a substituent", a "lower alkoxy group which may have a substituent", a "lower alkylcarbonyl group which may have a substituent", a "lower alkenylcarbonyl group which may have a substituent", a "lower alkynylcarbonyl group which may have a substituent", a "lower alkoxycarbonyl group which may have a substituent", a "lower alkenyloxycarbonyl group which may have a substituent", a "lower alkynyloxycarbonyl group which may have a substituent", a "lower alkylaminocarbonyl group which may have a substituent", and/or a "lower alkylsulfonyloxy group" refer to a "lower alkyl group", a "lower alkenyl group", a "lower alkynyl group", a "lower alkoxy group", a "lower alkylcarbonyl group", a "lower alkenylcarbonyl group", a "lower alkynylcarbonyl group", a "lower alkoxycarbonyl group", a "lower alkenyloxycarbonyl group", a "lower alkynyloxycarbonyl group", a "lower alkylaminocarbonyl group", or a "lower alkylsulfonyloxy group" which may have one or more substituents selected from the α¹ group below.

### [α¹ group]

A halogen atom, a lower cycloalkyl group, an aryl group, an aryl group substituted with a halogen atom, an aryl group substituted with a lower alkyl group, an aryl group susbtituted with a hydroxy group, an aryl group substituted with a lower alkoxy group, a heterocyclic group, a hydroxy group, an ester of a hydroxy group, a lower alkoxy group, a lower alkoxy group substituted with a halogen atom, a lower alkenyloxy group, a lower alkynyloxy group, a lower cycloalkyloxy group, an aryloxy group, a heterocyclic oxy group, a mercapto group, an ester of a mercapto group, a lower alkylthio group, a lower alkenylthio group, a lower alkynylthio group, a lower cycloalkylthio group, an arylthio group, a heterocyclic thio group, an amino group, an amide of an amino group, a lower alkylamino group, an amide of a lower alkylamino group, an arylamino group, an amide of an arylamino group, a heterocyclic amino group, an amide of a heterocyclic amino group, a formyl group, a lower alkylcarbonyl group, a lower alkenylcarbonyl group, a lower alkynylcarbonyl group, a lower cycloalkylcarbonyl group, an arylcarbonyl group, a heterocyclic carbonyl group, a carboxy group, an ester of a carboxy group, an amide of a carboxy group, a lower alkoxycarbonyl group, a lower alkenyloxycarbonyl group, a lower alkynyloxycarbonyl group, a lower cycloalkyloxycarbonyl group, an aryloxycarbonyl group, a heterocycic oxycarbonyl group, a lower alkylsulfinyl group, an arylsulfinyl group, a lower alkylsulfonyl group, an arylsulfonyl group, a sulfinic acid group, an ester of a sulfinic acid group, an amide of a sulfinic acid group, a sulfonic acid group, an ester of a sulfonic acid group, an amide of a sulfonic acid group, a nitro group, and a cyano group.

As used herein, a "lower cycloalkyl group which may have a substituent", an "aryl group which may have a substituent", a "heterocyclic group which may have a substituent", a "lower cycloalkylcarbonyl group which may have a substituent", an "arylcarbonyl group which may have a substituent", a "heterocyclic carbonyl group which may have a substituent", a "lower cycloalkyloxycarbonyl group which may have a substituent", an "aryloxycarbonyl group which may have a substituent", a "heterocyclic oxycarbonyl group which may have a substituent", an "arylaminocarbonyl group which may have a substituent", and/or a "heterocyclic aminocarbonyl group which may have a substituent" refer to a "lower cycloalkyl group", an "aryl group", a "heterocyclic group", a "lower cycloalkylcarbonyl group", an "arylcarbonyl group", a "heterocyclic carbonyl group", a "lower cycloalkyloxycarbonyl group", an "aryloxycarbonyl group", a "heterocyclic oxycarbonyl group", an "arylaminocarbonyl group", or a "heterocyclic aminocarbonyl group" which may have one or more substituents selected from the β¹ group below.

### [β¹ group]

A halogen atom, a lower alkyl group, a lower alkyl group substituted with a halogen atom, a lower alkyl group substituted with a hydroxy group, a lower alkyl group substituted with a lower alkoxy group, a lower alkyl group substituted with an amino group, a lower alkyl group substituted with a lower alkylamino group, a lower alkyl group substituted with a carboxy group, a lower alkyl group substituted with a lower alkoxycarbonyl group, a lower alkenyl group, a lower alkynyl group, a lower cycloalkyl group, an aryl group, a heterocyclic group, a hydroxy group, an ester of a hydroxy group, a lower alkoxy group, a lower alkoxy group substituted with a halogen atom, a lower alkenyloxy group, a lower alkynyloxy group, a lower cycloalkyloxy group, an aryloxy group, a heterocyclic oxy group, a mercapto group, an ester of a mercapto group, a lower alkylthio group, a lower alkenylthio group, a lower alkynylthio group, a lower cycloalkylthio group, an arylthio group, a heterocyclic thio group, an amino group, an amide of an amino group, a lower alkylamino group, an amide of a lower alkylamino group, an arylamino group, an amide of an arylamino group, a heterocyclic amino group, an amide of a heterocyclic amino group, a formyl group, a lower alkylcarbonyl group, a lower alkenylcarbonyl group, a lower alkynylcarbonyl group, a lower cycloalkylcarbonyl group, an arylcarbonyl group, a heterocyclic carbonyl group, a carboxy group, an amide of a carboxy group, a lower alkoxycarbonyl group, a lower alkenyloxycarbonyl group, a lower alkynyloxycarbonyl group, a lower cycloalkyloxycarbonyl group, an aryloxycarbonyl group, a heterocyclic oxycarbonyl group, a lower alkylsulfinyl group, an arylsulfinyl group, a lower alkylsulfonyl group, an arylsulfonyl group, a sulfinic acid group, an ester of a sulfinic acid group, an amide of a sulfinic acid group, a sulfonic acid group, an ester of a sulfonic acid group, an amide of a sulfonic acid group, a nitro group, a cyano group, an aminocarbonyloxy group, a lower alkylaminocarbonyloxy group, and an arylaminocarbonyloxy group.

In a preferred embodiment, the conditions, disorders, or diseases targeted by the present invention are disorders related to Fuchs' endothelial corneal dystrophy. It is demonstrated that TGF-β induction in corneal endothelial cells is involved in Fuchs' endothelial corneal dystrophy. It is also demonstrated that this may be involved in cell lost in FECD.

Since the medicament of the present invention can treat cell damage or the like that is induced by TGF-β2, which can be one of the important causes of abnormalities or disorders in Fuchs' endothelial corneal dystrophy, the medicament is understood to be useful in treating or preventing Fuchs' endothelial corneal dystrophy. In particular, the present invention was able to suppress cell damage or programmed cell death induced by TGF-β2 in a Fuchs' endothelial corneal dystrophy model in the Examples, so that the present invention can be considered usable in therapy for patients with severe TGF-β2 associated disease in a Fuchs' endothelial corneal dystrophy model. The present invention can treat or prevent damage to corneal endothelial cells in Fuchs' endothelial corneal dystrophy, decreased corneal endothelial density, guttae formation, hypertrophy of the Descemet's membrane, hypertrophy of a cornea, corneal epithelial disorder, turbidity in corneal stroma, photophobia, blurred vision, visual impairment, ophthalmalgia, epiphora, hyperemia, pain, bullous keratopathy, eye discomfort, diminished contrast, glare, edema of the corneal stroma, and the like.

### (General techniques)

Molecular biological methodology, biochemical methodology, microbiological methodology used herein are well known and conventionally used in the art, which are described for example in Sambrook J. et al. (1989). Molecular Cloning: A Laboratory Manual, Cold Spring Harbor and 3rd Ed. thereof (2001); Ausubel, F.M. (1987). Current Protocols in Molecular Biology, Greene Pub. Associates and Wiley-Interscience; Ausubel, F.M. (1989). Short Protocols in Molecular Biology: A Compendium of Methods from Current Protocols in Molecular Biology, Greene Pub. Associates and Wiley-Interscience; Innis, M.A. (1990). PCR Protocols: A Guide to Methods and Applications, Academic Press; Ausubel, F.M. (1992). Short Protocols in Molecular Biology: A Compendium of Methods from Current Protocols in Molecular Biology, Greene Pub. Associates; Ausubel, F.M. (1995). Short Protocols in Molecular Biology: A Compendium of Methods from Current Protocols in Molecular Biology, Greene Pub. Associates; Innis, M.A. et al. (1995). PCR Strategies, Academic Press; Ausubel, F.M. (1999). Short Protocols in Molecular Biology: A Compendium of Methods from Current Protocols in Molecular Biology, Wiley, and annual updates; Sninsky, J.J. et al. (1999). PCR Applications: Protocols for Functional Genomics, Academic Press, Gait, M.J. (1985). Oligonucleotide Synthesis: A Practical Approach, IRL Press; Gait, M.J. (1990). Oligonucleotide Synthesis: A Practical Approach, IRL Press; Eckstein, F. (1991). Oligonucleotides and Analogues: A Practical Approach, IRL Press; Adams, R.L. et al. (1992). The Biochemistry of the Nucleic Acids, Chapman & Hall; Shabarova, Z. et al. (1994). Advanced Organic Chemistry of Nucleic Acids, Weinheim; Blackburn, G.M. et al. (1996). Nucleic Acids in Chemistry and Biology, Oxford University Press; Hermanson, G.T. (1996). Bioconjugate Techniques, Academic Press, Bessatsu Jikken Igaku [Experimental Medicine, Supplemental Volume], Idenshi Donyu Oyobi Hatsugen Kaiseki Jikken Ho [Experimental Methods for Transgenesis & Expression Analysis], Yodosha, 1997, or the like. The reports by Nancy Joyce et al {Joyce, 2004 #161} and {Joyce, 2003 #7} are well known for corneal endothelial cells. However, as discussed above, long-term culture or subculture results in fibroblast-like transformation, and research for an effective culturing method are currently ongoing. Relevant portions (which may be all) thereof are incorporated herein by reference.

### (Disclosure of preferred embodiments)

The preferred embodiments of the present invention are described hereinafter. It is understood that the embodiments are exemplification of the present invention, so that the scope of the present invention is not limited to such preferred embodiments. It should be understood that those skilled in the art can refer to the following preferred embodiments to readily make modifications within the scope of the present invention. These embodiments of the present invention can be appropriately combined with any embodiment by those skilled in the art.

### <Medicament>

In one aspect, the present invention discovered that a compound which was not previously known to have inhibiting activity of caspase activity in corneal endothelial cells has caspase activity, and provides a medicament for use in treating or preventing a corneal endothelial condition, disorder, or disease, comprising the compound. Specifically, the present invention provides a composition for treating or preventing a corneal endothelial condition, disorder, or disease, comprising a compound which, when contacted with immortalized Fuchs' endothelial corneal dystrophy cells, exhibits: (i) cell viability (%) of the cells of about 90% or more (that is, less toxic or non-toxic) after being cultured in Dulbecco's Modified Eagle Medium (DMEM) + 2% fetal bovine serum (FBS) + 1% penicillin/streptomycin (P/S) for 24 to 28 hours; and (ii) ratio of caspase activity (preferably, caspase 3/7 activity) (%) in the presence of TGF-β with respect to the cell viability (%) of less than 1.0, 0.95 or less, 0.9 or less, 0.85 or less, or 0.8 or less (caspase inhibiting activity) after being cultured in Dulbecco's Modified Eagle Medium (DMEM) + 2% fetal bovine serum (FBS) + 1% penicillin/streptomycin (P/S) for 24 to 28 hours (e.g., 24 hours or 28 hours). Thus, the medicament of the present invention can treat or prevent a corneal endothelial condition, disorder, or disease due to transforming growth factor-β (TGF-β). The culturing time is preferably 24 hours.

For example, the compound that can be used in the medicament of the present invention includes, but is not limited to, the compounds listed in Table 1. These compounds are a group of compounds that were not previously known to have caspase inhibiting activity and were not previously known to have caspase inhibiting activity particularly under the condition of a corneal endothelium. Thus, it was not known that these compounds can treat or prevent a corneal endothelial condition, disorder, or disease due to TGF-β. Therefore, these compounds are surprising and were unexpected. Advantageous compounds having relatively strong caspase activity are listed in Table 2.

The compounds show in Table 1 and Table 2 below are exemplary compounds which, when contacted with immortalized Fuchs' endothelial corneal dystrophy cells, exhibit: (i) cell viability (%) of the cells of about 90% or more; and (ii) ratio of caspase 3/7 activity (%) in the presence of TGF-β with respect to the cell viability (%) of less than 1.0 (Table 1) or 0.8 or less (Table 2) after being cultured in Dulbecco's Modified Eagle Medium (DMEM) + 2% fetal bovine serum (FBS) + 1% penicillin/streptomycin (P/S) for 24 to 28 hours (e.g., 24 hours or 28 hours). The culturing time is preferably 24 hours.

**[Table 1-1]**

| |
|---|
| Amlexanox |
| Leflunomide |
| Olsalazine•Na |
| Hydroxocobalamin•HCl |

**[Table 1-2]**

| |
|---|
| Everolimus |
| Mometasone Furoate |
| Febuxostat |
| Sirolimus (Rapamycin) |
| Orphenadrine Citrate |
| Clobetasol Propionate |
| Loteprednol Etabonate |
| Difluprednate |
| Dexamethasone |
| Dobutamine•HCl |
| Amcinonide |
| Flurandrenolide |
| Fluorouracil (5-Fluorouracil) |
| Prednisolone |
| Fluocinolone Acetonide |
| Desonide |
| Triamcinolone Acetonide |
| Ketoconazole |
| Flurbiprofen |
| Budesonide |
| Fludrocortisone Acetate |
| Fluocinonide |
| Methylprednisolone |
| Betamethasone |
| Desoximetasone |
| Toremifene Base |
| Terazosin•HCl |
| Halcinonide |
| Bromocriptine Mesylate |
| Fluorometholone |
| Oxaliplatin |
| Calcipotriene |
| Beclomethasone Dipropionate |
| Reserpine |
| Phenoxybenzamine•HCl |

**[Table 1-3]**

| |
|---|
| Droperidol |
| Promethazine•HCl |
| Esmolol |
| Dutasteride |
| Dihydroergotamine Mesylate |
| Imipramine•HCl |
| Betaxolole•HCl |
| Trimethobenzamide•HCl |
| Nitazoxanide |
| Pimecrolimus |
| Deferasirox |
| Mycophenolate Mofetil |
| Carvedilol |
| Atomoxetine•HCl |
| Colistimethate•Na |
| Fulvestrant |
| Terbinafine•HCl |
| Tolterodine Tartrate |
| Hydrocortisone Acetate |
| Fenoldopam Mesylate |
| Calcitriol |
| Dicyclomine•HCl |
| Paliperidone |
| Diclofenac•Na Salt |
| Salmeterol |
| Desogestrel |
| Procainamide•HCl |
| Butoconazole Nitrate |
| Guanidine•HCl |
| Rimantadine•HCl |
| Megestrol Acetate |
| Mycophenolic Acid |
| Propofol |
| Fluoxetine•HCl |
| Meclizine Dihydrochloride |

**[Table 1-4]**

| |
|---|
| Trimipramine Maleate |
| Nefazodone•HCl |
| Buspirone•HCl |
| Miconazole |
| Asenapine Maleate |
| Acebutolol•HCl |
| Metoprolol Tartrate |
| Methyldopa Sesquihydrate (L-A-Methyl-Dopa Sesquihydrate) |
| Amoxapine |
| Quinidine•HCl•H2O |
| Tolcapone |
| Thioridazine•HCl |
| Oxybutynin Chloride |
| Nisoldipine |
| Tetracycline |
| Ropinirole•HCl |
| Milrinone |
| Diphenhydramine•HCl |
| Primaquine Phosphate |

**[Table 2-1]**

| |
|---|
| Amlexanox |
| Leflunomide |
| Olsalazine·Na |
| Hydroxocobalamin•HCl |
| Everolimus |
| Mometasone Furoate |
| Febuxostat |
| Sirolimus (Rapamycin) |
| Orphenadrine Citrate |
| Clobetasol Propionate |
| Loteprednol Etabonate |
| Difluprednate |

**[Table 2-2]**

| |
|---|
| Dexamethasone |
| Dobutamine•HCl |
| Amcinonide |
| Flurandrenolide |
| Fluorouracil (5-Fluorouracil) |
| Prednisolone |
| Flucinolone Acetonide |
| Desonide |
| Triamcinolone Acetonide |
| Ketoconazole |
| Flurbiprofen |
| Budesonide |
| Fludrocortisone Acetate |
| Fluocinonide |
| Methylprednisolone |
| Betamethasone |
| Desoximetasone |
| Toremifene Base |
| Terazosin•HCl |
| Halcinonide |
| Bromocriptine Mesylate |
| Fluorometholone |
| Oxaliplatin |
| Calcipotriene |
| Beclomethasone Dipropionate |
| Reserpine |
| Phenoxybenzamine•HCl |
| Droperidol |
| Promethazine•HCl |
| Esmolol |
| Dutasteride |
| Dihydroergotamine Mesylate |
| Imipramine•HCl |
| Betaxolol•HCl |
| Trimethobenzamide•HCl |

In particular, Table 2 contains, for example, many agents that are known as an anti-inflammatory drug (such as a steroidal anti-inflammatory drug or a nonsteroidal anti-inflammatory drug (NSAID)). It was not known that an anti-inflammatory drug can treat or prevent a corneal endothelial condition, disorder, or disease due to TGF-β. Further, Table 2 contains vitamins (such as vitamin B12 group or vitamin D group). It was not known that vitamins (such as vitamin B12 group or vitamin D group) also can treat or prevent a corneal endothelial condition, disorder, or disease due to TGF-β. Further, a selective glucocorticoid receptor agonist (SEGRA) and a selective glucocorticoid receptor modulator (SEGRM) were also discovered to be able to treat or prevent a corneal endothelial condition, disorder, or disease due to TGF-β. However, it was not known that these compounds can treat or prevent a corneal endothelial condition, disorder, or disease due to TGF-β.

In some embodiments, a compound used for the medicament of the present invention is a anti-inflammatory drug. The anti-inflammatory drug may be a steroidal anti-inflammatory drug, or may be a nonsteroidal anti-inflammatory drug (NSAID). The steroidal anti-inflammatory drug includes, but is not limited to, Mometasone Furoate, Clobetasol Propionate, Loteprednol Etabonate, Difluprednate, Dexamethasone, Amcinonide, Flurandrenolide, Prednisolone, Fluocinolone Acetonide, Desonide, Triamcinolone Acetonide, Budesonide, Fludrocortisone Acetate, Fluocinonide, Methylprednisolone, Betamethasone, Desoximetasone, Halcinonide, Fluorometholone, Beclomethasone Dipropionate, and Dutasteride. The nonsteroidal anti-inflammatory drug (NSAID) includes, but is not limited to, Amlexanox, Leflunomide, Olsalazine•Na, Orphenadrine Citrate, Flurbiprofen, and Phenoxybenzamine•HCl.

In some embodiment, a compound used for the medicament of the present invention is a vitamin such as vitamin B16 and vitamin D. Vitamin B16 and vitamin D may be a derivative or an analogue thereof. For example, vitamin B16 includes, but is not limited to, hydroxocobalamin, cyanocobalamin, mecobalamin and deoxyadenosylcobalamin. In a preferred embodiment, vitamin B16 can be Hydroxocobalamin•HCl. Vitamin D includes vitamin D and a derivative or an analogue having activity similar to that of vitamin D. A derivative or an analogue of vitamin D is described in detail in Japanese National Phase PCT Laid-Open Publication No. 2013-518812, Japanese National Phase PCT Laid-Open Publication No. 2013-515018 and the like that are incorporated by reference herein. In a preferred embodiment, vitamin D can be Calcipotriene.

In one embodiment, the medicament of the present invention can treat or prevent a corneal endothelial condition, disorder, or disease due to transforming growth factor-β (TGF-β) in corneal endothelial cells. In a specific example, the corneal endothelial condition, disorder, or disease due to TGF-β is selected from the group consisting of Fuchs' endothelial corneal dystrophy, post-corneal transplant disorder, corneal endotheliitis, trauma, post-ophthalmic surgery disorder, post-ophthalmic laser surgery disorder, aging, posterior polymorphous dystrophy (PPD), congenital hereditary endothelial dystrophy (CHED), idiopathic corneal endothelial disorder, and cytomegalovirus corneal endotheliitis. In a preferred embodiment, the corneal endothelial condition, disorder, or disease due to TGF-β is Fuchs' endothelial corneal dystrophy.

In another embodiment, the above compound used in the present invention is a compound which, when further contacted with immortalized human corneal endothelial cells, exhibits (i) cell viability (%) of the cells of about 90% or more after being cultured in Dulbecco's Modified Eagle Medium (DMEM) + 2% fetal bovine serum (FBS) + 1% penicillin/streptomycin (P/S) for 18 hours and (ii) ratio of caspase activity (e.g., caspase 3/7 activity) in the presence of MG-132 with respect to the cell viability (%) of less than 1.0, 0.95 or less, 0.9 or less, 0.85 or less, or 0.8 or less (caspase inhibiting activity) after being cultured in Dulbecco's Modified Eagle Medium (DMEM) + 2% fetal bovine serum (FBS) + 1% penicillin/streptomycin (P/S) for 18 hours. Such a compound further has inhibiting activity against MG-132, and can treat or prevent a corneal endothelial condition, disorder, or disease due to endoplasmic reticulum (ER) associated stress. MG-132 is known as substance that can induce abnormal folding of proteins (accumulation of unfolded proteins), which is one of the causes of endoplasmic reticulum associated stress. In a certain embodiment, the corneal endothelial condition, disorder, or disease due to endoplasmic reticulum (ER) associated stress is selected from conditions, disorders, or diseases associated with endoplasmic reticulum (ER) stress among damage to corneal endothelial cells in Fuchs' endothelial corneal dystrophy, corneal endothelial disorder, decreased corneal endothelial density, guttae formation, hypertrophy of the Descemet's membrane, hypertrophy of a cornea, turbidity, corneal epithelial disorder, turbidity in corneal stroma, photophobia, blurred vision, visual impairment, ophthalmalgia, epiphora, hyperemia, pain, bullous keratopathy, eye discomfort, diminished contrast, glare, edema of the corneal stroma, corneal epithelial erosion, and angiogenesis.

The compounds shown in Table 3 below are exemplary compounds which, when contacted with immortalized human corneal endothelial cells, exhibit (i) cell viability (%) of the cells of about 90% or more after being cultured in Dulbecco's Modified Eagle Medium (DMEM) + 2% fetal bovine serum (FBS) + 1% penicillin/streptomycin (P/S) for 18 hours and (ii) ratio of caspase 3/7 activity in the presence of MG-132 with respect to the cell viability (%) of 0.8 or less after being cultured in Dulbecco's Modified Eagle Medium (DMEM) + 2% fetal bovine serum (FBS) + 1% penicillin/streptomycin (P/S) for 18 hours among the compounds listed in Table 2.

**[Table 3]**

| Name of compound |
|---|
| Amlexanox |
| Olsalazine•Na |
| Hydroxocobalamin•HCl |
| Leflunomide |
| Febuxostat |
| Flurbiprofen |
| Terazosin•HCl |
| Fluorouracil (5-Fluorouracil) |

In a certain embodiment, the medicament of the present invention comprises a compound that can treat or prevent a disease due to both TGF-β and endoplasmic reticulum (ER) associated stress. Such a compound includes, but is not limited to, Amlexanox, Olsalazine•Na, Hydroxocobalamin HCl, Leflunomide, Febuxostat, Flurbiprofen, Terazosin•HCl, and Fluorouracil (5-Fluorouracil). Fuchs' endothelial corneal dystrophy is known to be associated with ER stress (Engler, C. et al. Am J Ophthalmol 149, 194-202 (2010)). For this reason, suppression of ER stress in addition to TGF-β means that therapy and prophylaxis of Fuchs' endothelial corneal dystrophy can be significantly improved, and completely healed in some cases.

In some embodiment, the above compound may be in the form of a derivative or an analogue thereof, or a pharmaceutically acceptable salt thereof, or a solvate thereof. A derivative or an analogue can have activity that is the same as or similar to that a parent compound originally has. Alternatively, a derivative or an analogue can have inhibiting activity against TGF-β and/or inhibiting activity against MG-132 which is the same as or similar to that of a parent compound.

In one embodiment, an endothelial condition, disorder, or disease due to endoplasmic reticulum (ER) associated stress in corneal endothelial cells can be caused by abnormal folding of proteins. In mammalian cells, it is known that proteins, which have aggregated due to being unfolded, misfolding, abnormality in proteolysis, or the like (also referred to as incompletely folded proteins or denatured proteins (unfolded proteins)), are ubiquitinated and accumulate near the centrosome by a dynein motor that moves on microtubules to form an inclusion body called aggresome. Aggresomes are generally formed by heat shock, viral infection, oxidative stress, or the like. Several diseases are known in humans that are associated with inclusion bodies in cells, such as Lewy bodies found in nerve cells in Parkinson's disease, Mallory bodies found in hepatocytes in alcoholic liver diseases, and glass-like bodies found in astrocytes in amyotrophic lateral sclerosis.

The compounds shown in Table E are listed compounds exhibiting caspase activity of less than 80% at any concentration as a result of using the compounds at various concentrations to confirm TGF-β inhibiting activity in Exmple 3. The compounds listed in Table C can be useful as a caspase inhibitor and/or a composition for treating or preventing a corneal endothelial condition, disorder, or disease (e.g., Fuchs' endothelial corneal dystrophy).

**[Table E-1]**

| |
|---|
| Terazosin•HCl |
| Ketoconazole |
| Toremifene Base |
| Nitazoxanide |
| Mometasone Furoate |
| Mycophenolate Mofetil |
| Mycophenolic Acid |
| Deferasirox |
| Carvedilol |
| Fluoxetine•HCl |
| Fulvestrant |
| Methylprednisolone |
| Dexamethasone |
| Betamethasone |
| Difluprednate |
| Prednisolone |
| Fludrocortisone Acetate |
| Flurandrenolide |
| Amcinonide |
| Budesonide |
| Clobetasol Propionate |
| Fluocinolone Acetonide |
| Tolterodine Tartrate |
| Imipramine•HCl |
| Beclomethasone Dipropionate |
| Pyrimethamine |
| Desonide |
| Oxaliplatin |
| Fluocinonide |
| Everolimus |
| Rifapentine |
| Bromocriptine Mesylate |
| Nisoldipine |
| Triamcinolone Acetonide |
| Fluorometholone |
| Desoximetasone |

**[Table E-2]**

| |
|---|
| Oxiconazole Nitrate |
| Halcinonide |
| Pimecrolimus |
| Calcitriol |
| Dihydroergotamine Mesylate |
| Aripiprazole |
| Asenapine Maleate |
| Terbinafine•HCl |

Among the above-listed compounds, Clobetasol Propionate exhibited caspase activity of 80% or less at a concentration of 1 pM, and Fluocinolone Acetonide and Fluocinonide exhibited caspase activity of 80% or less at a concentration of 10 pM and at a concentration of 100 pM, respectively (Figure **4A-1****,** Figure **4A-2** and Figure **4A-3****,** and Figure **4B-1****,** Figure **4B-2** and Figure **4B-3****).** These compounds exhibited caspase activity of 80% or less even at a very low concentration of less than 1 nM. Surprisingly, these compounds are all steroidal anti-inflammatory drugs, which suggests that a steroidal anti-inflammatory drug has strong caspase inhibiting activity against, for example, caspase activation in response to TGF-β stimulation in corneal endothelial cells. Thus, in a preferred embodiment, a compound selected from the group consisting of Clobetasol Propionate, Fluocinolone Acetonide and Fluocinonide, more preferably a compound selected from the group consisting of Clobetasol Propionate and Fluocinolone Acetonide, and still more preferably Clobetasol Propionate are intended for the present invention. However, the present invention is not limited to such compounds.

Difluprednate and Budesonide, which are steroidal anti-inflammatory drugs, and Tolterodine Tartrate, which is an anti-cholin agent, also exhibited caspase activity of 80% or less at a concentration of 1 nM (Figure **4A-1****,** Figure **4A-2** and Figure **4A-3****,** and Figure **4B-1****,** Figure **4B-2** and Figure **4B-3****).** Thus, a compound selected from the group consisting of Clobetasol Propionate, Fluocinolone Acetonide, Fluocinonide, Difluprednate, Budesonide, and Tolterodine Tartrate, which is an anti-cholin agent, can be one preferred embodiment.

In one preferred embodiment, a compound selected from the group consisting of Mometasone Furoate, Mycophenolate Mofetil, Mycophenolic Acid, Fulvestrant, Dexamethasone, Difluprednate, Flurandrenolide, Amcinonide, Budesonide, Clobetasol Propionate, Fluocinolone Acetonide, Tolterodine Tartrate, Fluocinonide, Everolimus, Triamcinolone Acetonide, Desoximetasone, Halcinonide and Pimecrolimus is selected. These compounds exhibited caspase activity of 80% or less at a concentration of 10 nM (Figure **4A-1****,** Figure **4A-2** and Figure **4A-3****,** and Figure **4B-1****,** Figure **4B-2** and Figure **4B-3**).

In one preferred embodiment, a compound selected from the group consisting of Mometasone Furoate, Mycophenolate Mofetil, Mycophenolic Acid, Fulvestrant, Methylprednisolone, Dexamethasone, Betamethasone, Difluprednate, Prednisolone, Fludrocortisone Acetate, Flurandrenolide, Amcinonide, Budesonide, Clobetasol Propionate, Fluocinolone Acetonide, Beclomethasone Dipropionate, Desonide, Fluocinonide, Everolimus, Triamcinolone Acetonide, Fluorometholone, Desoximetasone and Halcinonide is selected. These compounds exhibited caspase activity of 80% or less at a concentration of 100 nM (Figure **4A-1****,** Figure **4A-2** and Figure **4A-3****,** and Figure **4B-1****,** Figure **4B-2** and Figure **4B-3**).

In one preferred embodiment, a compound selected from the group consisting of Mometasone Furoate, Mycophenolate Mofetil, Mycophenolic Acid, Fulvestrant, Methylprednisolone, Dexamethasone, Betamethasone, Difluprednate, Prednisolone, Fludrocortisone Acetate, Flurandrenolide, Amcinonide, Budesonide, Clobetasol Propionate, Fluocinolone Acetonide, Tolterodine Tartrate, Beclomethasone Dipropionate, Desonide, Fluocinonide, Everolimus, Rifapentine, Triamcinolone Acetonide, Fluorometholone, Desoximetasone, Halcinonide and Pimecrolimus is selected. These compounds exhibited caspase activity of 80% or less at a concentration of 1 µM (Figure **4A-1****,** Figure **4A-2** and Figure **4A-3****,** and Figure **4B-1****,** Figure **4B-2** and Figure **4B-3****).**

Most of the compounds listed in the above Table E (compounds other than Rifapentine and Terbinafine•HCl) exhibited caspase activity of 80% or less at 10 µM (Figure **4A-1****,** Figure **4A-2** and Figure **4A-3****,** and Figure **4B-1****,** Figure **4B-2** and Figure **4B-3****).** Thus, in another embodiment, a compound selected from the group consisting of Terazosin•HCl, Ketoconazole, Toremifene Base, Nitazoxanide, Mometasone Furoate, Mycophenolate Mofetil, Mycophenolic Acid, Deferasirox, Carvedilol, Fluoxetine•HCl, Fulvestrant, Methylprednisolone, Dexamethasone, Betamethasone, Difluprednate, Prednisolone, Fludrocortisone Acetate, Flurandrenolide, Amcinonide, Budesonide, Clobetasol Propionate, Fluocinolone Acetonide, Tolterodine Tartrate, Imipramine•HCl, Beclomethasone Dipropionate, Pyrimethamine, Desonide, Oxaliplatin, Fluocinonide, Everolimus, Bromocriptine Mesylate, Nisoldipine, Triamcinolone Acetonide, Fluorometholone, Desoximetasone, Oxiconazole Nitrate, Halcinonide, Pimecrolimus, Calcitriol, Dihydroergotamine Mesylate, Aripiprazole and Asenapine Maleate is selected.

In another embodiment, the present invention provides a composition for treating or preventing a corneal endothelial condition, disorder, or disease, comprising a selective glucocorticoid receptor agonist (SEGRA) or a selective glucocorticoid receptor modulator (SEGRM). SEGRA and SEGRM (collectively referred to as SEGRAM) are typically compounds developed such that they do not have transactivation activity, which is a cause of side-effects, or they partially have transactivation activity even if they have any, and such that they have transrepression activity in order to reduce the side-effects of a steroid. SEGRAM is a group of compounds that only act on a glucocorticoid receptor. However, since it was not known so far that the action on a glucocorticoid receptor affects the TGF-β signaling, it was unexpected that SEGRAM can suppress a disorder in corneal endothelial cells due to a TGF-β signal. Furthermore, it was revealed that the toxicity to cells is low, that is, the cell viability is high in the presence of SEGRAM. Thus, SEGRAM used in the composition of the present invention may have transactivation activity or does not need to have transactivation activity. However, it is preferable that SEGRAM used in the composition of the present invention does not have transactivation activity, or partially has transactivation activity even if it has any.

In one embodiment, a selective glucocorticoid receptor modulator (SEGRM) is a selective glucocorticoid receptor agonist that does not have a basic skeleton of a steroid, which is, for example, a compound represented by the following general formula (1) or general formula (2) 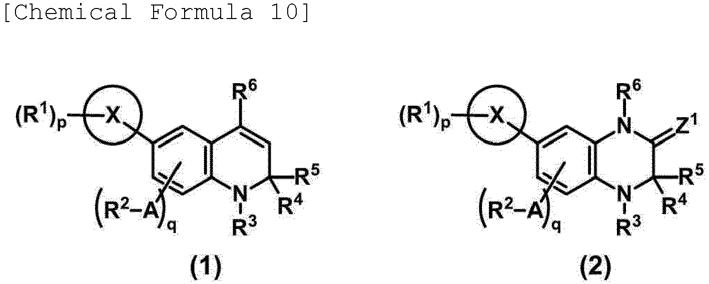 or a salt thereof, wherein:
ring X represents a benzene ring or a pyridine ring;
R¹ represents a halogen atom, a lower alkyl group which may have a substituent, a lower cycloalkyl group which may have a substituent, an aryl group which may have a substituent, an arylalkyloxy group which may have a substituent, an arylalkyloxyalkyloxy group, a heterocyclic group which may have a substituent, a hydroxy group, an ester of a hydroxy group, a lower alkoxy group which may have a substituent, a lower cycloalkyloxy group which may have a substituent, an aryloxy group which may have a substituent, a heterocyclic oxy group which may have a substituent, a mercapto group, an ester of a mercapto group, a lower alkylthio group which may have a substituent, a lower cycloalkylthio group which may have a substituent, an arylthio group which may have a substituent, a heterocyclic thio group which may have a substituent, a lower alkylamino group which may have a substituent, a lower cycloalkylamino group which may have a substituent, an arylamino group which may have a substituent, a heterocyclic amino group which may have a substituent, an amide of an amino group, an amide of a lower alkylamino group which may have a substituent, an amide of a lower cycloalkylamino group which may have a substituent, an amide of an arylamino group which may have a substituent, an amide of a heterocyclic amino group which may have a substituent, a formyl group, a lower alkylcarbonyl group which may have a substituent, a lower cycloalkylcarbonyl group which may have a substituent, an arylcarbonyl group which may have a substituent, a heterocyclic carbonyl group which may have a substituent, a carboxy group, an ester of a carboxy group, an amide of a carboxy group, a lower alkylsulfonyl group which may have a substituent, a lower cycloalkylsulfonyl group which may have a substituent, an arylsulfonyl group which may have a substituent, a heterocyclic sulfonyl group which may have a substituent, a sulfonic acid group, an ester of a sulfonic acid group, an amide of a sulfonic acid group, a lower alkenyloxy group which may have a substituent, a silyl group which may have a substituent, -W¹-C(=O)-R¹⁸, -W¹-C(=O)-O-R¹⁸, -W¹-S(=O)-R¹⁹, -W¹-S(=O)₂-R²⁰, -W¹-C(=O)NR²¹R²²,-OCH₂CH(R²³)-OR²⁴, an amino group which may have a substituent, a nitro group, or a cyano group;
R^{18,} R¹⁹, R²⁰, R²¹ and R²² each independently represent a hydrogen atom, a lower alkyl group which may have a substituent, a lower alkenyl group which may have a substituent, a lower alkynyl group which may have a substituent, a lower cycloalkyl group which may have a substituent, an aryl group which may have a substituent, a heterocyclic group which may have a substituent, an aralkyl group which may have a substituent, a heterocyclic alkyl group which may have a substituent, an amino lower alkyl group which may have a substituent, a lower alkoxy group which may have a substituent, a lower alkenyloxy group which may have a substituent, a lower alkynyloxy group which may have a substituent, a lower cycloalkyloxy group which may have a substituent, an aryloxy group which may have a substituent, or a heterocyclic oxy group which may have a substituent;
p represents an integer of 0 to 5;
when p is 2 to 5, each R¹ may be the same or different;
W¹ represents an oxygen atom, a sulfur atom, or -N(R²⁵)-;
Z¹ represents an oxygen atom, a sulfur atom, or =N-R²⁵;
R²³ represents a hydrogen atom, a lower alkyl group which may have a substituent, a carboxy group, an ester of a carboxy group, an amide of a carboxy group, or a cyano group;
R²⁴ represents a hydrogen atom, a lower alkylcarbonyl group which may have a substituent, a lower cycloalkylcarbonyl group which may have a substituent, an arylcarbonyl group which may have a substituent, a heterocyclic carbonyl group which may have a substituent, an ester of a carboxy group, an amide of a carboxy group, a phosphoric acid group, or an ester of a phosphoric acid group;
R²⁵ represents a hydrogen atom, a lower alkyl group which may have a substituent, a lower alkenyl group which may have a substituent, a lower alkynyl group which may have a substituent, an aryl group which may have a substituent, a lower alkylcarbonyl group which may have a substituent, a lower alkenylcarbonyl group which may have a substituent, an arylalkyl group which may have a substituent, an arylalkyloxyalkyl group which may have a substituent, a heterocyclic alkyl group which may have a substituent, a silyl group which may have a substituent, a lower alkynylcarbonyl group which may have a substituent, or an arylcarbonyl group which may have a substituent;
R² represents a halogen atom, a lower alkyl group which may have a substituent, -OR⁸, -NR⁸R⁹, -SR⁸, -S(=O)-R⁸ or -S(=O)₂-R⁸;
q represents an integer of 0 to 3;
when q is 2 or 3, each R² may be the same or different;
R³ represents a hydrogen atom, a lower alkyl group which may have a substituent, a lower alkenyl group which may have a substituent, a lower alkynyl group which may have a substituent, an aryl group which may have a substituent, a lower alkylcarbonyl group which may have a substituent, a lower alkenylcarbonyl group which may have a substituent, an arylalkyl group which may have a substituent, an arylalkyloxyalkyl group which may have a substituent, a silyl group which may have a substituent, a lower alkynylcarbonyl group which may have a substituent, or an arylcarbonyl group which may have a substituent;
R⁴ and R⁵ each independently represent a hydrogen atom, a halogen atom, a lower alkyl group which may have a substituent, a lower alkenyl group which may have a substituent, a lower alkynyl group which may have a substituent, a lower cycloalkyl group which may have a substituent, an aryl group which may have a substituent, or a heterocyclic group which may have a substituent, or R⁴ and R⁵ may together form a 3- to 8-membered lower cycloalkane ring which may have a substituent;
R⁶ represents a hydrogen atom, a lower alkyl group which may have a substituent, a lower alkenyl group which may have a substituent, a lower alkynyl group which may have a substituent, a lower cycloalkyl group which may have a substituent, an aryl group which may have a substituent, or a heterocyclic group which may have a substituent;
A represents a lower alkylene group which may have a substituent or a single bond;
R⁸ and R⁹ each independently represent a hydrogen atom, a lower alkyl group which may have a substituent, a lower alkenyl group which may have a substituent, a lower alkynyl group which may have a substituent, a lower cycloalkyl group which may have a substituent, an aryl group which may have a substituent, a heterocyclic group which may have a substituent, a formyl group, a lower alkylcarbonyl group which may have a substituent, a lower alkenylcarbonyl group which may have a substituent, a lower alkynylcarbonyl group which may have a substituent, a lower cycloalkylcarbonyl group which may have a substituent, an arylcarbonyl group which may have a substituent, a heterocyclic carbonyl group which may have a substituent, a carboxy group, a lower alkoxycarbonyl group which may have a substituent, a lower alkenyloxycarbonyl group which may have a substituent, a lower alkynyloxycarbonyl group which may have a substituent, a lower cycloalkyloxycarbonyl group which may have a substituent, an aryloxycarbonyl group which may have a substituent, a heterocyclic oxycarbonyl group which may have a substituent, an arylcarbonyloxy group which may have a substituent, a heterocyclic carbonyloxy group which may have a substituent, an aryloxy group which may have a substituent, a heterocyclic oxy group which may have a substituent, an arylthio group which may have a substituent, a lower alkylsulfonyl group which may have a substituent, a lower alkenylsulfonyl group which may have a substituent, a lower alkynylsulfonyl group which may have a substituent, a lower cycloalkylsulfonyl group which may have a substituent, an arylsulfonyl group which may have a substituent, a heterocyclic sulfonyl group which may have a substituent, an arylamino group which may have a substituent, an aminocarbonyl group, a lower alkylaminocarbonyl group which may have a substituent, a lower alkenylaminocarbonyl group which may have a substituent, a lower alkynylaminocarbonyl group which may have a substituent, a lower cycloalkylaminocarbonyl group which may have a substituent, an arylaminocarbonyl group which may have a substituent, or a heterocyclic aminocarbonyl group which may have a substituent; and
when R² is NR⁸R⁹, R⁸ and R⁹ may together form a 3- to 8-membered nitrogen-containing heterocycle which may have a substituent. These compounds and a salt thereof can be comprised as components of the composition of the present invention.

The compounds shown in the above general formulas (1) and (2) are disclosed in, for example, Japanese Laid-Open Publication No. 2017-43614, Japanese Patent No. 4817020, Japanese Patent No. 4825636, Japanese Patent No. 5054996, and Japanese Patent No. 5635743 (they are incorporated herein by reference).

In another embodiment, the following compounds also can be used as SEGRM:
a compound represented by the following general formula (3), (4), (5), (6), (7), (8), (9), (10-a), (10-b), (12), (13-a), (13-b), (14), (15), (16), (17), (18), (19), or (20) 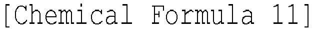 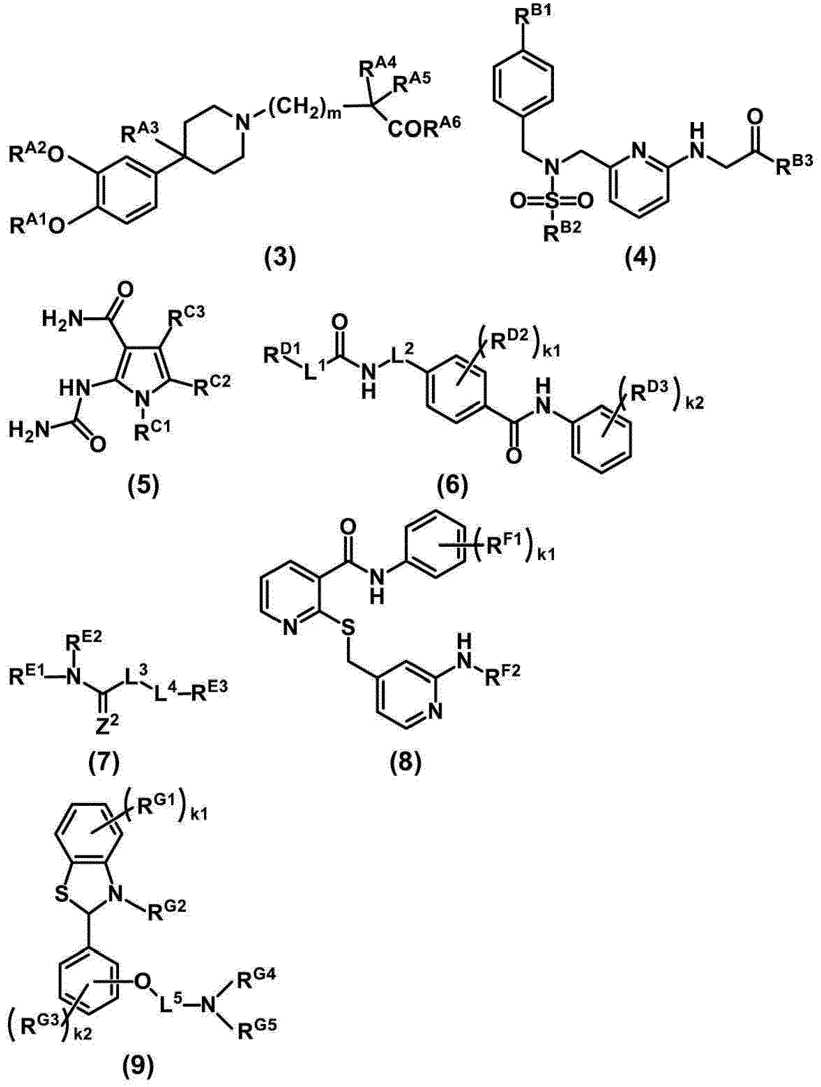 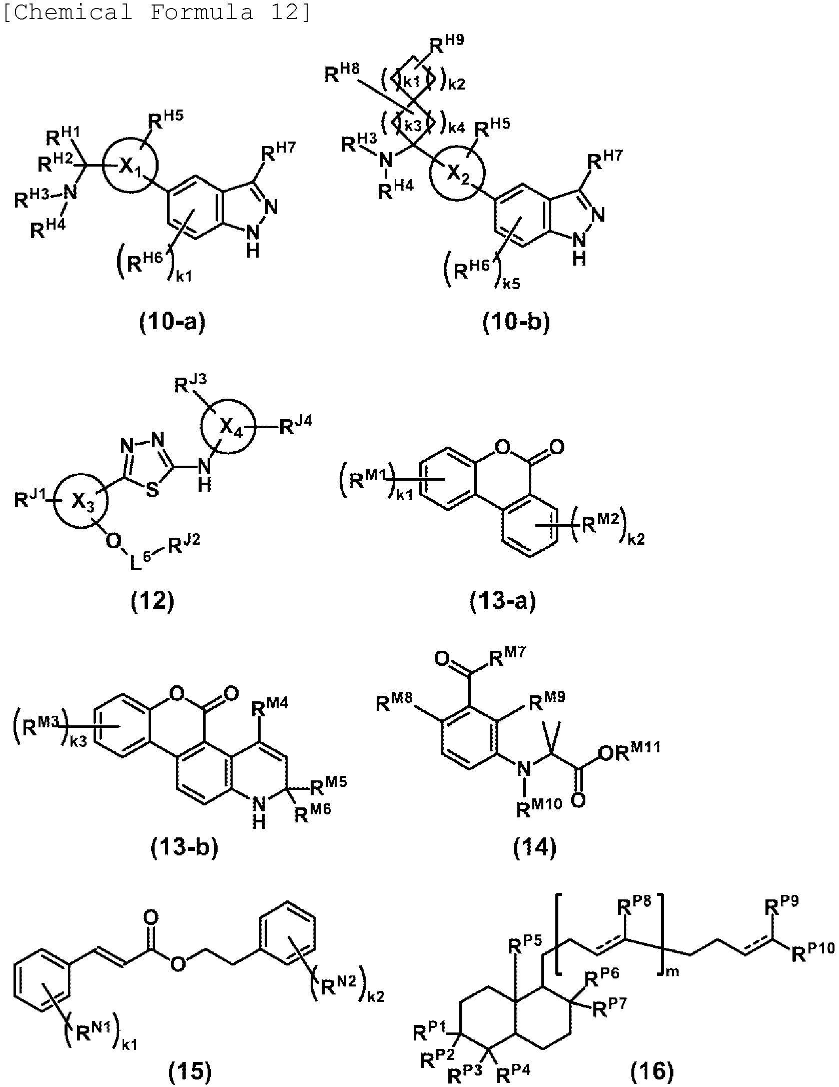 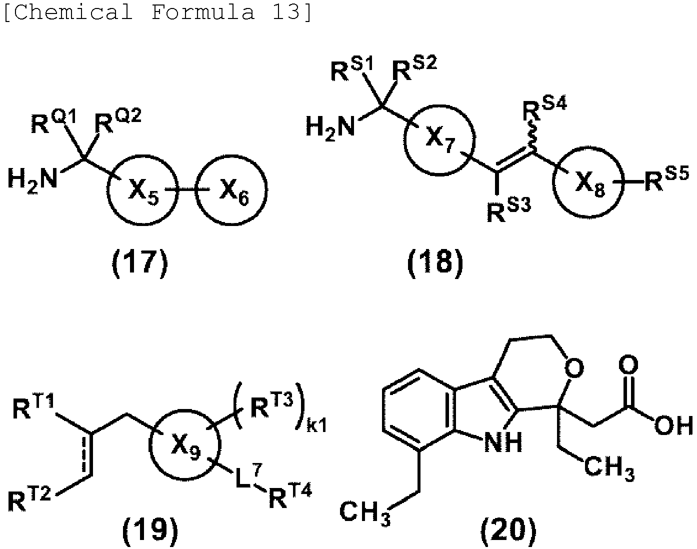 or a salt thereof, wherein

[Chemical Formula 14] **---**

represents a single bond or a double bond,
R^{A1}, R^{A2}, R^{A3}, R^{A4}, R^{A5}, R^{A6}, R^{B1}, R^{B2}, R^{B3}, R^{C1}, R^{C2}, R^{C3}, R^{D1}, R^{D2}, R^{D3}, R^{E1}, R^{E2}, R^{E3}, R^{F1}, R^{F2}, R^{G1}, R^{G2}, R^{G3}, R^{G4}, R^{G5}, R^{H1}, R^{H2}, R^{H3}, R^{H4}, R^{H5}, R^{H6}, R^{H7}, R^{H8}, R^{H9}, R^{J1}, R^{J2}, R^{J3}, R^{J4}, R^{M1}, R^{M2}, R^{M3}, R^{M4}, R^{M5}, R^{M6}, R^{M7}, R^{M8}, R^{M9}, R^{M10}, R^{M11}, R^{N1}, R^{N2}, R^{P1}, R^{P2}, R^{P3}, R^{P4}, R^{P5}, R^{P6}, R^{P7}, R^{P8}, R^{P9}, R^{P10}, R^{Q1}, R^{Q2}, R^{S1}, R^{S2}, R^{S3}, R^{S4}, R^{S5}, R^{T1}, R^{T2}, R^{T3}, and R^{T4} are each independently selected from the substituent group A,
ring X₁, X₂, X₃, X₄, X₅, X₆, X₇, X₈, and X₉ are each independently a lower cycloalkyl group which may have a substituent, a lower cycloalkenyl group which may have a substituent, an aryl group which may have a substituent, or a heterocyclic group which may have a substituent,
L¹, L², L³, L⁴, L⁵, L⁶ and L⁷ are each independently a single bond, a lower alkylene group which may have a substituent, an arylene group which may have a substituent, a heteroarylene group which may have a substituent, a heterocyclylene group which may have a substituent, -C(=O)-, -C(=O)-O-, -S(=O)-, -S(=O)₂- or -C(=O)-NH-,
each m, each k1, each k2, each k3, each k4, and each k5 are independently 0, 1, 2, 3, 4, or 5, and
a wavy line bond is a single bond representing a stereoisomerism of (E) or (Z). These compounds and a salt thereof can be comprised as components of the composition of the present invention.

In one embodiment, the following compounds also can be used as SEGRM:
a compound selected from the group consisting of (R)-trans-N-(pyridine-4-yl)-4-(1-aminoethyl)cyclohexane carboxamide: 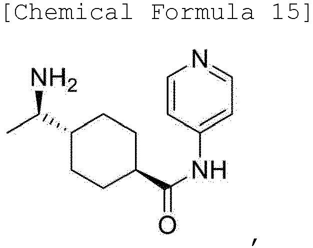 (R)-(+)-N-(1H-pyrrolo[2,3-b]pyridine-4-yl)-4-(1-aminoethyl)benzamide: 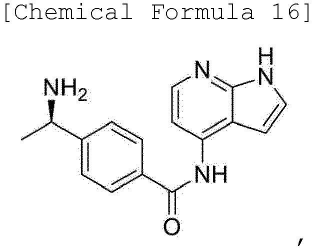 1-(5-isoquinolinesulfonyl)homopiperazine: 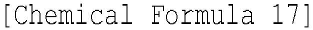 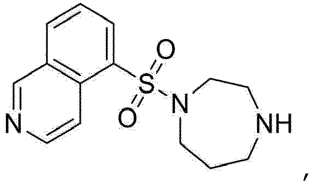 and
1-(5-isoquinolinesulfonyl)-2-methylpiperazine: 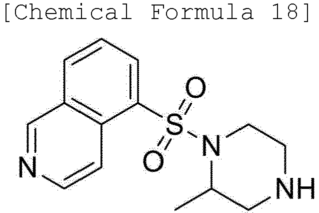 or a salt thereof. These compounds and a salt thereof can be comprised as components of the composition of the present invention.

A group "which may have a substituent" may have one or more substituents selected from the group (which may be herein referred to as the substituent group A) consisting of
a halogen atom (e.g., a fluorine atom, a chlorine atom, a bromine atom, an iodine atom or the like),
a lower alkyl group (e.g., C₁₋₈ alkyl group or the like),
a lower alkyl group substituted with a halogen atom,
a lower alkyl group substituted with a hydroxy group,
a lower alkenyl group (e.g., C₂₋₈ alkenyl group or the like), a lower alkynyl group (e.g., C₂₋₈ alkynyl group or the like), a lower cycloalkyl group (e.g., C₃₋₈ cycloalkyl group or the like),
an aryl group (e.g., a C₆₋₁₄ monocyclic or condensed polycyclic aryl group or the like),
a heterocyclic group (e.g., a residue in which one hydrogen atom has been removed from a 3- to 14-membered saturated or unsaturated monocyclic heterocycle or a condensed polycyclic aromatic hydrocarbon which is bicyclic or tricyclic having one or more heteroatoms selected from a nitrogen atom, an oxygen atom, and a sulfur atom in the ring, which may be nonaromatic or aromatic),
a hydroxy group,
an ester of a hydroxy group,
a lower alkoxy group (e.g., C₁₋₈ alkyl-O-group or the like), a lower alkoxy group substituted with a halogen atom,
a lower alkenyloxy group (e.g., C₂₋₈ alkenyl-O-group or the like),
a lower alkynyloxy group (e.g., C₂₋₈ alkynyl-O-group or the like),
a lower cycloalkyloxy group (e.g., C₃₋₈ cycloalkyl-O-group or the like),
an aryloxy group (e.g., C₆₋₁₄ or C₆₋₁₀ aryl-O-group or the like),
a heterocyclic oxy group (e.g., 3- to 14-membered or 3- to 10-membered heterocyclic-O-group or the like),
a mercapto group,
an ester of a mercapto group,
a lower alkylthio group (e.g., C₁₋₈ alkyl-S-group or the like),
a lower alkenylthio group (e.g., C₂₋₈ alkenyl-S-group or the like),
a lower alkynylthio group (e.g., C₂₋₈ alkynyl-S-group or the like),
a lower cycloalkylthio group (e.g., C₃₋₈ cycloalkyl-S-group or the like),
an arylthio group (e.g., C₆₋₁₄ aryl-S-group or the like),
a heterocyclic thio group (e.g., 3- to 14-membered or 3- to 10-membered heterocyclic-S-group or the like),
a lower alkylamino group (e.g., C₁₋₈ alkyl-NR^{10a}-group or the like),
a lower cycloalkylamino group (e.g., C₃₋₈ cycloalkyl-NR^{10a}-group or the like),
an arylamino group (e.g., C₆₋₁₄ or C₆₋₁₀ aryl-NR^{10a}-group or the like),
a heterocyclic amino group (e.g., 3- to 14-membered or 3-to 10-membered heterocyclic-NR^{10a}-group or the like),
an amide of an amino group,
an amide of a lower alkylamino group,
an amide of a lower cycloalkylamino group,
an amide of an arylamino group,
an amide of a heterocyclic amino group,
a formyl group,
a lower alkylcarbonyl group (e.g., C₁₋₈ alkyl-C(=O)-group or the like),
a lower alkenylcarbonyl group (e.g., C₂₋₈ alkenyl-C(=O)-group or the like),
a lower alkynylcarbonyl group (e.g., C₂₋₈ alkynyl-C(=O)-group or the like),
a lower cycloalkylcarbonyl group (e.g., C₃₋₈ cycloalkyl-C(=O)-group or the like),
an arylcarbonyl group (e.g., C₆₋₁₄ or C₆₋₁₀ aryl-C(=O)-group or the like),
a heterocyclic carbonyl group (e.g., 3- to 14-membered or 3- to 10-membered heterocyclic-C(=O)-group or the like),
a carboxy group,
an ester of a carboxy group,
an amide of a carboxy group,
a lower alkylsulfinyl group (e.g., C₁₋₈ alkyl-S(=O)-group or the like),
a lower cycloalkylsulfinyl group (e.g., C₃₋₈ cycloalkyl-S(=O)-group or the like),
an arylsulfinyl group (e.g., C₆₋₁₄ or C₆₋₁₀ aryl-S (=O)-group or the like),
a lower alkylsulfonyl group (e.g., C₁₋₈ alkyl-S(=O)₂-group or the like),
a lower cycloalkylsulfonyl group (e.g., C₃₋₈ cycloalkyl-S(=O)₂-group or the like),
an arylsulfonyl group (e.g., C₆₋₁₄ or C₆₋₁₀ aryl-S(=O)₂-group or the like),
a heterocyclic sulfonyl group (e.g., 3- to 14-membered or 3- to 10-membered heterocyclic-S(=O)₂-group or the like), an aralkyl group (also called arylalkyl group) (e.g., C₆₋₁₄ aryl-C₁₋₈ alkyl group and C₆₋₁₀ aryl-C₁₋₈ alkyl group or the like),
a heterocyclic alkyl group (e.g., 3- to 14-membered heterocyclic-C₁₋₈ alkyl group and 3- to 10-membered heterocyclic-C₁₋₈ alkyl group or the like),
a sulfinic acid group,
an ester of a sulfinic acid group,
an amide of a sulfinic acid group,
a sulfonic acid group,
an ester of a sulfonic acid group,
an amide of a sulfonic acid group,
a nitro group,
a cyano group,
a lower alkylaminocarbonyloxy group (e.g., C₁₋₈ alkyl-NR^{10a}-C(=O)O-group or the like),
an arylaminocarbonyloxy group (e.g., C₆₋₁₄ or C₆₋₁₀ aryl-NR^{10a}-C(=O)O-group or the like),

-NR^{10a}R^{11a},

-SO₂-NR^{10b}R^{11b},

-NR^{10c}-C(=O)R^{11c},

-NR^{10d}-C(=O)OR^{11d},

-NR^{12a}-C(=O)NR^{10e}R^{11e},

-NR^{10f}-C(=S)R^{11f},

-NR^{10g}-C(=S)OR^{11g},

-NR^{12b}-C(=S)NR^{10h}R^{11h},

-NR¹⁰ⁱ-SO₂-R¹¹ⁱ,

-NR^{12c}-SO₂-NR^{10j}R^{11j},

-C(=O)OR^{10k},

-C(=O)NR¹⁰¹R^{11k},

-C(=O)NR^{10m}OR¹¹¹,

-C(=O)NR^{12d}-NR¹⁰ⁿR^{11m},

-C(=S)OR^{10o},

-C(=S)NR^{10p}R¹¹ⁿ,

-C(=S)NR^{10q}OR^{11o},

-C(=S)NR^{12e}-NR^{10r}R^{11p},

-C(=NR^{13a})R^{10s},

-C(=NR^{13b})CHO,

-C(=NR^{13c})NR^{10t}R^{11q},

-C(=NR^{13d})NR^{12f}-NR^{10u}R^{11r},

-NR^{17c}-C(=NR^{13k})R^{17d},

-NR^{12g}-C(=NR^{13e})-NR^{10v}R^{11s},

-NR¹⁴-C(=NR^{13f})-NR^{12h}-NR^{10w}R^{11t},

-OC(=O)R^{10x},

-OC(=O)OR^{10y},

-OC(=O)NR^{10z1}R^{11u},

-NR¹²ⁱ-NR^{10z2}R^{11v},

-NR^{10z3}OR^{11w},

-C(=N-OR^{13a})R^{10s},

-C(=N-OR^{13b})CHO,

-C(=N-OR^{13c})NR^{10t}R^{11q}

and
-C(=N-OR^{13d})NR^{12f}-NR^{10u}R^{11r}. The alkyl group, alkenyl group, alkynyl group, alkoxy group, cycloalkyl group, aryl group, heterocyclic group, hydroxy group, mercapto group, and carboxy group (as one group it self or a part of a certain group) may be substituted with one or more substituents selected from the group (which may be herein referred to as the substituent group B) consisting of
a halogen atom,
a hydroxy group,
a carboxy group,
a cyano group,
a lower cycloalkyl group,
a lower alkoxy group,
a lower cycloalkyloxy group,
a lower alkylthio group,
a 5- or 6-membered heteroarylthio group, C₆₋₁₀ aryl,
a 5- or 6-membered aromatic heterocycle,
a 4- to 10-membered nonaromatic heterocycle,
a lower alkylcarbonyl group,
a lower cycloalkylcarbonyl group,
an arylcarbonyl group,
a 5- or 6-membered aromatic heterocyclic carbonyl group,
a 4- to 10-membered nonaromatic heterocyclic carbonyl group,

-NR^{15a}R^{16a},

-SO₂-NR^{15b}R^{16b},

-NR^{15c}-C(=O)R^{16c},

-NR^{17a}-C(=O)NR^{15d}R^{16d},

-C(=O)NR^{15e}R^{16e},

-C(=NR^{13g})R^{15f},

-C(=NR^{13h})NR^{15g}R^{16f},

-NR^{16g}-C(=NR¹³ⁱ)R^{15h},

-NR^{17b}-C(=NR^{13j})-NR¹⁵ⁱR^{16h},

-C(=N-OR^{13g})R^{15f}

and
-C(=N-OR^{13h})NR^{15g}R^{16f}. R^{13a}, R^{13b}, R^{13c}, R^{13d}, R^{13e}, R^{13f}, R^{13g}, R^{13h}, R¹³ⁱ, R^{13j}, and R^{13k} in the above formulas are the same or different and are each independently a hydrogen atom, a hydroxy group, a C₁₋₆ alkyl group, or a C₁₋₆ alkoxy group, R^{10a}, R^{10b}, R^{10c}, R^{10d}, R^{10e}, R^{10f}, R^{10g}, R^{10h}, R¹⁰ⁱ, R^{10j}, R^{10k}, R^{10l}, R^{10m}, R¹⁰ⁿ, R^{10o}, R^{10p}, R^{10q}*,* R^{10r}, R^{10s}, R^{10t}*,* R^{10u}, R^{10v}, R^{10w}, R^{10x}, R^{10y}*,* R^{10z1}, R^{10z2}*,* R^{10z3}, R^{11a}, R^{11b}*,* R^{11c}, R^{11d}, R^{11e}, R^{11f}, R^{11g}, R^{11h}, R¹¹ⁱ*,* R^{11j}, R^{11k}, R^{11l}, R^{11m}, R¹¹ⁿ, R^{11o}, R^{11p}, R^{11q}, R^{11r}, R^{11s}, R^{11t}*,* R^{11u}, R^{11v}, R^{11w}, R^{12a}, R^{12b}, R^{12c}, R^{12d}, R^{12e}, R^{12f}, R^{12g}, R^{12h}, R¹²ⁱ, R¹⁴*,* R^{15a}, R^{15b}, R^{15c}, R^{15d}, R^{15e}, R^{15f}, R^{15g}, R^{15h}, R¹⁵ⁱ, R^{16a}, R^{16b}, R^{16c}, R^{16d}, R^{16e}, R^{16f}, R^{16g}, R^{16h}, R^{17a}, R^{17b}, R^{17c}, and R^{17d} are the same or different and are each independently a hydrogen atom or a C₁₋₆ alkyl group (said group may be substituted with 1 to 3 substituents that are the same or different and are selected from a hydroxy group, a cyano group, a C₁₋₆ alkoxy group, and -NR^{18a}R^{18b}), and R^{18a} and R^{18b} are the same or different and are each independently a hydrogen atom or a C₁₋₆ alkyl group. These compounds and a salt thereof can be comprised as components of the composition of the present invention.

The above compounds that can be used as SEGRM are disclosed in Japanese Laid-Open Publication No. 2017-186375, Japanese Laid-Open Publication No. 2017-31177, Japanese Laid-Open Publication No. 2017-14269, Japanese Laid-Open Publication No. 2017-43614, Japanese Laid-Open Publication No. 2017-14209, International Publication No. WO 2015/105144, Japanese Laid-Open Publication No. 2016-27060, Japanese Laid-Open Publication No. 2015-147763, Japanese Laid-Open Publication No. 2015-147762, Japanese Laid-Open Publication No. 2015-7122, Japanese Laid-Open Publication No. 2015-57381, Japanese Laid-Open Publication No. 2014-208611, Japanese Laid-Open Publication No. 2014-139161, Japanese Laid-Open Publication No. 2014-31369, Japanese Laid-Open Publication No. 2013-166752, Japanese Laid-Open Publication No. 2014-19650, Japanese Laid-Open Publication No. 2012-180345, Japanese National Phase PCT Laid-Open Publication No. 2009-538879, Japanese Laid-Open Publication No. 2010-90117, Japanese Laid-Open Publication No. 2010-77119, Japanese Laid-Open Publication No. 2009-298775, Japanese Laid-Open Publication No. 2009-143940, International Publication No. WO 2007/142323, Japanese Laid-Open Publication No. 2009-29828, Japanese Laid-Open Publication No. 2009-84274, Japanese Laid-Open Publication No. 2009-84273, Japanese Laid-Open Publication No. 2009-7344, Japanese Laid-Open Publication No. 2008-266323, Japanese Laid-Open Publication No. 2008-255112, Japanese Laid-Open Publication No. 2008-266295, Japanese Laid-Open Publication No. 2008-143913, Japanese Laid-Open Publication No. 2008-143891, Japanese Laid-Open Publication No. 2008-143890, Japanese Laid-Open Publication No. 2008-143889, Japanese Laid-Open Publication No. 2008-74829, Japanese Laid-Open Publication No. 2007-230993, Japanese Laid-Open Publication No. 2006-257080, International Publication No. WO 2005/035506, Japanese Laid-Open Publication No. 2006-117654, Japanese Laid-Open Publication No. 2006-117653, Japanese Laid-Open Publication No. 2007-77049, Japanese Laid-Open Publication No. 2005-336174, Japanese Laid-Open Publication No. 2005-336173, Japanese Laid-Open Publication No. 2007-297283, Japanese Laid-Open Publication No. 2005-47909, Japanese Laid-Open Publication No. 2005-41866, Japanese Laid-Open Publication No. 2004-256524, International Publication No. WO 2003/011276, Japanese Laid-Open Publication No. 2007-15928, Japanese Laid-Open Publication No. 2007-8816, Japanese Laid-Open Publication No. 2004-137267, Japanese Laid-Open Publication No. 2004-107335, Japanese Laid-Open Publication No. 2004-2352, Japanese Laid-Open Publication No. 2004-331501, Japanese Laid-Open Publication No. 4-134093, International Publication No. WO 2017/034006, International Publication No. WO 2017/002941, International Publication No. WO 2015/105135, International Publication No. WO 2015/105134, International Publication No. WO 2015/020204, International Publication No. WO 2014/148574, International Publication No. WO 2014/098046, International Publication No. WO 2014/010654, International Publication No. WO 2012/108455, International Publication No. WO 2010/029986, International Publication No. WO 2009/139361, International Publication No. WO 2009/035068, International Publication No. WO 2009/035067, International Publication No. WO 2008/146871, International Publication No. WO 2008/111632, International Publication No. WO 2008/093674, International Publication No. WO 2008/059867, International Publication No. WO 2008/059866, International Publication No. WO 2008/059865, International Publication No. WO 2007/105766, International Publication No. WO 2007/032556, International Publication No. WO 2005/102331, International Publication No. WO 2005/007161, and International Publication No. WO 2004/069822 (they are incorporated herein by reference).

In one embodiment, a selective glucocorticoid receptor agonist (SEGRA) has a steroid as a basic skeleton, and can be selected from the group consisting of, for example, fluticasone (e.g., fluticasone propionate or fluticasone furoate), flumethasone, 9-fluoro-11β-hydroxy-16α-methyl-3,20-dioxopregna-1,4-dien-21-carbonitrile (RU-24858).

Compounds that can be used as SEGRA are disclosed in US Patent No. 8,663,639 (which is incorporated herein by reference).

In one embodiment, a selective glucocorticoid receptor modulator (SEGRM) can be selected from, but is not limited to, the group consisting of: Mapracorat, (+)-4-(2,3-dihydro-1-benzofuran-7-yl)-2-hydroxy-4-methyl-N-(4-methyl-1-oxo-2,3-benzoxazine-6-yl)-2-(trifluoromethyl)pentanamide (ZK216348), (±)-4-(2,3-dihydro-1-benzofuran-7-yl)-2-hydroxy-4-methyl-N-(4-methyl-1-oxo-2,3-benzoxazine-6-yl)-2-(trifluoromethyl)pentanamide (ZK209614), Dagrocorat, Fosdagrocorat, 2-((4-acetoxyphenyl)-2-chloro-N-methyl)ethylammonium chloride (Compound A), 10-methoxy-2,2,4-trimethyl-5-prop-2-enyl-1,5-dihydrochromeno[3,4-f]quinoline (AL-438), 5-[(Z)-2-fluoro-3-methylbenzylidene]-2,5-dihydro-10-metoxy-2,2,4-trimethyl-1H-6-oxa-1-azachrysene-9-ol (LGD-5552), (4aR)-6-(4-tert-butylphenyl)sulfonyl-1-(4-fluorophenyl)-4a-(2-methoxyethoxymethyl)-4,5,7,8-tetrahydropyrazolo[3,4-g]isoquinoline (C108297), [(-)-N-(2S,10S,14bS)]-N-(8-cyano-1,2,3,4,10,14b-hexahydro-10-methyldibenzo[c,f]pyrido[1,2-a]azepin-2-yl)-4-methyl-1,2,3-thiadiazole-5-carboxamide (Org 214007-0), and (4bS,7R,8aR)-4b-benzyl-7-hydroxy-N-(2-methylpyridin-3-yl)-7-(trifluoromethyl)-4b,5,6,7,8,8a,9,10-octahydrophenanthrene-2-carboxamide (PF-802).

As SEGRM, a compound having the following structure: 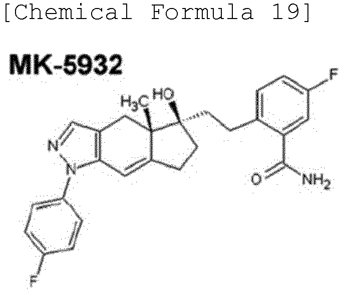 (MK-5932), or a compound having the following structure: 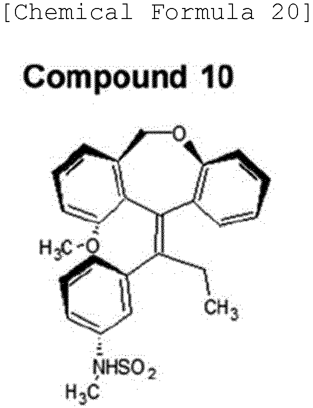 (Compound 10)
may be used.

As described above, SEGRA has a steroid skeleton, whereas SEGRM does not have a steroid skeleton, but both exhibited inhibiting activity against TGF-β. Although not wishing to be bound by any theory, it is suggested that a compound exhibits inhibiting activity against TGF-β as long as the compound has agonist activity for a glucocorticoid receptor regardless of whether the compound has a steroid skeleton. In addition, these compounds or a salt thereof can be comprised as components of the composition of the present invention as long as they exhibit the above activity regardless of the form or structure.

In one embodiment, examples of utilization methods of the present invention include, but are not limited to, eye drops, as well as administration methods such as injection into the anterior chamber, impregnation into a controlled-release agent, subconjunctival injection, and systemic administration (oral administration and intravenous injection).

The above compounds may be used alone or in combination in the medicament of the present invention. The concentration of a compound used in the present invention is about 0.001 nM (nmol/l) to 100 µM (µmol/l), 0.01 nM to 100 µM, or about 0.1 nM to 100 µM, generally about 1 nM to 100 µM, about 10 nM to 100 µM, preferably about 0.1 to 30 µM, more preferably about 1 to 10 µM, and the upper limit and the lower limit thereof can be appropriately set in combination. When two or more types of compounds are used in combination, the concentration can be appropriately changed. Examples of other concentration ranges can include, but are not limited to, generally about 0.1 nM to 100 µM, or about 0.001 to 100 µM, preferably about 0.01 to 75 µM, about 0.05 to 50 µM, about 1 to 50 µM, about 0.01 to 10 µM, about 0.05 to 10 µM, about 0.075 to 10 µM, about 0.1 to 10 µM, about 0.5 to 10 µM, about 0.75 to 10 µM, about 1.0 to 10 µM, about 1.25 to 10 µM, about 1.5 to 10 µM, about 1.75 to 10 µM, about 2.0 to 10 µM, about 2.5 to 10 µM, about 3.0 to 10 µM, about 4.0 to 10 µM, about 5.0 to 10 µM, about 6.0 to 10 µM, about 7.0 to 10 µM, about 8.0 to 10 µM, about 9.0 to 10 µM, about 0.01 to 5.0 µM, about 0.05 to 5.0 µM, about 0.075 to 5.0 µM, about 0.1 to 5.0 µM, about 0.5 to 5.0 µM, about 0.75 to 5.0 µM, about 1.0 to 5.0 µM, about 1.25 to 5.0 µM, about 1.5 to 5.0 µM, about 1.75 to 5.0 µM, about 2.0 to 5.0 µM, about 2.5 to 5.0 µM, about 3.0 to 5.0 µM, about 4.0 to 5.0 µM, about 0.01 to 3.0 µM, about 0.05 to 3.0 µM, about 0.075 to 3.0 µM, about 0.1 to 3.0 µM, about 0.5 to 3.0 µM, about 0.75 to 3.0 µM, about 1.0 to 3.0 µM, about 1.25 to 3.0 µM, about 1.5 to 3.0 µM, about 1.75 to 3.0 µM, about 2.0 to 3.0 µM, about 0.01 to 1.0 µM, about 0.05 to 1.0 µM, about 0.075 to 1.0 µM, about 0.1 to 1.0 µM, about 0.5 to 1.0 µM, about 0.75 to 1.0 µM, about 0.09 to 3.5 µM, about 0.09 to 3.2 µM, and more preferably about 0.05 to 1.0 µM, about 0.075 to 1.0 µM, about 0.1 to 1.0 µM, about 0.5 to 1.0 µM, and about 0.75 to 1.0 µM.

When used as an eye drop, the formulation concentration can be determined using about 1 to 10000-fold, preferably about 100 to 10000-fold such as about 1000-fold of the above effective concentration as a reference while considering dilution with tear fluid or the like and paying attention to toxicity. It is also possible to set a higher concentration. For example, the concentration is about 0.01 µM (µmol/l) to 1000 mM (mmol/l), about 0.1 µM to 100 mM, about 1 µM to 100 mM, about 10 µM to 100 mM, or about 0.1 µM to 30 mM, about 1 µM to 30 mM, more preferably about 1 µM to 10 mM, about 10 µM to 10 mM, about 100 µM to 10 mM, about 10 µM to 100 mM, about 100 µM to 100 mM, or can be about 1 mM to 10 mM, about 1 mM to 100 mM. The upper limits and lower limits thereof can be appropriately set in combination and when two or more types of compounds are used in combination, the concentration can be appropriately changed.

In one embodiment, a therapeutic or prophylactic medicament of the present invention can be targeted for any animal with a corneal endothelium, such as mammals. Such a medicament is preferably intended for treating or preventing a primate corneal endothelium. The subject of therapy or prophylaxis is preferably a human corneal endothelium.

In another aspect, the present invention provides a method for treating or preventing a corneal endothelial condition, disorder, or disease, comprising administering an effective amount of a compound of the present invention to a subject in need thereof.

As used herein, a "subject" refers to a target of administration (transplant) of a therapeutic or prophylactic medicament or method of the present invention. Examples of subjects include mammals (e.g., human, mouse, rat, hamster, rabbit, cat, dog, cow, horse, sheep, monkey and the like), but primates are preferable and humans are especially preferable. As used herein, an "object" refers to an object which a compound of the present invention or a composition comprising the compound, or a caspase inhibitor is administered to or contacted with (e.g., organism such as a human (e.g., the above subject), or organ or cell extracted from the organism, or the like).

The effective amount of the medicament of the present invention, which is effective in treating a specific disease, disorder, or condition, can vary depending on the properties of a disorder or condition, but the effective amount can be determined by those skilled in the art with standard clinical techniques based on the descriptions in the present specification. It is also possible to use an in vitro assay to assist in identifying the optimal range of dosage as needed. Since an accurate dose to be used in a formulation can vary depending on the route of administration and the severity of a disease or disorder, the dose should be determined in accordance with the judgment of a physician and the condition of each patient. However, the dosage, while not particularly limited, may be, for example, 0.001, 1, 5, 10, 15, 100, or 1000 mg/kg body weight or a value between any two such values per dose. The interval of administration, while not particularly limited, may be for example one or two doses for every 1, 7, 14, 21, or 28 days, or one or two doses for a number of days between any two such values. The dosage, number of doses, administration interval, and administration method may be appropriately selected depending on the age or body weight of a patient, condition, dosage form, target organ, or the like. For example, the present invention can be used as an eye drop. The medicament of the present invention can also be injected into the anterior chamber. A therapeutic drug preferably comprises a therapeutically effective amount or an effective amount of active ingredients at which a desired action is exerted. It may be determined that there is a therapeutic effect when a therapeutic marker significantly decreases after administration. The effective amount can be estimated from a dose-response curve obtained from an in vitro or animal model testing system.

### <Caspase inhibitor>

A compound used in the present invention also can be useful as a caspase inhibitor. It is understood that the compound used in the present invention as a caspase inhibitor can include any compound described in the section of <Medicament> continuing to the preceding paragraph or a combination thereof, and any embodiment described in <Medicament> is available as a caspase inhibitor. As described above, it is known that TGF-β is a factor inducing cell death (apoptosis). It is understood that a compound used in the present invention is useful as a caspase inhibitor (e.g., reagent, or component of a pharmaceutical product) because said compound can suppress caspase activity (e.g., caspase 3/7 activity) in the presence of TGF-β. Further, a part of compounds that can suppress caspase activity in the presence of TGF-β also can suppress caspase activity in the presence of MG-132. It is understood that such compounds are very strong caspase activity because said compounds can suppress caspase activity which is induced by various factors.

### <Composition for preservation>

In another aspect, the present invention provides a composition for preservation of corneal endothelial cells, comprising the above compound. It is understood that a compound which is used as a component of a composition for preservation of corneal endothelial cells in the present invention can include any compound described in the section of <Medicament> or a combination thereof, and any embodiment described in <Medicament> is available as a composition for preservation of corneal endothelial cells. In a preferred embodiment, preservation is cryopreservation. It is understood that a compound used in the present invention can have any form explained herein, such as an embodiment that is suitable as a composition for preservation among the embodiments explained as a medicament. As used herein, a "composition for preservation" is a composition for preserving a cornea fragment extracted from a donor until the fragment is transplanted into a recipient, or for preserving corneal endothelial cells before being grown or after being grown.

In one embodiment, the composition for preservation of the present invention can be prepared by adding a compound of the present invention to a conventionally used preservative or preservation solution. Examples of such a cornea preservation solution include preservation solutions that are commonly used for corneal transplant (sclerocornea fragment preservation solution (Optisol GS®) or eye ball preservation solution for corneal transplant (EPII®)), saline, phosphate-buffered saline (PBS) and the like.

The composition for preservation of the present invention is used for preserving a cornea that is used in organ transplant or the like. The composition for preservation of the present invention is also used as a preservation solution for cryopreserving corneal endothelial cells or as a component thereof.

In another embodiment of the composition for preservation of the present invention used for cryopreservation, an existing cryopreservation solution can be used by adding the composition for preservation comprising a caspase inhibitor of the present invention. Examples of a cryopreservation solution include, but are not limited to, CELLBANKER® series provided by Takara Bio (CELL BANKER PLUS (catalog number: CB021), CELL BANKER 2 (catalog number: CB031), STEM-CELLBANKER (catalog number: CB043) and the like), KM BANKER (Kohjin Bio, catalog number: KOJ-16092005), and Freezing Medium, Animal Component Free, CRYO Defined (also denoted as Cnt-CRYO) (CELLNTEC, catalog number: CnT-CRYO-50). In yet another embodiment, the cryopreservation solution used may be KM BANKER. It is understood that those skilled in the art can use a suitable modified cryopreservation solution by appropriately changing a constituent component of the above cryopreservation solution or by adding an additional constituent component. Glycerol, dimethyl sulfoxide, propylene glycol, acetamide, or the like may be further added to the preservation solution of the present invention for cryopreservation.

Reference literature such as scientific literature, patents, and patent applications cited herein is incorporated herein by reference to the same extent that the entirety of each document is specifically described.

The present invention has been explained while showing preferred embodiments to facilitate understanding. The present invention is explained hereinafter based on Examples. The above explanation and the following Examples are not provided to limit the present invention, but for the sole purpose of exemplification. Thus, the scope of the present invention is not limited to the embodiments and Examples that are specifically disclosed herein and is limited only by the scope of claims.

### [Examples]

Hereinafter, examples of the present invention are described. Biological samples or the like, where applicable, were handled in compliance with the standards enacted by the Ministry of Health, Labour and Welfare, Ministry of Education, Culture, Sports, Science and Technology, or the like and, where applicable, based on the Helsinki Declaration or ethical codes prepared based thereon. For the donation of eyes used for the study, consent was obtained from close relatives of all deceased donors. The present study was approved by the ethics committee or a corresponding body of the University of Erlangen-Nuremberg (Germany) and SightLife™ (Seattle, WA) eye bank.

### (Preparation Example: Production of Fuchs' endothelial corneal dystrophy patient derived immortalized corneal endothelial cell line (iFECD) and immortalized cells of normal corneal endothelial cells (iHCEC))

In this example, immortalized corneal endothelial cell lines (iFECD and iHCEC) were made from corneal endothelial cells from Fuchs' endothelial corneal dystrophy patients and healthy subjects.

### (Culture method)

Corneal endothelial cells were mechanically peeled off with a basal membrane from a cornea for research purchased from the Seattle Eye Bank. After using collagenase to detach and collect the corneal endothelial cell from the basal membrane, the cells were subjected to primary culture. For a medium, Opti-MEM I Reduced-Serum Medium, Liquid (INVITROGEN catalog number.: 31985-070), to which 8% FBS (BIOWEST, catalog number: S1820-500), 200 mg/ml of CaCl₂•2H₂O (SIGMA catalog number: C7902-500G), 0.08% of chondroitin sulfate (SIGMA catalog number: C9819-5G), 20 µg/ml of ascorbic acid (SIGMA catalog number: A4544-25G), 50 µg/ml of gentamicin (INVITROGEN catalog number: 15710-064) and 5 ng/ml of EGF (INVITROGEN catalog number: PHG0311) were added, and conditioned for a 3T3 feeder cell was used as a basal medium. Further, the cells were cultured in a basal medium to which SB431542 (1 µmol/l) and SB203580 (4-(4-fluorophenyl)-2-(4-methylsulfonylphenyl)-5(4-pyridyl) imidazole<4-[4-(4-fluorphenyl)-2-(4-methylsulfinylphenyl)-1 H-imidazole-5-yl]pyridine) (1 µmol/l) were added (referred to as "SB203580+SB431542+3T3 conditioned medium").

### (Method of acquisition)

Corneal endothelial cells were obtained with approval from an ethics committee and written consent from 3 human patients who suffered from bullous keratopathy according to a clinical diagnosis of Fuchs' endothelial corneal dystrophy and underwent corneal endothelial transplant (Descemet's Membrane Endothelial Keratoplasty = DMEK). For DMEK, pathological corneal endothelial cells were mechanically peeled off with the basal membrane, i.e., the Descemet's membrane, and immersed in a cornea preservation solution Optisol-GS (Bausch & Lomb). Collagenase treatment was then applied to enzymatically collect the corneal endothelial cells, and the cells were cultured in a SB203580+SB431542+3T3 conditioned medium. For cultured corneal endothelial cells from a Fuchs' endothelial corneal dystrophy patient, SV40 large T antigen and hTERT gene were amplified by PCR and introduced into a lentiviral vector (pLenti6.3_V5-TOPO; Life Technologies Inc). The lentiviral vector was then used to infect 293T cells (RCB2202; Riken Bioresource Center, Ibaraki, Japan) with a transfection reagent (Fugene HD; Promega Corp., Madison, WI) and three types of helper plasmids (pLP1, pLP2, pLP/VSVG; Life Technologies Inc.). Culture supernatant comprising viruses was collected after 48 hours from the infection. 5 µg/ml of polybrene was used and added to a culture solution of cultured corneal endothelial cells from a Fuchs' endothelial corneal dystrophy patient, and SV40 large T antigen and hTERT gene were introduced. Images of immortalized corneal endothelial cell line (iFECD) from Fuchs' endothelial corneal dystrophy patients from a phase difference microscope were studied. Cultured corneal endothelial cells from a research cornea imported from the Seattle Eye Bank were immortalized by the same method to make an immortalized cell line of normal corneal endothelial cells (iHCEC). When images of the immortalized corneal endothelial cell line (iFECD) and the immortalized corneal endothelial cell line from a healthy donor (iHCEC) from a phase difference microscope are studied, both iHCEC and iFECD have a layer of polygonal form as in normal corneal endothelial cells. IHCEC and iFECD were maintained and cultured in Dulbecco's Modified Eagle Medium (DMEM) + 10% fetal bovine serum (FBS).

### (Example 1: Confirmation of inhibiting activity against TGF-β using immortalized Fuchs' endothelial corneal dystrophy cells)

In this example, immortalized Fuchs' endothelial corneal dystrophy cells made in the above preparation example were used to confirm inhibiting activity of an agent against TGF-β. In this example, TGF-β2 was used as a substance inducing cell damage of corneal endothelial cells.

### (Materials and Methods)

7 × 10³ immortalized Fuchs' endothelial corneal dystrophy cells were seeded on a 96-well plate and were cultured for 24 hours under the condition of 5% CO₂ at 37°C. Dulbecco's Modified Eagle Medium (DMEM) (nacalai tesque, 26252-94) + 10% FBS (Biological Industries/04-001-1A) + 1% penicillin-streptomycin (nacalai tesque, 26252-94) <DMEM + 10% FBS + 1% P/S> was used as the medium.

Next, TGF-β2 (manufacturer: R&D Systems, Inc., distributor: Wako Pure Chemical Industries, Ltd./manufacturer codes 302-B2-002, 302-B2-010, distributor codes: 553-62881, 559-62883) (5 ng/mL) alone, or both TGF-β2 (5 ng/mL) and each agent of SCREEN-WELL® FDA Approved Drug Library V2, Japan version (10 µM) were added. After 24 to 28 hours under the condition of 5% CO₂ at 37°C, cell morphology was observed using a phase difference microscope. Caspase 3/7 activity was then measured using Caspase-Glo 3/7 Assay (Promega, #G8091). DMEM + 2% FBS + 1% P/S was used as the medium. Caspase 3/7 activity and cell viability in this example were measured using Veritas® Microplate Luminometer. Such a device enables many samples to be simultaneously measured.

The control group was supplemented with a solvent of each reagent, i.e., DMSO (Dimethyl Sulfoxide, Sterile-filtered) (nacalai tesque, 13408-64). Further, Z-VD-FMK (isomer mixture) (Wako Pure Chemical Industries, Ltd./262-02061) (10 µM), which is a caspase inhibitor, was used as the positive control.

### (Results)

Caspase 3/7 activity was evaluated, assuming that the TGF-β2 (5 ng/mL) supplemented group is 100% (n = 2). Caspase 3/7 activity was measured with n = 2 for each of the control group, TGF-β2 (5 ng/mL) supplemented group, TGF-β2 (5 ng/mL) + Z-VD-FMK supplemented group (10 µM), and TGF-β2 (5 ng/mL) + each agent of SCREEN-WELL® FDA Approved Drug Library V2, Japan version supplemented group (10 µM). Compounds with caspase 3/7 activity of less than 100% (that is, compounds discovered to exhibit any caspase inhibiting activity) are shown in Table 4. These compounds are compounds that had not been discovered to exhibit caspase inhibiting activity. All of these compounds can be useful as a caspase inhibiting reagent, and can be useful as a composition for treating or preventing a corneal endothelial condition, disorder (e.g., Fuchs' endothelial corneal dystrophy).

**[Table 4-1]**

| | | |
|---|---|---|
| Amlexanox | Didanosine | Neomycin Sulfate |
| Leflunomide | Phenelzine Sulfate | Isosorbide Dinitrate |
| Olsalazine•Na | Erythromycin | Indapamide |
| Febuxostat | Acrivastine | Bethanechol Chloride |
| Nitazoxanide | Esomeprazole Potassium | Oxtriphylline |
| Butoconazole Nitrate | Gemfibrozil | L-Ascorbic Acid |
| Nelfinavir Mesylate | Aprepitant | Trientine Dihydrochloride |

**[Table 4-2]**

| | | |
|---|---|---|
| Mometasone Furoate | Penciclovir | Etidronate Disodium |
| Mycophenolate Mofetil | Tiagabine•HCl | Sulconazole Nitrate |
| Salmeterol | Doxycycline Monohydrate | Methenamine Hippurate |
| Mycophenolic Acid | Guanabenz Acetate | Minocycline |
| Hydroxocobalamin•HCl | Naloxone•HCl | Acetohexamide |
| Deferasirox | Penicillin V Potassium | Nelarabine |
| Carvedilol | Tranylcypromine Hemisulfate | Articaine•HCl |
| Thioridazine•HCl | Bupivacaine•HCl | Pyrazinamide |
| Fluoxetine•HCl | Dimenhydrinate | Zafirlukast |
| Flurbiprofen | Phytonadione | Clavulanate Potassium |
| Terazosin•HCl | Etonogestrel | Nicotine |
| Fulvestrant | Glyburide | Mepivacaine•HCl |
| Orphenadrine Citrate | Cefprozil | Aminohippurate•Na |
| Fluorouracil (5-Fluorouracil) | Levetiracetam | Decitabine |
| Reserpine | Piperacillin | Benazepril•HCl |
| Methylprednisolone | Clemastine Fumarate | Telithromycin |
| Calcipotriene | Thioguanine (6-Thioguanine) | Lisinopril•2H₂O |

**[Table 4-3]**

| | | |
|---|---|---|
| Dexamethasone | Metoclopramide•HCl | Tamsulosin•HCl |
| Betamethasone | Flunisolide | Darunavir |
| Difluprednate | Trihexyphenidyl•HCl | Betaine |
| Ketoconazole | Clomipramine•HCl | Mesalamine (5-Aminosalicylic Acid) |
| Toremifene Base | Nevirapine | Argatroban |
| Prednisolone | Clarithromycin | Olanzapine |
| Fludrocortisone Acetate | Mupirocin | Chlorothiazide |
| Sirolimus (Rapamycin) | Levonorgestrel | Tiludronate Disodium |
| Flurandrenolide | Tropicamide | Trimethoprim |
| Amcinonide | Niacin (Known as Vitamin B3, Nicotinic Acid, And Vitamin PP) | Fluconazole |
| Budesonide | Almotriptan malate | Cilastatin•Na |
| Rimantadine•HCl | Ipratropium•Br | Enalapril |
| Clobetasol Propionate | Indinavir | Ciprofloxacin |
| Esmolol | Tacrine•HCl | Cefazolin•Na |
| Megestrol Acetate | Butenafine•HCl | Amoxicillin |
| Fluocinolone Acetonide | Cyclopentolate | Methacholine Chloride |

**[Table 4-4]**

| | | |
|---|---|---|
| Tolterodine Tartrate | Mefenamic Acid | Terbutaline Hemisulfate |
| Imipramine•HCl | Risperidone | Enalapril Maleate |
| Beclomethasone Dipropionate | Sulfasalazine | Darifenacin•HBr |
| Tetracycline | Deferoxamine Mesylate | Oxcarbazepine |
| Trimipramine Maleate | Phenytoin | Lamivudine |
| Pyrimethamine | Fosinopril•Na | Dantrolene•Na |
| Atomoxetine•HCl | Dalfampridine (4-Aminopyridine) | Verapamil•HCl |
| Dobutamine•HCl | Rasagiline Mesylate | Clindamycin Palmitate•HCl |
| Milrinone | Gemifloxacin | Tobramycin |
| Amoxapine | Theophylline | Ganciclovir |
| Paliperidone | Aspirin (Acetylsalicylic Acid) | Leucovorin Calcium Pentahydrate |
| Desonide | Nizatidine | Zolmitriptan |
| Oxaliplatin | 4-Aminosalicylic Acid | Mitotane |
| Fluocinonide | Benztropine Mesylate | Stavudine |
| Everolimus | Isotretinoin (13-Cis-Retinoic Acid) | Ambrisentan |
| Apomorphine•HCl Hemihydrate | Amifostine | Biperiden•HCl |

**[Table 4-5]**

| | | |
|---|---|---|
| Tolcapone | Quetiapine Fumarate | Cefuroxime Axetil |
| Rifapentine | Homatropine Methylbromide | Lincomycin•HCl |
| Bromocriptine Mesylate | Tramadol•HCl | Posaconazole |
| Nisoldipine | Tenofovir | Isocarboxazid |
| Droperidol | Valacyclovir•HCl | Fosfomycin Calcium |
| Diphenhydramine•HCl | Bupropion | Balsalazide |
| Anastrozole | Etomidate | Nepafenac |
| Mifepristone | Minoxidil | Fomepizole |
| Triamcinolone Acetonide | Cycloserine | Dinoprostone |
| Ketotifen Fumarate | Maraviroc | Ibutilide Fumarate |
| Nefazodone•HCl | Caffeine | Chloramphenicol |
| Fluorometholone | Gentamicin Sulfate | Lenalidomide |
| Loperamide•HCl | Indomethacin | Proparacaine•HCl |
| Desogestrel | Lidocaine•HCl•H₂O | Epinastine•HCl |
| Desoximetasone | Metyrapone | Nortriptyline•HCl |
| Oxiconazole Nitrate | Maprotiline•HCl | Alosetron•HCl |
| Granisetron•HCl | Quinapril•HCl | Citalopram•HBr |
| Halcinonide | Nabumetone | Ifosfamide |

**[Table 4-6]**

| | | |
|---|---|---|
| Fenoldopam Mesylate | Misoprostol | Sotalol•HCl |
| Promethazine•HCl | Atazanavir | Acetylcholine Chloride |
| Oxaprozin | Succinylcholine Chloride•2H₂O | Bisoprolol Fumarate |
| Trimethobenzamide•HCl | Amrinone | Telbivudine |
| Propofol | Bendroflumethiazide | Gabapentin |
| Guanidine•HCl | Sulfadiazine | Desipramine•HCl |
| Quinidine•HCl•H₂O | Triptorelin Acetate | Nateglinide |
| Pimecrolimus | Dapsone | Olopatadine |
| Calcitriol | Alendronate•Na Trihydrate | Nadolol |
| Dihydroergotamine Mesylate | Pindolol | Cefotetan Disodium |
| Phenoxybenzamine•HCl | Drospirenone | Zidovudine (3'-Azido-3'-Deoxythymidine) |
| Aripiprazole | Fexofenadine•HCl | Cefuroxime•Na |
| Letrozole | Acetaminophen | Candesartan |
| Metoprolol Tartrate | Hexachlorophene | Captopril |
| Procainamide•HCl | Sulindac | Crotamiton |
| Dutasteride | Cefpodoxime Proxetil | Levobunolol•HCl |
| Miconazole | Pancuronium•2Br | Cetirizine HCl |

**[Table 4-7]**

| | | |
|---|---|---|
| Loteprednol Etabonate | Levofloxacin•HCl | Thalidomide |
| Dolasetron | Tiotropium Bromide | Labetalol•HCl |
| Ropinirole•HCl | Pramipexole Dihydrochloride Monohydrate | Sodium Phenylbutyrate |
| Colistimethate•Na (Colistin sodium methanesulfonate) | Busulfan | Cyclophosphamide (Free Base) |
| Betaxolol•HCl | Cefadroxil H2O | Eszopiclone |
| Buspirone•HCl | Irbesartan | Scopolamine•HBr |
| Hydrocortisone Acetate | Dexchlorpheniramine Maleate | Mesna |
| Topiramate | Methoxsalen (Xanthotoxin) | Capreomycin sulfate |
| Asenapine Maleate | Pentoxifylline | Cortisone Acetate |
| Alitretinoin | Ezetimibe | Kanamycin Sulfate |
| Meclizine Dihydrochloride | Rizatriptan Benzoate | Zaleplon |
| Terbinafine•HCl | Cysteamine•HCl | Altretamine |
| Pemirolast Potassium | Norfloxacin | Hydroxyurea |
| Oxybutynin Chloride (Oxybutynin hydrochloride) | Dopamine•HCl | Temozolomide |
| Bexarotene | Lacosamide | Memantine•HCl |

**[Table 4-8]**

| | | |
|---|---|---|
| Acebutolol•HCl | Prazosin•HCl | Moexipril•HCl |
| Primaquine Phosphate | Ketorolac Tromethamine | Tetrahydrozoline•HCl |
| Dicyclomine•HCl | Cloxacillin•Na | Zalcitabine (2',3'-Dideoxycytidine) |
| Diclofenac•Na | Hydralazine•HCl | Diltiazem•HCl |
| Tigecycline | Metronidazole | Testosterone Enanthate |
| Methyldopa Sesquihydrate (α-Methyl-L-Dopa Sesquihydrate) | Ibuprofen | Malathion |
| Felodipine | Anagrelide | Ethacrynic acid |
| Cyproheptadine•HCl Sesquihydrate | Emtricitabine | Penicillin G Potassium (Benzylpenicillin) |
| Meropenem | Imipenem | Tizanidine•HCl |
| Hydrocortisone | Naftifine•HCl | Ceftriaxone•Na |
| Bisacodyl | Medroxyprogesterone Acetate | Adenosine |
| Levocetirizine Dihydrochloride | Capecitabine | Demeclocycline•HCl |
| Clopidogrel Hydrogen Sulfate | Dexmedetomidine•HCl | Iloperidone |
| Ropivacaine•HCl hydrate | Rifaximin | Daptomycin |

**[Table 4-9]**

| | | |
|---|---|---|
| Oxytetracycline•HCl | Carbamazepine | Disopyramide |
| Ranolazine•2HCl | Olmesartan | Streptomycin Sulfate |
| Dofetilide | Pyridostigmine Bromide | Fludarabine Phosphate |
| Tolazamide | Glipizide | Liothyronine•Na |
| Nicardipine•HCl | Foscarnet•Na (Sodium Phosphonoformate Tribasic Hexahydrate) | Hydroflumethiazide |
| Zileuton | Ramipril | Tirofiban HCl monohydrate |
| Amlodipine | Propranolol•HCl | (±)-Atenolol |
| Melphalan | Sitagliptin Phosphate | Doxepin•HCl |
| Fluticasone Propionate | Ribavirin | Glycopyrrolate Iodide (Glycopyrronium Iodide) |
| Metolazone | Regadenoson | Cilostazol |
| Streptozocin | Colistin Sulfate | Fosphenytoin disodium salt |
| Progesterone | Azelaic Acid | Montelukast•Na |
| Dyphylline (Diprophylline) | Alfuzosin | Carbinoxamine Maleate |
| Diflunisal | Carmustine | Micafungin |

**[Table 4-10]**

| | | |
|---|---|---|
| Methscopolamine Bromide ((-)-Scopolamine Methyl Bromide) | Doripenem | Ticlopidine•HCl |
| Acetylcysteine | Brimonidine | Meloxicam |
| Naphazoline•HCl | Nilutamide | Hydrochlorothiazide |
| Amikacin Sulfate | Bromfenac | Atropine Sulfate hydrate |
| Entacapone | Miglitol | (±) Isoproterenol•HCl |
| Cabergoline | Rifabutin | Desloratadine |
| Carbidopa | Norepinephrine Bitartrate Monohydrate | Risedronic Acid |
| Tetrabenazine | Acarbose | Isradipine |
| Nitrofurantoin | Pentostatin | Flumazenil |
| Sertaconazole | Sulfamethoxazole | Trimethadione |
| Carboplatin | Bosentan | Trazodone•HCl |
| Sulfanilamide | Haloperidol | Doxazosin Mesylate |
| Methazolamide | Ramelteon | Vardenafil |
| Azacitidine | Cefditoren Pivoxil | Oxacillin sodium monohydrate |
| Loxapine Succinate | Ofloxacin | Lansoprazole |

**[Table 4-11]**

| | | |
|---|---|---|
| Methylergonovine Maleate | Trospium Chloride | Cyclobenzaprine•HCl |
| Protriptyline•HCl | Lactulose | Chlorzoxazone |
| Pentamidine Isethionate | Mexiletine•HCl | Chenodiol (Chenodeoxycholic Acid) |
| Econazole Nitrate | Penicillamine (D-Penicillamine) | Rufinamide |
| Dipyridamole | Miglustat (N-Butyldeoxynojirimycin•HCl) | Paromomycin Sulfate |
| Norethindrone (Norethisterone) | Methyl Aminolevulinate•HCl | Ampicillin Trihydrate |
| Repaglinide | Raltegravir Potassium | Vecuronium Bromide |
| Epinephrine (L-(-)-Epinephrine-(+)-Bitartrate) | Aminolevulinic Acid•HCl | |
| Phentolamine•HCl | Cisplatin (Cis-Diammineplatinum(II)Dichloride) | |
| Palonosetron•HCl | Paroxetine•HCl | |
| Lomustine | Doxapram•HCl H₂O | |

Next, an agent exhibiting caspase 3/7 activity of 75% or less in the above assay was used for the next experiment to perform further analysis.

CellTiter-Glo® Luminescent Cell Viability Assay (Promega catalog number: G7570) was used to calculate cell viability of an agent with caspase 3/7 activity of 75% or less, with n = 2 as an indicator of toxicity. The cell viability was measured in the absence of TGFβ.

Caspase 3/7 activity of an agent with caspase 3/7 activity of 75% or less was further evaluated, with n = 5.

Figure **1-1** and Figure **1-2****,** and Figure **2-1** and Figure **2-****2** show the agent name of each agent that exhibited activity, results of caspase 3/7 activity (n = 5) under TGF-β2 stimulation, results of cell viability, and values obtained by dividing a value of caspase 3/7 activity by a value of the cell viability ((caspase 3/7 activity)/(cell viability) (%)). It is shown that the lower (caspase 3/7 activity)/(cell viability) (%) an agent has, the more useful the agent is. The agents shown in Figure **1-1** and Figure **1-2****,** and Figure **2-1** and Figure **2-2** exhibit caspase 3/7 activity/cell viability (%) of 80% or less, have inhibiting activity against TGF-β2, are less toxic, and are effective for a disease in a corneal endothelium or the like. Figure **1-1** and Figure **1-2** show the agents of which value of caspase 3/7 activity/cell viability (%) was ranked from the 1st to the 25th, and Figure **2-1** and Figure **2-2** show the agents of which value of caspase 3/7 activity/cell viability (%) was ranked from the 26th to the 47th. The agents with cell viability of below 90% were excluded from the ranking. Figure **1-1** and Figure **1-2****,** and Figure **2-1** and Figure **2-2** are summarized in Table 2 herein. Further, the agents ranked 48th or less and exhibiting caspase 3/7 activity of less than 100% are summarized in Table 1 herein. Each agent shown in these Table 1 and Table 2 can be useful as a preferred caspase reagent, and can be useful as a preferred composition for treating or preventing a corneal endothelial condition, disorder, or disease (e.g., Fuchs' endothelial corneal dystrophy).

### (Example 2: Confirmation of inhibiting activity against MG-132 using immortalized human corneal endothelial cells)

In this example, immortalized human corneal endothelial cells made in the above preparation example were used to confirm inhibiting activity of an agent against MG-132. In this example, MG-132, which is known as a substance inducing endoplasmic reticulum (ER) associated stress, was used as a substance inducing cell damage.

### (Materials and Methods)

7 × 10³ immortalized human corneal endothelial cells (iHCEC) were seeded on a 96-well plate and were cultured for 24 hours under the condition of 5% CO₂ at 37°C. Dulbecco's Modified Eagle Medium (DMEM) (nacalai tesque, 26252-94) + 10% FBS (Biological Industries/04-001-1A) + 1% penicillin-streptomycin (nacalai tesque, 26252-94) was used as the medium. Next, MG-132 (SIGMA, M7449) (0.1 µM) alone, or both MG-132 (0.1 µM) and each agent of the top 40 types that exhibited the value of (caspase 3/7 activity)/(cell viability) (%) of 80% or less in Example 1 (10 µM) were added. After 18 hours under the condition of 5% CO₂ at 37°C, cell morphology was observed using a phase difference microscope. Caspase 3/7 activity was then measured using Caspase-Glo® 3/7 Assay (Promega, #G8091). In the same manner as found in a patient with Fuchs' endothelial corneal dystrophy, MG-132 has action of increasing accumulation of unfolded proteins in cells and induces cell death via endoplasmic reticulum stress. DMEM + 2% FBS + 1% P/S was used as the medium. Furthermore, the control group was supplemented with a solvent of each reagent, i.e., DMSO (Dimethyl Sulfoxide, Sterile-filtered) (nacalai tesque, 13408-64). Further, Z-VD-FMK (isomer mixture) (Wako Pure Chemical Industries, Ltd./262-02061) (10 µM), which is a caspase inhibitor, was used as the positive control. Caspase 3/7 activity and cell viability were measured by the same procedure as Example 2 (n = 2 for each of them).

### (Results)

Figure **3** shows the name of each agent, results of screening of caspase 3/7 activity under MG-132 (0.1 µM) stimulation, results of cell viability, and values obtained by dividing a value of caspase 3/7 activity by a value of the cell viability ((caspase 3/7 activity)/(cell viability) (%)). The agents shown in Figure **3** exhibit caspase 3/7 activity/cell viability (%) of 80% or less, have inhibiting activity against MG-132, are less toxic, and are effective for a disease in a corneal endothelium due to endoplasmic reticulum (ER) associated stress or the like. These compounds are also summarized in Table 3. These compounds can be useful as a further preferred caspase reagent, and can be useful as a further preferred composition for treating or preventing a corneal endothelial condition, disorder, or disease (e.g., Fuchs' endothelial corneal dystrophy).

The compounds identified in this example can be effective and advantageous for a disease in a corneal endothelium due to endoplasmic reticulum (ER) associated stress as well as a disease in a corneal endothelium due to a TGF-β signal or the like. In particular, a corneal endothelial disorder such as Fuchs' endothelial corneal dystrophy is known to be caused by a TGF-β signal and endoplasmic reticulum (ER) associated stress. A compound which is capable of suppressing both a TGF-β signal and endoplasmic reticulum (ER) associated stress is especially effective for a corneal endothelial disorder such as Fuchs' endothelial corneal dystrophy. Furthermore, the compounds were confirmed to be less toxic to iFECD and iHCEC in Examples 1 and 2, and the compounds identified in this example exhibit low toxicity to both iFECD and iHCEC. Thus, it is expected that said compounds can be used with very high safety.

### (Example 3: Concentration-dependent TGF-β inhibiting activity)

The agent concentration of 10 µM was used for the test in Example 1. In this example, TGF-β inhibiting activity at various concentrations was confirmed for some of the agents shown in Table 4.

### (Materials and Methods)

7 × 10³ immortalized Fuchs' endothelial corneal dystrophy cells were seeded on a 96-well plate and were cultured for 24 hours under the condition of 5% CO₂ at 37°C. Dulbecco's Modified Eagle Medium (DMEM) (nacalai tesque, 08456-36) + 10% FBS (Sigma, 13H467) + 1% penicillin-streptomycin (nacalai tesque, 26252-94) <DMEM + 10% FBS + 1% P/S> was used as the medium.

Next, TGF-β2 (manufacturer: WAKO, distributor: Wako Pure Chemical Industries, Ltd./manufacturer code 200-19911) (10 ng/mL) alone, or both TGF-β2 (10 ng/mL) and each agent (shown in Figure **4**) (100 µM, 10 µM, 1 µM, 100 nM, 10 nM, 1 nM, 100 pM, 10 pM, 1 pM) were added. After 28 hours under the condition of 5% CO₂ at 37°C, cell morphology was observed using a phase difference microscope. Caspase 3/7 activity was then measured using Caspase-Glo 3/7 Assay (Promega, #G8091) . DMEM + 2% FBS + 1% P/S was used as the medium. Caspase 3/7 activity and cell viability in this example were measured using GloMax-Multi Detection System (Promega, E7051).

### (Results)

The results are shown in Figure **4A-1****,** Figure **4A-2** and Figure **4A-3****,** and Figure **4B-1****,** Figure **4B-2** and Figure **4B-3****.** Caspase 3/7 activity below 80% was used as a indicator indicating whether an agent can be used for treating or preventing a corneal endothelial condition, disorder, or disease, or whether an agent can be used as a caspase inhibitor (the underlines in Figure **4A-1****,** Figure **4A-2** and Figure **4A-3****,** and Figure **4B-1****,** Figure **4B-2** and Figure **4B-3**). When an agent has caspase 3/7 activity of below 80% at any of the agent concentrations, the agent can be used as a caspase inhibitor and can be useful as a composition for treating or preventing a corneal endothelial condition, disorder, or disease (e.g., Fuchs' endothelial corneal dystrophy)). Those skilled in the art can test whether an agent other than those shown in Figure **4A-1****,** Figure **4A-2** and Figure **4A-3****,** and Figure **4B-1****,** Figure **4B-2** and Figure **4B-3** is useful as a caspase inhibitor or as a composition for treating or preventing a corneal endothelial condition, disorder, or disease at various concentrations by appropriately selecting an agent listed in Table 4 and conducting the same test.

### (Example 4: Confirmation of inhibiting activity of fluticasone furoate against TGF-β)

In this example, TGF-β inhibiting activity of fluticasone furoate, which is one of SEGRAs, was tested.

### (Materials and Methods)

### (Observation using a phase contrast microscope)

The medium was removed from the culture dish in which Fuchs' endothelial corneal dystrophy patient derived immortalized human corneal endothelial cells were being cultured, and the cells were supplemented with 1 × PBS (-) that was preheated to 37°C, and were washed. This was repeated twice. The cells were supplemented with 1 × PBS (-) again and incubated for 5 minutes at 37°C (5% CO₂). After removing the PBS (-), the cells were supplemented with 0.05% Trypsin-EDTA (nacalai tesque, 32778-34) and incubated for 5 minutes at 37°C (5% CO₂). The cells were then suspended in a medium, and collected by centrifugation at 1500 rpm for 3 minutes. DMEM (nacalai tesque, 08456-36) + 10% FBS (Sigma, 13H467) + 1% P/S (nacalai tesque, 26252-94) was used as the medium.

Fuchs' endothelial corneal dystrophy patient derived immortalized human corneal endothelial cells (lot: iFECD3-5) were seeded on a 12-well plate at a ratio of 5.0 × 10⁴ cells per well, and cultured for 24 hours at 37°C (5% CO₂). DMEM + 10% FBS + 1% P/S was used as the medium.

After 24 hours, the medium was removed, fluticasone furoate (AXON MEDCHEM, 1172) was added so that the final concentration was each 0.0001, 0.0003, 0.001, 0.003, 0.01, 0.03, 0.1, 0.3, 1, and 3 µM, and the cells were cultured for 24 hours. DMEM + 2% FBS + 1% P/S was used as the medium.

After 24 hours, the medium was removed, fluticasone furoate was added together with 10 ng/ml Transforming Growth Factor-β2 Human recombinant (WAKO, 200-19911) so that the final concentration was each 0.0001, 0.0003, 0.001, 0.003, 0.01, 0.03, 0.1, 0.3, 1, and 3 µM, and the cells were cultured for 24 hours. Medium: DMEM + 2% FBS + 1% P/S

After 24 hours, cell morphology and apoptosis were observed using a phase difference microscope.

### (Measurement of caspase activity and cell viability)

The medium was removed from the culture dish in which Fuchs' endothelial corneal dystrophy patient derived immortalized human corneal endothelial cells were being cultured, and the cells were supplemented with 1 × PBS (-) that was preheated to 37°C, and were washed. This was repeated twice. The cells were supplemented with 1 × PBS (-) again and incubated for 5 minutes at 37°C (5% CO₂). After removing the PBS (-), the cells were supplemented with 0.05% Trypsin-EDTA (nacalai tesque, 32778-34) and incubated for 5 minutes at 37°C (5% CO₂). The cells were then suspended in a medium, and collected by centrifugation at 1500 rpm for 3 minutes. DMEM (nacalai tesque, 08456-36) + 10% FBS (Sigma, 13H467) + 1% P/S (nacalai tesque, 26252-94) was used as the medium.

Fuchs' endothelial corneal dystrophy patient derived immortalized human corneal endothelial cells (lot: iFECD3-5) were seeded on a 96-well plate at a ratio of 7 × 10³ cells per well, and cultured at 37°C (5% CO₂) until reaching confluence. DMEM + 10% FBS + 1% P/S was used as the medium.

After 24 hours, the medium was removed, fluticasone furoate (AXON MEDCHEM, 1172) was added so that the final concentration was each 0.0001, 0.0003, 0.001, 0.003, 0.01, 0.03, 0.1, 0.3, 1, and 3 µM, and the cells were cultured for 24 hours. DMEM + 2% FBS + 1% P/S was used as the medium.

After 24 hours, the medium was removed, 10 ng/ml Transforming Growth Factor-β2 Human recombinant (WAKO, 200-19911) and fluticasone furoate were added so that the final concentration was each 0.0001, 0.0003, 0.001, 0.003, 0.01, 0.03, 0.1, 0.3, 1, and 3 µM, and the cells were cultured for 24 hours. DMEM + 2% FBS + 1% P/S was used as the medium.

After 24 hours, cell morphology was observed using a phase difference microscope.

After observation, measurement of caspase 3/7 activity by Caspase-Glo 3/7 Assay was carried out with the following procedure.

The medium was discarded to achieve 50 µl per well. 50 µl/well of CaspaseGlo 3/7 Assay Reagent (mixture of Caspase-Glo 3/7 Assay Buffer and Caspase-Glo 3/7 Assay Substrate) (Promega, G8091) solution was added so as to achieve 1:1 with the medium. The operations hereafter were carried out while being shaded. A shaker was used to mix the content well for 2 minutes at about 120 r/min to then be left still for 40 minutes at room temperature. After being left still, 80 µl was transferred to Assay plate (Corning, 3912, Assay plate 96well, white polystyrene) and absorbance was measured using GloMax-Multi Detection System (Promega, E7051). Furthermore, 10 µM Z-VD-FMK (WAKO, 262-02061), which is a caspase inhibitor, was used as the positive control.

Cell viability was analyzed using Cell Titer-Glo Luminescent Cell Viability Assay by the following procedure.

The medium was discarded to achieve 50 µl per well. 50 µl/well of Cell Titer-Glo Luminescent Cell Viability Assay solution (Promega, G7572) was added so as to achieve 1:1 with the medium. The operations hereafter were carried out while being shaded. A shaker was used to mix the content well for 2 minutes at about 120 r/min to then be left still for 10 minutes. After being left still, 80 µl was transferred to Assay plate (Corning, 3912, Assay plate 96well, white polystyrene) and absorbance was measured using GloMax-Multi Detection System (Promega, E7051).

### (Results)

### (Observation using a phase contrast microscope)

When Fuchs' endothelial corneal dystrophy patient derived immortalized human corneal endothelial cells were stimulated with TGF-β, cells were notably damaged. When pretreated with fluticasone furoate, it was observed that a disorder in corneal endothelial cells was effectively suppressed particularly at 0.001, 0.003, 0.01, 0.03, 0.1, 0.3, 1, and 3 µM. Further, the effect of suppressing a disorder in corneal endothelial cells was confirmed at 0.0001 and 0.0003 µM as well.

### (Caspase Activity)

Caspase-Glo 3/7 Assay can measure the activity of Caspase 3/7 that is involved in apoptosis induction. That is, the higher the activity of Caspase 3/7 is, the more cell damage is induced. When stimulated with TGF-β, it was observed that Caspase 3/7 was significantly activated as compared to a case without stimulation. Meanwhile, when fluticasone furoate was added, the activity of Caspase 3/7 was decreased in the order of 0.0001, 0.0003, and 0.001. In particular, the values at 0.003, 0.01, 0.03, 0.1, 0.3, 1, and 3 µM were almost the same as those in the Control group that had not been stimulated with TGF-β. Thus, it was demonstrated that fluticasone furoate inhibits the activity of Caspase 3/7 in a concentration-dependent manner, and most effectively exhibits the effect at 0.003, 0.01, 0.03, 0.1, 0.3, 1, and 3 µM.

### (Cell viability)

Cell viability was measured by Cell Titer-Glo Luminescent Cell Viability Assay. As a result, no significant difference from the Control group was observed in the cell viability at all concentrations of 0.0001, 0.0003, 0.001, 0.003, 0.01, 0.03, 0.1, 0.3, 1, and 3 µM of fluticasone furoate when added. This suggested that addition of fluticasone furoate does not cause a disorder to cells at the concentration of 0.0001, 0.0003, 0.001, 0.003, 0.01, 0.03, 0.1, 0.3, 1, and 3 µM.

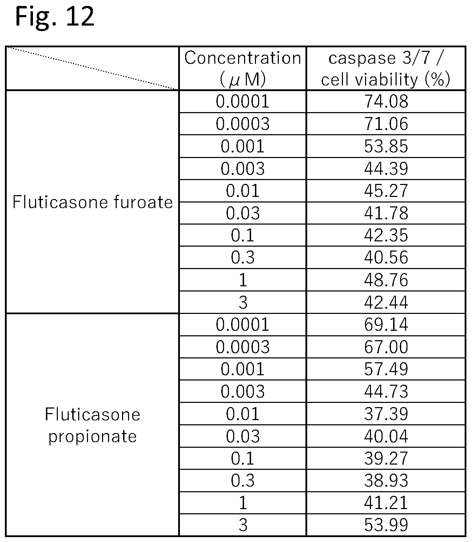

Figure **12** shows the ratio of (caspase 3/7 activity)/(cell viability) when fluticasone furoate is used. Although the agent concentration was lower (0.0001 to 3 µM) than 10 µM, which was employed in Example 1, the ratio which is well below 0.8 was obtained. This indicated that fluticasone furoate is able to very strongly inhibit TGF-β. Further, it was demonstrated that fluticasone furoate is useful as a caspase inhibitor as well.

### (Example 5: Confirmation of inhibiting activity of fluticasone propionate against TGF-β)

In this example, TGF-β inhibiting activity of fluticasone propionate (AXON MEDCHEM, 1404), which is one of SEGRAs, was tested. The test was conducted by the same procedure as Example 4.

### (Results)

When Fuchs' endothelial corneal dystrophy patient derived immortalized human corneal endothelial cells were stimulated with TGF-β, cells were notably damaged. When pretreated with fluticasone propionate, it was observed that a disorder in corneal endothelial cells was effectively suppressed particularly at 0.001, 0.003, 0.01, 0.03, and 0.1 µM. Further, the effect of suppressing a disorder in corneal endothelial cells was confirmed at 0.0001, 0.0003, 1, and 3 µM as well.

Figure **12** shows the ratio of (caspase 3/7 activity)/(cell viability) when fluticasone propionate is used. Although the agent concentration was lower (0.0001 to 3 µM) than 10 µM, which was employed in Example 1, the ratio which is well below 0.8 was obtained. This indicated that fluticasone furoate is able to very strongly inhibit TGF-β. Further, it was demonstrated that fluticasone furoate is useful as a caspase inhibitor as well.

### (Results)

When stimulated with TGF-β, it was observed that Caspase 3/7 was significantly activated as compared to a case without stimulation. Meanwhile, when fluticasone propionate was added, the activity of Caspase 3/7 was decreased in the order of 0.0001, 0.0003, 0.001, and 0.003. In particular, the values at 0.01, 0.03, 0.1, 0.3, and 1 µM were almost the same as those in the Control group that had not been stimulated with TGF-β. Thus, it was demonstrated that fluticasone propionate inhibits the activity of Caspase 3/7 in a concentration-dependent manner, and most effectively exhibits the effect at 0.01, 0.03, 0.1, 0.3, and 1 µM.

### (Results)

Cell viability was measured by Cell Titer-Glo Luminescent Cell Viability Assay. As a result, no significant difference from the Control group was observed in the cell viability at all concentrations of 0.0001, 0.0003, 0.001, 0.003, 0.01, 0.03, 0.1, 0.3, 1, and 3 µM when fluticasone propionate was added. This suggested that addition of fluticasone propionate does not cause a disorder to cells at the concentration of 0.0001, 0.0003, 0.001, 0.003, 0.01, 0.03, 0.1, 0.3, 1, and 3 µM.

### (Example 6: Confirmation of inhibiting activity of ZK216348 against TGF-β)

In this example, TGF-β inhibiting activity of ZK216348 (AXON MEDCHEM, AXON2239), which is one of SEGRMs, was tested. The operation by a phase contrast microscope was carried out in the same manner as Example 4. 10 µM of ZK216348 was used as the final concentration.

After 24 hours, the medium was removed, ZK216348 was added together with 10 ng/ml Transforming Growth Factor-β2 Human recombinant (WAKO, 200-19911) so that the final concentration was 10 M, and the cells were cultured for 24 hours. DMEM + 2% FBS + 1% P/S was used as the medium.

After 24 hours, cell morphology and apoptosis were observed using a phase difference microscope.

### (Results)

When Fuchs' endothelial corneal dystrophy patient derived immortalized human corneal endothelial cells were stimulated with TGF-β, cells were notably damaged. When pretreated with ZK216348, it was observed that free cells were decreased and a disorder in corneal endothelial cells was suppressed. ZK216348 is an agent classified into nonsteroidal SEGRA which does not have a steroidal skeleton. This result shows that general SEGRAs have action of suppressing a corneal endothelial disorder regardless of the presence or absence of a steroid skeleton.

### (Example 7: Diagnosis and therapy examples)

The present invention is used when diagnosed with Fuchs' endothelial corneal dystrophy or a similar corneal endothelial disease (specific examples thereof include 1) observation of guttae formation, hypertrophy of the Descemet's membrane, corneal epithelial edema, or edema of the corneal stroma by slit-lamp microscopy, 2) observation of images of guttae or corneal endothelial disorder with a specular microscope, 3) observation of corneal edema with a Pentacam, OCT, ultrasonic corneal thickness measuring apparatus, or the like, and 4) when determined as high risk by genetic diagnosis). The composition of the present invention can be used as eye drops, injection into the anterior chamber, administration using controlled-release agent, intravitreal injection, or subconjunctival injection for therapy.

A commercially available substance that is compatible with the Japanese Pharmacopoeia, an equivalent product thereof or the like can be used as each component other than the active ingredient.

### (Example 8: Formulation Example: Cornea preservation solution)

As a formulation example, this example manufactures a cornea preservation solution containing a caspase inhibitor as follows.

The following preservation solution is prepared by a conventional method.

| | |
|---|---|
| Any compound shown in Table 4 | effective amount |
| Optisol-GS (Bausch-Lomb) | optimal dose |
| | Total amount 100 mL |

A compound provided by various companies (e.g., amlexanox is available from Takeda Pharmaceutical Company Limited. or the like) can be used for any compound shown in Table 4.

### (Example 9: In vivo evaluation in a mouse model)

In vivo evaluation can be carried out using Alpha2 Collagen VIII (Col8a2) Q455K knock-in mouse (Hum Mol Genet. 2012 Jan 15; 21(2): 384-93.), which is a Fuchs' endothelial corneal dystrophy model mouse. Deposition of an extracellular matrix to an endothelial corneal basement membrane (Descemet's membrane) called guttae and cell density reduction due to corneal endothelial damage were found in the model mouse in the same manner as those found in Fuchs' endothelial corneal dystrophy in a human. It is possible to treat or prevent outbreak of Fuchs' endothelial corneal dystrophy or delay the progression of the pathology by carrying out instillation administration, anterior chamber administration, intravitreal administration, subconjunctival administration, or systemic administration of the compound of the present invention to such a mouse.

### (Example 10: Preparation example for eye drops)

As a formulation example, this example manufactures an eye drop containing a caspase inhibitor as follows.

The composition of test substance at each concentration is shown below.

| | |
|---|---|
| Any compound shown in Table 4 amount | effective |
| Sodium chloride | 0.85 g |
| Sodium dihydrogen phosphate dehydrate | 0.1 g |
| (Optionally) Benzalkonium chloride | 0.005 g |
| Sodium hydroxide | optimal dose |
| Purified water | optimal dose |
| Total amount | 100 mL (pH 7.0) |

The concentration may be diluted using a base consisting of the following components.

| | |
|---|---|
| Sodium chloride | 0.85 g |
| Sodium dihydrogen phosphate dehydrate | 0.1 g |
| (Optionally) Benzalkonium chloride | 0.005 g |
| Sodium hydroxide | optimal dose |
| Purified water | optimal dose |
| Total amount | 100 mL (pH 7.0) |

As disclosed above, the present invention is exemplified by the use of its preferred embodiments of the present invention. However, it is understood that the scope of the present invention should be interpreted solely based on the Claims. It is also understood that any patent, any patent application, and any references cited herein should be incorporated herein by reference in the same manner as the contents are specifically described herein. The present invention claims priority to Japanese Patent Application 2017-118617 (filed on June 16, 2017), Japanese Patent Application 2018-10825 (filed on January 25, 2018) and Japanese Patent Application 2018-53235 (filed on March 20, 2018). The entire content thereof is incorporated herein by reference.

### [Industrial Applicability]

The present invention provides a medicament for use in treating or preventing a corneal endothelial condition, disorder, or disease due to a transforming growth factor-β (TGF-β) signal, in particular, provides a medicament for use in treating or preventing a corneal endothelial disorder in Fuchs' endothelial corneal dystrophy. The present invention provides a technique available to industries (pharmaceutical or the like) involved in techniques associated with formulation or the like based on such a technique.

## Claims

1. A composition for treating or preventing a corneal endothelial condition, disorder, or disease, the composition comprising a compound which, when contacted with immortalized Fuchs' endothelial corneal dystrophy cells, exhibits: (i) cell viability (%) of the cells of about 90% or more after being cultured in Dulbecco's Modified Eagle Medium (DMEM) + 2% fetal bovine serum (FBS) + 1% penicillin/streptomycin (P/S) for 24 to 28 hours; and (ii) ratio of caspase 3/7 activity (%) in the presence of TGF-β with respect to the cell viability (%) of 0.8 or less after being cultured in Dulbecco's Modified Eagle Medium (DMEM) + 2% fetal bovine serum (FBS) + 1% penicillin/streptomycin (P/S) for 24 to 28 hours.

2. The composition of claim 1, wherein the compound is selected from the group consisting of an anti-inflammatory drug, vitamin B12 group, vitamin D group, selective glucocorticoid receptor agonist (SEGRA), selective glucocorticoid receptor modulator (SEGRM) and a combination thereof.

3. The composition of claim 1, comprising an anti-inflammatory drug.

4. The composition of claim 2 or 3, wherein the anti-inflammatory drug is a steroidal anti-inflammatory drug or a nonsteroidal anti-inflammatory drug (NSAID), or a combination thereof.

5. The composition of claim 2 or 3, wherein the anti-inflammatory drug is a steroidal anti-inflammatory drug, the steroidal anti-inflammatory drug being a compound selected from the group consisting of Mometasone Furoate, Clobetasol Propionate, Loteprednol Etabonate, Difluprednate, Dexamethasone, Amcinonide, Flurandrenolide, Prednisolone, Fluocinolone Acetonide, Desonide, Triamcinolone Acetonide, Budesonide, Fludrocortisone Acetate, Fluocinonide, Methylprednisolone, Betamethasone, Desoximetasone, Halcinonide, Fluorometholone, Beclomethasone Dipropionate, and Dutasteride, a derivative or an analogue thereof, or a pharmaceutically acceptable salt thereof, or a solvate thereof.

6. The composition of claim 2 or 3, wherein the anti-inflammatory drug is a nonsteroidal anti-inflammatory drug (NSAID), the nonsteroidal anti-inflammatory drug (NSAID) being a compound selected from the group consisting of Amlexanox, Leflunomide, Olsalazine•Na, Orphenadrine Citrate, Flurbiprofen, and Phenoxybenzamine•HCl, a derivative or an analogue thereof, or a pharmaceutically acceptable salt thereof, or a solvate thereof.

7. The composition of claim 2, wherein the compound includes at least one selected from the group consisting of a selective glucocorticoid receptor agonist (SEGRA) and a selective glucocorticoid receptor modulator (SEGRM).

8. The composition of claim 2, wherein the compound includes a selective glucocorticoid receptor agonist (SEGRA) .

9. The composition of claim 2, wherein the compound includes a selective glucocorticoid receptor modulator (SEGRM).

10. The composition of claim 1, wherein the compound is a compound represented by the following general formula (1) or general formula (2) 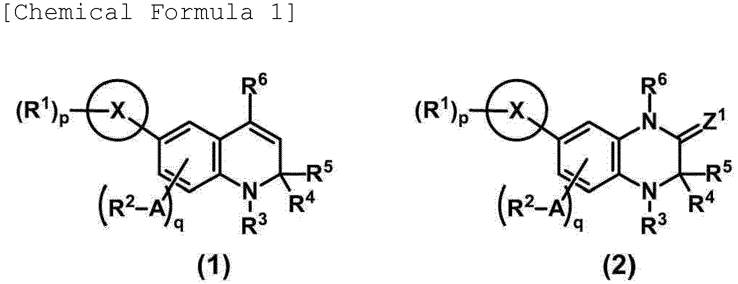 or a salt thereof, wherein:
ring X represents a benzene ring or a pyridine ring;
R¹ represents a halogen atom, a lower alkyl group which may have a substituent, a lower cycloalkyl group which may have a substituent, an aryl group which may have a substituent, an arylalkyloxy group which may have a substituent, an arylalkyloxyalkyloxy group, a heterocyclic group which may have a substituent, a hydroxy group, an ester of a hydroxy group, a lower alkoxy group which may have a substituent, a lower cycloalkyloxy group which may have a substituent, an aryloxy group which may have a substituent, a heterocyclic oxy group which may have a substituent, a mercapto group, an ester of a mercapto group, a lower alkylthio group which may have a substituent, a lower cycloalkylthio group which may have a substituent, an arylthio group which may have a substituent, a heterocyclic thio group which may have a substituent, a lower alkylamino group which may have a substituent, a lower cycloalkylamino group which may have a substituent, an arylamino group which may have a substituent, a heterocyclic amino group which may have a substituent, an amide of an amino group, an amide of a lower alkylamino group which may have a substituent, an amide of a lower cycloalkylamino group which may have a substituent, an amide of an arylamino group which may have a substituent, an amide of a heterocyclic amino group which may have a substituent, a formyl group, a lower alkylcarbonyl group which may have a substituent, a lower cycloalkylcarbonyl group which may have a substituent, an arylcarbonyl group which may have a substituent, a heterocyclic carbonyl group which may have a substituent, a carboxy group, an ester of a carboxy group, an amide of a carboxy group, a lower alkylsulfonyl group which may have a substituent, a lower cycloalkylsulfonyl group which may have a substituent, an arylsulfonyl group which may have a substituent, a heterocyclic sulfonyl group which may have a substituent, a sulfonic acid group, an ester of a sulfonic acid group, an amide of a sulfonic acid group, a lower alkenyloxy group which may have a substituent, a silyl group which may have a substituent, - W¹-C(=O)-R¹⁸, -W¹-C(=O)-O-R¹⁸, -W¹-S(=O)-R¹⁹, -W¹-S(=O)₂-R²⁰, - W¹-C(=O)NR²¹R²², -OCH₂CH(R²³)-OR²⁴, an amino group which may have a substituent, a nitro group, or a cyano group;
R¹⁸, R¹⁹, R²⁰, R²¹ and R²² each independently represent a hydrogen atom, a lower alkyl group which may have a substituent, a lower alkenyl group which may have a substituent, a lower alkynyl group which may have a substituent, a lower cycloalkyl group which may have a substituent, an aryl group which may have a substituent, a heterocyclic group which may have a substituent, an aralkyl group which may have a substituent, a heterocyclic alkyl group which may have a substituent, an amino lower alkyl group which may have a substituent, a lower alkoxy group which may have a substituent, a lower alkenyloxy group which may have a substituent, a lower alkynyloxy group which may have a substituent, a lower cycloalkyloxy group which may have a substituent, an aryloxy group which may have a substituent, or a heterocyclic oxy group which may have a substituent;
p represents an integer of 0 to 5;
when p is 2 to 5, each R¹ may be the same or different;
W¹ represents an oxygen atom, a sulfur atom, or -N(R²⁵)-;
Z¹ represents an oxygen atom, a sulfur atom, or =N-R²⁵;
R²³ represents a hydrogen atom, a lower alkyl group which may have a substituent, a carboxy group, an ester of a carboxy group, an amide of a carboxy group, or a cyano group;
R²⁴ represents a hydrogen atom, a lower alkylcarbonyl group which may have a substituent, a lower cycloalkylcarbonyl group which may have a substituent, an arylcarbonyl group which may have a substituent, a heterocyclic carbonyl group which may have a substituent, an ester of a carboxy group, an amide of a carboxy group, a phosphoric acid group, or an ester of a phosphoric acid group;
R²⁵ represents a hydrogen atom, a lower alkyl group which may have a substituent, a lower alkenyl group which may have a substituent, a lower alkynyl group which may have a substituent, an aryl group which may have a substituent, a lower alkylcarbonyl group which may have a substituent, a lower alkenylcarbonyl group which may have a substituent, an arylalkyl group which may have a substituent, an arylalkyloxyalkyl group which may have a substituent, a heterocyclic alkyl group which may have a substituent, a silyl group which may have a substituent, a lower alkynylcarbonyl group which may have a substituent, or an arylcarbonyl group which may have a substituent;
R² represents a halogen atom, a lower alkyl group which may have a substituent, -OR⁸, -NR⁸R⁹, -SR⁸, -S(=O)-R⁸ or-S(=O)₂-R⁸;
q represents an integer of 0 to 3;
when q is 2 or 3, each R² may be the same or different;
R³ represents a hydrogen atom, a lower alkyl group which may have a substituent, a lower alkenyl group which may have a substituent, a lower alkynyl group which may have a substituent, an aryl group which may have a substituent, a lower alkylcarbonyl group which may have a substituent, a lower alkenylcarbonyl group which may have a substituent, an arylalkyl group which may have a substituent, an arylalkyloxyalkyl group which may have a substituent, a silyl group which may have a substituent, a lower alkynylcarbonyl group which may have a substituent, or an arylcarbonyl group which may have a substituent;
R⁴ and R⁵ each independently represent a hydrogen atom, a halogen atom, a lower alkyl group which may have a substituent, a lower alkenyl group which may have a substituent, a lower alkynyl group which may have a substituent, a lower cycloalkyl group which may have a substituent, an aryl group which may have a substituent, or a heterocyclic group which may have a substituent, or R⁴ and R⁵ may together form a 3- to 8-membered lower cycloalkane ring which may have a substituent;
R⁶ represents a hydrogen atom, a lower alkyl group which may have a substituent, a lower alkenyl group which may have a substituent, a lower alkynyl group which may have a substituent, a lower cycloalkyl group which may have a substituent, an aryl group which may have a substituent, or a heterocyclic group which may have a substituent;
A represents a lower alkylene group which may have a substituent or a single bond;
R⁸ and R⁹ each independently represent a hydrogen atom, a lower alkyl group which may have a substituent, a lower alkenyl group which may have a substituent, a lower alkynyl group which may have a substituent, a lower cycloalkyl group which may have a substituent, an aryl group which may have a substituent, a heterocyclic group which may have a substituent, a formyl group, a lower alkylcarbonyl group which may have a substituent, a lower alkenylcarbonyl group which may have a substituent, a lower alkynylcarbonyl group which may have a substituent, a lower cycloalkylcarbonyl group which may have a substituent, an arylcarbonyl group which may have a substituent, a heterocyclic carbonyl group which may have a substituent, a carboxy group, a lower alkoxycarbonyl group which may have a substituent, a lower alkenyloxycarbonyl group which may have a substituent, a lower alkynyloxycarbonyl group which may have a substituent, a lower cycloalkyloxycarbonyl group which may have a substituent, an aryloxycarbonyl group which may have a substituent, a heterocyclic oxycarbonyl group which may have a substituent, an arylcarbonyloxy group which may have a substituent, a heterocyclic carbonyloxy group which may have a substituent, an aryloxy group which may have a substituent, a heterocyclic oxy group which may have a substituent, an arylthio group which may have a substituent, a lower alkylsulfonyl group which may have a substituent, a lower alkenylsulfonyl group which may have a substituent, a lower alkynylsulfonyl group which may have a substituent, a lower cycloalkylsulfonyl group which may have a substituent, an arylsulfonyl group which may have a substituent, a heterocyclic sulfonyl group which may have a substituent, an arylamino group which may have a substituent, an aminocarbonyl group, a lower alkylaminocarbonyl group which may have a substituent, a lower alkenylaminocarbonyl group which may have a substituent, a lower alkynylaminocarbonyl group which may have a substituent, a lower cycloalkylaminocarbonyl group which may have a substituent, an arylaminocarbonyl group which may have a substituent, or a heterocyclic aminocarbonyl group which may have a substituent; and
when R² is NR⁸R⁹, R⁸ and R⁹ may together form a 3- to 8-membered nitrogen-containing heterocycle which may have a substituent.

11. The composition of claim 1, wherein the compound is a compound represented by the following general formulas (3), (4), (5), (6), (7), (8), (9), (10-a), (10-b), (12), (13-a), (13-b), (14), (15), (16), (17), (18), (19) or (20) 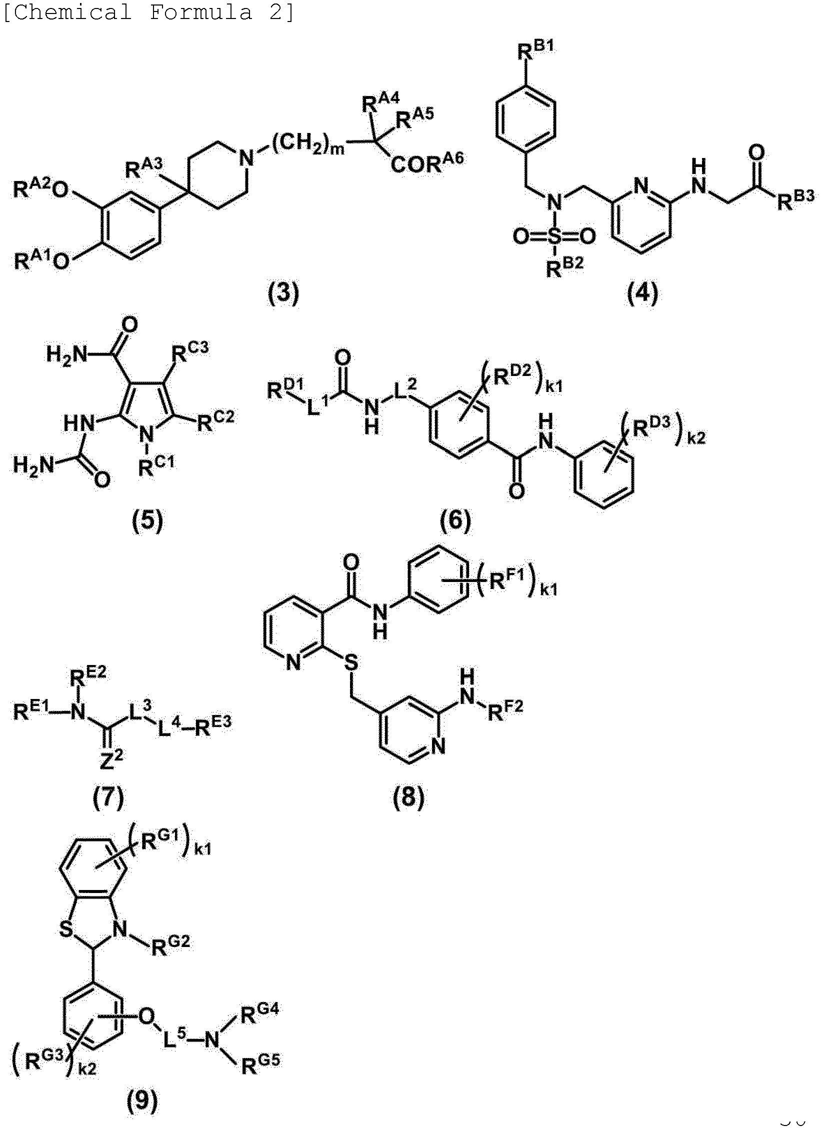 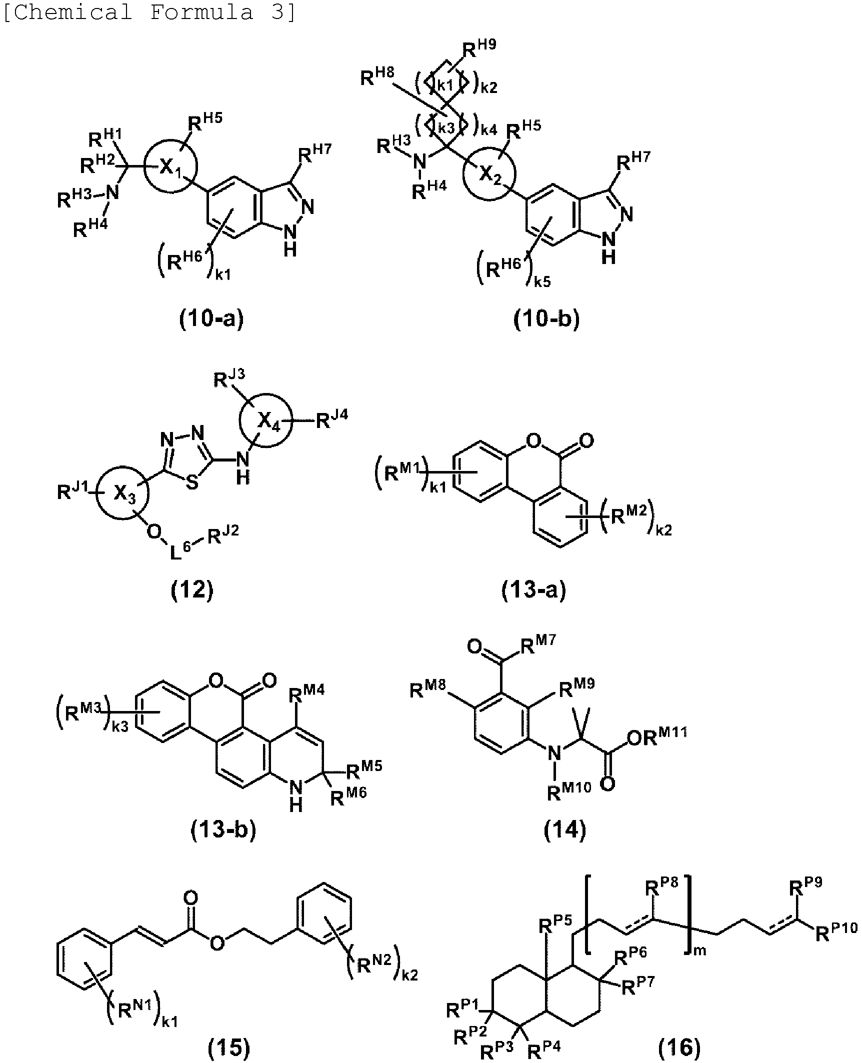 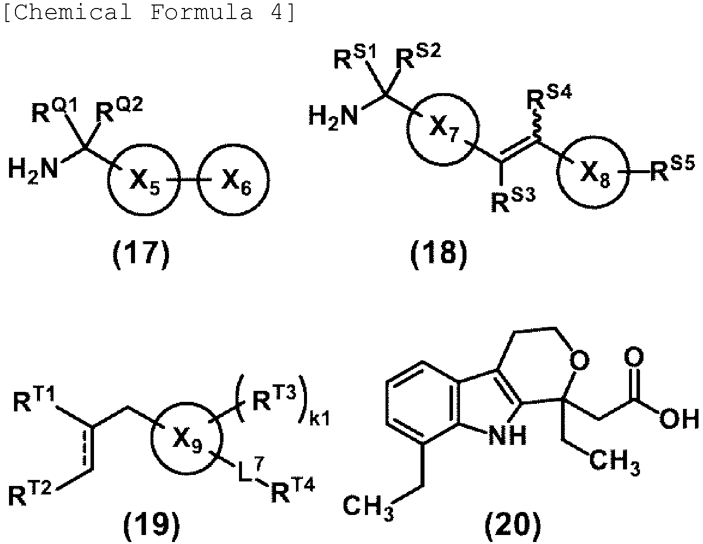 or a salt thereof, wherein
[Chemical Formula 5] **---**
represents a single bond or a double bond,
R^{A1}, R^{A2}, R^{A3}, R^{A4}, D^{A5}, R^{A6}, R^{B1}, R^{B2}, R^{B3}, R^{C1}, R^{C2}, R^{C3}, R^{D1}, R^{D2}, R^{D3}, R^{E1}, R^{E2}, R^{E3}, R^{F1}, R^{F2}, R^{G1}, R^{G2}, R^{G3}, R^{G4}, R^{G5}, R^{H1}, R^{H2}, R^{H3}, R^{H4}, R^{H5}, R^{H6}, R^{H7}, R^{H8}, R^{H9}, R^{J1}, R^{J2}, R^{J3}, R^{J4}, R^{M1}, R^{M2}, R^{M3}, R^{M4}, R^{M5}, R^{M6}, R^{M7}, R^{M8}, R^{M9}, R^{M10}, R^{M11}, R^{N1}, R^{N2}, R^{P1}, R^{P2}, R^{P3}, R^{P4}, R^{P5}, R^{P6}, R^{P7}, R^{P8}, R^{P9}, R^{P10}, R^{Q1}, R^{Q2}, R^{S1}, R^{S2}, R^{S3}, R^{S4}, R^{S5}, R^{T1}, R^{T2}, R^{T3}, and R^{T4} are each independently selected from the substituent group A,
ring X₁, X₂, X₃, X₄, X₅, X₆, X₇, X₈, and X₉ are each independently a lower cycloalkyl group which may have a substituent, a lower cycloalkenyl group which may have a substituent, an aryl group which may have a substituent, or a heterocyclic group which may have a substituent,
L¹, L², L³, L⁴, L⁵, L⁶ and L⁷ are each independently a single bond, a lower alkylene group which may have a substituent, an arylene group which may have a substituent, a heteroarylene group which may have a substituent, a heterocyclylene group which may have a substituent, -C(=O)-, -C(=O)-O-, -S(=O)-, -S(=O)₂- or -C(=O)-NH-,
each m, each k1, each k2, each k3, each k4, and each k5 are independently 0, 1, 2, 3, 4, or 5, and
a wavy line bond is a single bond representing a stereoisomerism of (E) or (Z).

12. The composition of claim 1, wherein the compound is a compound selected from the group consisting of (R)-trans-N-(pyridine-4-yl)-4-(1-aminoethyl)cyclohexane carboxamide: 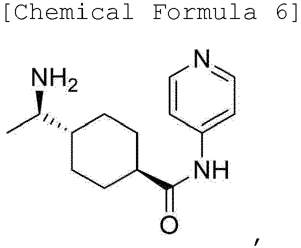 (R)-(+)-N-(1H-pyrrolo[2,3-b]pyridine-4-yl)-4-(1-aminoethyl)benzamide: 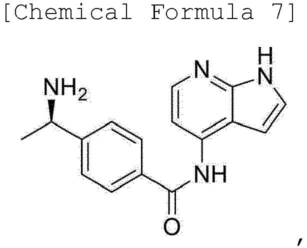 1-(5-isoquinolinesulfonyl)homopiperazine: 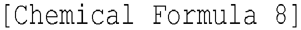 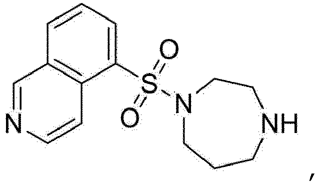 and
1-(5-isoquinolinesulfonyl)-2-methylpiperazine: 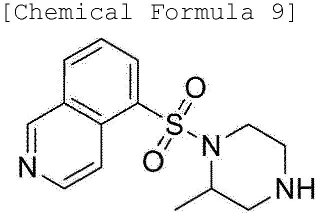 or a salt thereof.

13. The composition of claim 1, wherein the compound is selected from the group consisting of fluticasone, flumethasone, and RU-24858.

14. The composition of claim 1, wherein the compound is fluticasone propionate or fluticasone furoate.

15. The composition of claim 1, wherein the compound is selected from the group consisting of Mapracorat, ZK216348, ZK209614, Dagrocorat, Fosdagrocorat, Compound A, AL-438, LGD-5552, C108297, MK-5932, Org 214007-0, PF-802, DE-110, and Compound 10.

16. The composition of claim 2, wherein the compound is a vitamin B12 group, the vitamin B12 group being Hydroxocobalamin•HCl, a derivative or an analogue thereof, or a pharmaceutically acceptable salt thereof, or a solvate thereof.

17. The composition of claim 2, wherein the compound is a vitamin D group, the vitamin D group being Calcipotriene, a derivative or an analogue thereof, or a pharmaceutically acceptable salt thereof, or a solvate thereof.

18. The composition of claim 1, wherein the compound is selected from at least one compound shown in Table A:
**[Table A-1]**
| |
|---|
| Amlexanox |
| Leflunomide |
| Olsalazine•Na |
| Hydroxocobalamin•HCl |
| Mometasone Furoate |
| Febuxostat |
| Orphenadrine Citrate |
| Clobetasol Propionate |
| Loteprednol Etabonate |
| Difluprednate |
| Dexamethasone |
| Dobutamine•HCl |
| Amcinonide |
| Flurandrenolide |
| Fluorouracil (5-Fluorouracil) |
| Prednisolone |
| Fluocinolone Acetonide |
| Desonide |
| Triamcinolone Acetonide |
| Ketoconazole |
| Flurbiprofen |
| Budesonide |
**[Table A-2]**
| |
|---|
| Fludrocortisone Acetate |
| Fluocinonide |
| Methylprednisolone |
| Betamethasone |
| Desoximetasone |
| Toremifene Base |
| Terazosin•HCl |
| Halcinonide |
| Bromocriptine Mesylate |
| Fluorometholone |
| Oxaliplatin |
| Calcipotriene |
| Beclomethasone Dipropionate |
| Reserpine |
| Phenoxybenzamine•HCl |
| Droperidol |
| Promethazine•HCl |
| Esmolol |
| Dutasteride |
| Dihydroergotamine Mesylate |
| Imipramine•HCl |
| Betaxolol•HCl |
| Trimethobenzamide•HCl |
, a derivative or an analogue thereof, or a pharmaceutically acceptable salt thereof, or a solvate thereof.

19. A composition for treating or preventing a corneal endothelial condition, disorder, or disease, the composition comprising at least one compound selected from the group consisting of a selective glucocorticoid receptor agonist (SEGRA) and a selective glucocorticoid receptor modulator (SEGRM).

20. The composition of any one of claims 1 to 19, wherein the condition, disorder, or disease is a corneal endothelial condition, disorder, or disease due to transforming growth factor-β (TGF-β).

21. The composition of any one of claims 1 to 20, wherein the condition, disorder, or disease is selected from the group consisting of Fuchs' endothelial corneal dystrophy, post-corneal transplant disorder, corneal endotheliitis, trauma, post-ophthalmic surgery disorder, post-ophthalmic laser surgery disorder, aging, posterior polymorphous dystrophy (PPD), congenital hereditary endothelial dystrophy (CHED), idiopathic corneal endothelial disorder, and cytomegalovirus corneal endotheliitis.

22. The composition of any one of claims 1 to 21, wherein the condition, disorder, or disease includes Fuchs' endothelial corneal dystrophy.

23. The composition of any one of claims 1 to 22, wherein the compound, when further contacted with immortalized human corneal endothelial cells, exhibits:
(i) cell viability (%) of the cells of about 90% or more after being cultured in Dulbecco's Modified Eagle Medium (DMEM) + 2% fetal bovine serum (FBS) + 1% penicillin/streptomycin (P/S) for 18 hours, and
(ii) ratio of caspase 3/7 activity (%) in the presence of MG-132 with respect to cell viability (%) of 0.8 or less after being cultured in Dulbecco's Modified Eagle Medium (DMEM) + 2% fetal bovine serum (FBS) + 1% penicillin/streptomycin (P/S) for 18 hours.

24. The composition of claim 23, wherein the compound is a compound selected from the group consisting of Amlexanox, Olsalazine•Na, Hydroxocobalamin•HCl, Leflunomide, Febuxostat, Flurbiprofen, Terazosin•HCl, and Fluorouracil (5-Fluorouracil), a derivative or an analogue thereof, or a pharmaceutically acceptable salt thereof, or a solvate thereof.

25. The composition of claim 23 or 24, wherein the condition, disorder, or disease is a corneal endothelial condition, disorder, or disease due to transforming growth factor-β (TGF-β) and endoplasmic reticulum (ER) associated stress.

26. The composition of any one of claims 23 to 25, wherein the endoplasmic reticulum (ER) associated stress is due to abnormal folding of proteins.

27. The composition of any one of claims 23 to 26, wherein the condition, disorder, or disease is selected from conditions, disorders, or diseases associated with endoplasmic reticulum (ER) stress among damage to corneal endothelial cells in Fuchs' endothelial corneal dystrophy, corneal endothelial disorder, decreased corneal endothelial density, guttae formation, hypertrophy of the Descemet's membrane, hypertrophy of a cornea, turbidity, corneal epithelial disorder, turbidity in corneal stroma, photophobia, blurred vision, visual impairment, ophthalmalgia, epiphora, hyperemia, pain, bullous keratopathy, eye discomfort, diminished contrast, glare, edema of the corneal stroma, corneal epithelial erosion, and angiogenesis.

28. The composition of any one of claims 23 to 27, wherein the condition, disorder, or disease includes Fuchs' endothelial corneal dystrophy.

29. A caspase inhibitor comprising a compound selected from at least one compound shown in Table B:
**[Table B-1]**
| |
|---|
| Amlexanox |
| Leflunomide |
| Olsalazine•Na |
| Hydroxocobalamin•HCl |
| Mometasone Furoate |
| Febuxostat |
| Orphenadrine Citrate |
| Clobetasol Propionate |
| Loteprednol Etabonate |
| Difluprednate |
| Dexamethasone |
| Dobutamine•HCl |
| Amcinonide |
| Flurandrenolide |
| Fluorouracil (5-Fluorouracil) |
| Prednisolone |
| Fluocinolone Acetonide |
| Desonide |
| Triamcinolone Acetonide |
| Ketoconazole |
| Flurbiprofen |
| Budesonide |
| Fludrocortisone Acetate |
| Fluocinonide |
| Methylprednisolone |
| Betamethasone |
| Desoximetasone |
| Toremifene Base |
| Terazosin•HCl |
| Halcinonide |
| Bromocriptine Mesylate |
| Fluorometholone |
| Oxaliplatin |
| Calcipotriene |
| Beclomethasone Dipropionate |
**[Table B-2]**
| |
|---|
| Reserpine |
| Phenoxybenzamine•HCl |
| Droperidol |
| Promethazine•HCl |
| Esmolol |
| Dutasteride |
| Dihydroergotamine Mesylate |
| Imipramine•HCl |
| Betaxolol•HCl |
| Trimethobenzamide•HCl |
, a derivative or an analogue thereof, or a pharmaceutically acceptable salt thereof, or a solvate thereof.

30. The caspase inhibitor of claim 29, inhibiting caspase 3/7 activity.

31. A composition for treating or preventing a corneal endothelial condition, disorder, or disease, the composition comprising a compound selected from at least one compound shown in Table C:
**[Table C]**
| |
|---|
| Nitazoxanide |
| Mycophenolate Mofetil |
| Mycophenolic Acid |
| Deferasirox |
| Carvedilol |
| Fluoxetine•HCl |
| Fulvestrant |
| Tolterodine Tartrate |
| Pyrimethamine |
| Rifapentine |
| Nisoldipine |
| Oxiconazole Nitrate |
| Pimecrolimus |
| Calcitriol |
| Aripiprazole |
| Asenapine Maleate |
| Terbinafine•HCl |
, a derivative or an analogue thereof, or a pharmaceutically acceptable salt thereof, or a solvate thereof.

32. A caspase inhibitor comprising a selected from at least one compound shown in Table D:
**[Table D]**
| |
|---|
| Nitazoxanide |
| Mycophenolate Mofetil |
| Mycophenolic Acid |
| Deferasirox |
| Carvedilol |
| Fluoxetine•HCl |
| Fulvestrant |
| Tolterodine Tartrate |
| Pyrimethamine |
| Rifapentine |
| Nisoldipine |
| Oxiconazole Nitrate |
| Pimecrolimus |
| Calcitriol |
| Aripiprazole |
| Asenapine Maleate |
| Terbinafine•HCl |
, a derivative or an analogue thereof, or a pharmaceutically acceptable salt thereof, or a solvate thereof.

33. A method for treating or preventing a corneal endothelial condition, disorder, or disease, the method comprising administering an effective amount of a compound to a subject in need thereof, wherein the compound, when contacted with immortalized Fuchs' endothelial corneal dystrophy cells, exhibits: (i) cell viability (%) of the cells of about 90% or more after being cultured in Dulbecco's Modified Eagle Medium (DMEM) + 2% fetal bovine serum (FBS) + 1% penicillin/streptomycin (P/S) for 24 to 28 hours; and (ii) ratio of caspase 3/7 activity (%) in the presence of TGF-β with respect to the cell viability (%) of 0.8 or less after being cultured in Dulbecco's Modified Eagle Medium (DMEM) + 2% fetal bovine serum (FBS) + 1% penicillin/streptomycin (P/S) for 24 to 28 hours.

34. A method for treating or preventing a corneal endothelial condition, disorder, or disease, the method comprising administering an effective amount of a compound to a subject in need thereof, wherein the compound includes at least one selected from the group consisting of a selective glucocorticoid receptor agonist (SEGRA) and a selective glucocorticoid receptor modulator (SEGRM).

35. A method for treating or preventing a corneal endothelial condition, disorder, or disease, the method comprising administering an effective amount of a compound to a subject in need thereof, wherein the compound is a compound selected from at least one compound shown in Table C:
**[Table C]**
| |
|---|
| Nitazoxanide |
| Mycophenolate Mofetil |
| Mycophenolic Acid |
| Deferasirox |
| Carvedilol |
| Fluoxetine•HCl |
| Fulvestrant |
| Tolterodine Tartrate |
| Pyrimethamine |
| Rifapentine |
| Nisoldipine |
| Oxiconazole Nitrate |
| Pimecrolimus |
| Calcitriol |
| Aripiprazole |
| Asenapine Maleate |
| Terbinafine•HCl |
, a derivative or an analogue thereof, or a pharmaceutically acceptable salt thereof, or a solvate thereof.

36. The method of any one of claims 33 to 35, comprising any one or more features of claims 1 to 32.

37. Use of a compound for manufacturing a medicament for treating or preventing a corneal endothelial condition, disorder, or disease, wherein the compound, when contacted with immortalized Fuchs' endothelial corneal dystrophy cells, exhibits: (i) cell viability (%) of the cells of about 90% or more after being cultured in Dulbecco's Modified Eagle Medium (DMEM) + 2% fetal bovine serum (FBS) + 1% penicillin/streptomycin (P/S) for 24 to 28 hours; and (ii) ratio of caspase 3/7 activity (%) in the presence of TGF-β with respect to the cell viability (%) of 0.8 or less after being cultured in Dulbecco's Modified Eagle Medium (DMEM) + 2% fetal bovine serum (FBS) + 1% penicillin/streptomycin (P/S) for 24 to 28 hours.

38. Use of a compound for manufacturing a medicament for treating or preventing a corneal endothelial condition, disorder, or disease, wherein the compound includes at least one selected from the group consisting of a selective glucocorticoid receptor agonist (SEGRA) and a selective glucocorticoid receptor modulator (SEGRM).

39. Use of a compound for manufacturing a medicament for treating or preventing a corneal endothelial condition, disorder, or disease, wherein the compound is a compound selected from at least one compound shown in Table C:
**[Table C]**
| |
|---|
| Nitazoxanide |
| Mycophenolate Mofetil |
| Mycophenolic Acid |
| Deferasirox |
| Carvedilol |
| Fluoxetine•HCl |
| Fulvestrant |
| Tolterodine Tartrate |
| Pyrimethamine |
| Rifapentine |
| Nisoldipine |
| Oxiconazole Nitrate |
| Pimecrolimus |
| Calcitriol |
| Aripiprazole |
| Asenapine Maleate |
| Terbinafine•HCl |
, a derivative or an analogue thereof, or a pharmaceutically acceptable salt thereof, or a solvate thereof.

40. The use of any one of claims 37 to 39, comprising any one or more features of claims 1 to 32.

41. A compound for treating or preventing a corneal endothelial condition, disorder, or disease, wherein the compound, when contacted with immortalized Fuchs' endothelial corneal dystrophy cells, exhibits: (i) cell viability (%) of the cells of about 90% or more after being cultured in Dulbecco's Modified Eagle Medium (DMEM) + 2% fetal bovine serum (FBS) + 1% penicillin/streptomycin (P/S) for 24 to 28 hours; and (ii) ratio of caspase 3/7 activity (%) in the presence of TGF-β with respect to the cell viability (%) of 0.8 or less after being cultured in Dulbecco's Modified Eagle Medium (DMEM) + 2% fetal bovine serum (FBS) + 1% penicillin/streptomycin (P/S) for 24 to 28 hours.

42. At least one compound for treating or preventing a corneal endothelial condition, disorder, or disease, wherein the at least one compound is selected from the group consisting of a selective glucocorticoid receptor agonist (SEGRA) and a selective glucocorticoid receptor modulator (SEGRM).

43. A compound which is a compound selected from at least one compound shown in Table C:
**[Table C]**
| |
|---|
| Nitazoxanide |
| Mycophenolate Mofetil |
| Mycophenolic Acid |
| Deferasirox |
| Carvedilol |
| Fluoxetine•HCl |
| Fulvestrant |
| Tolterodine Tartrate |
| Pyrimethamine |
| Rifapentine |
| Nisoldipine |
| Oxiconazole Nitrate |
| Pimecrolimus |
| Calcitriol |
| Aripiprazole |
| Asenapine Maleate |
| Terbinafine•HCl |
, a derivative or an analogue thereof, or a pharmaceutically acceptable salt thereof, or a solvate thereof.

44. The use of a compound of any one of claims 41 to 43, comprising any one or more features of claims 1 to 32.
